# EUROPEAN PATENT APPLICATION

(11) **EP 3 861 982 A2**
(43) Date of publication of application: **11.08.2021**
(21) Application number: 20208058.6
(22) Date of filing: 05.12.2014
(51) Int. Cl.: A61K 9/00, A61K 47/34, A61K 31/5575, A61P 27/06, A61P 27/02

(54) **INTRACAMERAL IMPLANT FOR TREATMENT OF AN OCULAR CONDITION**

(30) Priority: 06.12.2013 US 201361912867 P; 10.01.2014 US 201461926112 P; 02.05.2014 US 201461987902 P
(62) Divisional of application: 14867106.8
(71) Applicant: ALLERGAN, INC., Irvine, CA 92612 (US)
(72) Inventor: Maynor, Benjamin, Durham, NC North Carolina 27707 (US); Garcia, Andres, Durham, NC North Carolina 27707 (US); Das, Sanjib Kumar, Cary, NC North Carolina 27519 (US); Navratil, Tomas, Carrboro, NC North Carolina 27510 (US); Yerxa, Benjamin Robinson, Raleigh, NC North Carolina 27607 (US); Tully, Janet, Cary, NC North Carolina 27513 (US)
(74) Representative: Hoffmann Eitle

(57) **Abstract**

The present invention relates to a pharmaceutical composition for treating an ocular condition, comprising a biodegradable polymer matrix and at least one therapeutic agent homogenously dispersed within the polymer matrix. The composition can be in the form of an ocular implant.

## Description

### CROSS REFERENCE TO RELATED APPLICATIONS

The present Application claims priority to U.S. Provisional Application No. 61/912,867, filed on December 06, 2013, and U.S. Provisional Application No. 61/926,112, filed on January 10, 2014, and U.S. Provisional Application No. 61/987,902, filed on May 02, 2014, the entire contents of each of which are hereby incorporated by reference in their entirety.

### FIELD

The present disclosure relates to the field of pharmaceutical compositions, implants formed from pharmaceutical compositions, methods of forming implants, and methods of treating ocular conditions.

### BACKGROUND

Glaucoma is a progressive optic neuropathy affecting more than three million Americans over the age of 39 and is a leading cause of blindness in adults over age 60. According to the National Eye Institute, more than 120,000 Americans are blind due to glaucoma (Quigley HA, Vitale S. "Models of open-angle glaucoma prevalence and incidence in the United States," Invest Ophthalmol & Visual Sci. 1997, 38(1):83-91.).

Elevated intraocular pressure (IOP) is the most important risk factor for the development of glaucoma and is a result of abnormally high resistance to aqueous humor drainage through the trabecular meshwork (TM), a multi-laminar array of collagen beams covered by endothelial-like cells.

Due to limited understanding of the pathophysiology of the optic neuropathy characteristic of glaucoma, current glaucoma therapies are focused on reducing IOP. The prostaglandin analogues (PGAs) are currently the most prescribed class of topical therapies for ocular hypertension or glaucoma in the United States. However, their use has been limited by several shortcomings.

First, the compliance with existing glaucoma topical therapies is generally low, with 30% to 60% of patients discontinuing the therapy within the first year of treatment.

Second, topical ophthalmic agents currently in use have local and systemic side effects. For example, these agents have a relatively high incidence of hyperemia accompanied by drug level peaks and troughs in the aqueous humor and the surrounding tissues, which potentially leads to 24 hour IOP fluctuations that may contribute to accelerated loss of visual field in susceptible patients (Caprioli J, Roht V. "Intraocular Pressure: Modulation as treatment for Glaucoma," Am J Ophthalmol. 2011;152(3):340-344.).

Lastly, the combination of these factors has been shown to increase the cost of patient care due to faster disease progression.

Therefore, there is a great need in the medical field for an alternative treatment using a sustained-release delivery system with an improved safety and efficacy profile. To date, there are no United States Food and Drug Administration (FDA) approved glaucoma therapies providing sustained release of a pharmacological agent directly to the desired site of action. Therefore, a sustained release pharmaceutical formulation administered directly to the anterior chamber of an eye would likely improve both compliance and the adverse event profile of current IOP-lowering profiles. Moreover, any extended release implant is highly dependent on the selection of polymers, co-polymers, drug-polymer interaction, load uniformity, porosity, size, surface-area to volume ratio, and the like for providing its drug release and degradation characteristics and the manufacturing techniques used in the prior art implants can induce inherent drawbacks in each of these parameters.

### BRIEF SUMMARY

The present disclosure addresses a crucial need in the art, by providing a sustained-release pharmaceutical formulation that may be directly administered to the anterior chamber of an eye and that does not suffer from the drawbacks of the current art.

Moreover, the present disclosure provides implants with highly uniform, tunable and reproducible size, shape, loading, composition, and load distribution, which provide implants having a desired extended drug release profile suitable for treating desired indications. In a particular embodiment, the implant is utilized to treat an ocular indication of an increased ocular pressure.

In certain embodiments, the disclosure relates to precisely engineered biodegradable drug delivery systems and methods of making and utilizing such systems.

The biodegradable drug delivery systems taught herein are, in some embodiments, engineered using a Particle Replication in Non-wetting Template (PRINT®) technology. The PRINT® Technology utilized in some embodiments allows for uniform size, shape, and dose concentration in the disclosed drug delivery systems.

Further, the disclosure provides methods of utilizing the taught precisely engineered biodegradable drug delivery systems to treat, *inter alia,* conditions of the eye.

Conditions treatable according to the present disclosure include glaucoma, elevated intraocular pressure, and ocular hypertension.

In certain embodiments, the present disclosure relates to pharmaceutical compositions for treating an ocular condition, comprising: a biodegradable polymer matrix and at least one therapeutic agent.

In certain embodiments, the present disclosure provides for pharmaceutical compositions for treating an ocular condition, comprising: an ocular implant. In aspects, the ocular implant comprises a biodegradable polymer matrix that contains a homogenously dispersed therapeutic agent therein. In some embodiments, the ocular implant is a "non-extruded" ocular implant, such as for example a molded implant.

In a particular embodiment, the disclosure provides a pharmaceutical composition for treating an ocular condition comprising: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 0.25 to 0.35 dL/g measured at 0.1% w/v CHCl₃ at 25°C measured with a Ubbelhode size 0c glass capillary viscometer.

Further, in particular embodiments, the poly(D,L-lactide-co-glycolide) copolymer comprises from about 73% to about 77% mole D,L-lactide and from about 23% to about 27% mole glycolide.

In certain embodiments, the biodegradable polymer matrix comprises as a % w/w of the pharmaceutical composition: about 10% to about 90% w/w, or about 10% to about 80%, or about 10% to about 70%, or about 10% to about 60%, or about 20% to about 90%, or about 20% to about 80%, or about 20% to about 70%, or about 20% to about 60%, or about 30% to about 90%, or about 30% to about 80%, or about 30% to about 70%, or about 30% to about 60%, or about 40% to about 90%, or about 40% to about 80%, or about 40% to about 70%, or about 40% to about 60%, or about 50% to about 90%, or about 50% to about 80%, or about 50% to about 70%, or about 50% to about 60%, or about 60% to about 90%, or about 60% to about 80%, or about 60% to about 75%, or about 60% to about 70%, or about 65% to about 75%, or about 68% to about 71%, or about 70%, w/w of the pharmaceutical composition.

In one embodiment, the biodegradable polymer matrix comprises from about 10% to about 30% of an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer and from about 70% to about 90% of an ester end-capped biodegradable poly(D,L-lactide) homopolymer having an inherent viscosity of 0.25 to 0.35 dL/g measured at 0.1% w/v CHCl₃ at 25°C with a Ubbelhode size 0c glass capillary viscometer.

In another embodiment, the biodegradable polymer matrix comprises from about 10% to about 20% of an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer and from about 80% to about 90% of an ester end-capped biodegradable poly(D,L-lactide) homopolymer having an inherent viscosity of 0.25 to 0.35 dL/g measured at 0.1% w/v CHCl₃ at 25°C with a Ubbelhode size 0c glass capillary viscometer.

In another embodiment, the biodegradable polymer matrix comprises about 15% of an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer and about 85% of an ester end-capped biodegradable poly(D,L-lactide) homopolymer having an inherent viscosity of 0.25 to 0.35 dL/g measured at 0.1% w/v CHCl₃ at 25°C with a Ubbelhode size 0c glass capillary viscometer.

In certain aspects, the pharmaceutical composition's at least one therapeutic agent comprises: about 20% to about 50% w/w, or about 20% to about 40% w/w, or about 20% to about 30%, or about 20% to about 35% w/w, or about 25% to about 35% w/w, or about 25%, or about 26%, or about 27%, or about 28%, or about 29%, or about 30%, or about 31%, or about 32%, or about 33%, or about 34%, or about 35%, or about 29% to about 32%, of the pharmaceutical composition.

In some embodiments, the at least one therapeutic agent is selected from the group consisting of a prostaglandin, prostaglandin prodrug, prostaglandin analogue, and prostamide, pharmaceutically acceptable salts thereof, and mixtures thereof.

In particular embodiments, the at least one therapeutic agent is selected from the group consisting of latanoprost, travoprost, bimatoprost, tafluprost, and unoprostone isopropyl.

In one embodiment, the at least one therapeutic agent comprises travoprost.

Further, in some embodiments, the pharmaceutical composition comprises an ocular implant, wherein said ocular implant comprises: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 0.25 to 0.35 dL/g measured at 0.1% w/v CHCl₃ at 25°C measured with a Ubbelhode size 0c glass capillary viscometer.

In some embodiments, the ocular implant is a rod-shaped implant comprising a shortest dimension of between about 150 to about 225 µm and a longest dimension of between about 1,500 to about 3,000 µm in length.

In other embodiments, the ocular implant is a rod-shaped implant selected from the group consisting of: a rod-shaped implant having dimensions of about 150 µm × about 150 µm × about 1,500 µm, a rod-shaped implant having dimensions of about 160 µm x about 180 µm × about 3,000 µm, and a rod-shaped implant having dimensions of about 225 µm × about 225 µm × about 2,925 µm.

In some aspects, the disclosure provides a pharmaceutical composition for treating an ocular condition, wherein the composition is fabricated as a rod-shaped ocular implant comprising a shortest dimension of between about 145-245 µm and a longest dimension of between about 1,500-3,000 µm in length.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 50 µm of each dimension, or a rod-shaped ocular implant having dimensions of 225 µm × 240 µm × 2,925 µm (W × H × L) ± 50 µm of each dimension.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 40 µm of each dimension, or a rod-shaped ocular implant having dimensions of 225 µm × 240 µm × 2,925 µm (W × H × L) ± 40 µm of each dimension.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 30 µm of each dimension, or a rod-shaped ocular implant having dimensions of 225 µm × 240 µm × 2,925 µm (W × H × L) ± 30 µm of each dimension.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 20 µm of each dimension, or a rod-shaped ocular implant having dimensions of 225 µm × 240 µm × 2,925 µm (W × H × L) ± 20 µm of each dimension.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 10 µm of each dimension, or a rod-shaped ocular implant having dimensions of 225 µm × 240 µm × 2,925 µm (W × H × L) ± 10 µm of each dimension.

In some aspects, the disclosure provides a pharmaceutical composition for treating an ocular condition, wherein the composition is fabricated as a rod-shaped ocular implant having dimensions of 150 µm × 180 µm × 1,500 µm (W × H × L) ± 5 µm of each dimension, or a rod-shaped ocular implant having dimensions of 225 µm × 240 µm × 2,925 µm (W × H × L) ± 5 µm of each dimension.

In some aspects, the disclosure provides a pharmaceutical composition for treating an ocular condition, wherein the composition is fabricated as a rod-shaped ocular implant having dimensions of about 150 µm × 180 µm × 1,500 µm (W × H × L) ±, or a rod-shaped ocular implant having dimensions of about 225 µm × 240 µm × 2,925 µm (W × H × L).

In an embodiment, the ocular implants can have an aspect ratio of width-to-length from 1:1 to greater than 1:30. In some embodiments, the width-to-length aspect ratio of the ocular implant is between 1:2 to 1:25. In some embodiments, the width-to-length aspect ratio of the ocular implant is between 1:5 to 1:20. In some embodiments, the width-to-length aspect ratio of the ocular implant is between 1:10 to 1:20. In some embodiments, the width-to-length aspect ratio of the ocular implant is between 1:15 to 1:20.

In embodiments, the PRINT® particle technology can be utilized in the present disclosure to fabricate implants in the size range of 10 micrometers in a broadest dimension or larger, depending on the size designed into the mold cavities (as further described herein and in the art incorporated herein by reference).

Importantly, for intraorbital ophthalmic applications, the density of the implant is fabricated to be greater than the density of the fluid environment in which the implant will be placed, such as for example the aqueous humor or the like, such that the implant settles and remains outside the field of view of the patient and the implant also remains in the eye.

Furthermore, the larger surface area to volume ratio of the particles having smaller overall dimensions, for example, a 10 micron cube compared to a 100 micron cube, will degrade more rapidly. Likewise, a collection of, for example, 10 micron cube particles having total overall volume equal to a 100x100x2000 micron implant will conform to the shape of the space to which they are implanted much more closely than the 100x100x2000 micron implant.

In some embodiments, the implants have a largest cross-sectional dimension of 10 micrometers and a density greater than that of the aqueous humor, vitreous humor, or the like such that the implant settles due to gravitational forces. In some embodiments, the implants have a largest cross-sectional dimension of 20 micrometers and a density greater than that of the aqueous humor, vitreous humor, or the like such that the implant settles due to gravitational forces. In some embodiments, the implants have a largest cross-sectional dimension of 50 micrometers and a density greater than that of the aqueous humor, vitreous humor, or the like such that the implant settles due to gravitational forces. In some embodiments, the implants have a largest cross-sectional dimension of 100 micrometers and a density greater than that of the aqueous humor, vitreous humor, or the like such that the implant settles due to gravitational forces. In some embodiments, the implants have a largest cross-sectional dimension of 200 micrometers and a density greater than that of the aqueous humor, vitreous humor, or the like such that the implant settles due to gravitational forces. In some embodiments, the implants have a largest cross-sectional dimension of 500 micrometers and a density greater than that of the aqueous humor, vitreous humor, or the like such that the implant settles due to gravitational forces.

Methods of the present disclosure for treating or preventing an ophthalmic condition include inserting more than 5 sustained release drug loaded biodegradable polymer based implants intraorbitally to treat or prevent the ophthalmic condition for more than 2 weeks. Methods of the present disclosure for treating or preventing an ophthalmic condition include inserting more than 10 sustained release drug loaded biodegradable polymer based implants intraorbitally to treat or prevent the ophthalmic condition for more than 2 weeks. Methods of the present disclosure for treating or preventing an ophthalmic condition include inserting more than 25 sustained release drug loaded biodegradable polymer based implants intraorbitally to treat or prevent the ophthalmic condition for more than 2 weeks. Methods of the present disclosure for treating or preventing an ophthalmic condition include inserting more than 50 sustained release drug loaded biodegradable polymer based implants intraorbitally to treat or prevent the ophthalmic condition for more than 2 weeks. Methods of the present disclosure for treating or preventing an ophthalmic condition include inserting more than 100 sustained release drug loaded biodegradable polymer based implants intraorbitally to treat or prevent the ophthalmic condition for more than 2 weeks. Methods of the present disclosure for treating or preventing an ophthalmic condition include inserting more than 500 sustained release drug loaded biodegradable polymer based implants intraorbitally to treat or prevent the ophthalmic condition for more than 2 weeks. Methods of the present disclosure for treating or preventing an ophthalmic condition include inserting more than 1,000 sustained release drug loaded biodegradable polymer based implants intraorbitally to treat or prevent the ophthalmic condition for more than 2 weeks. Methods of the present disclosure for treating or preventing an ophthalmic condition include inserting more than 10,000 sustained release drug loaded biodegradable polymer based implants intraorbitally to treat or prevent the ophthalmic condition for more than 2 weeks. Methods of the present disclosure for treating or preventing an ophthalmic condition include inserting more than 100,000 sustained release drug loaded biodegradable polymer based implants intraorbitally to treat or prevent the ophthalmic condition for more than 2 weeks. Methods of the present disclosure for treating or preventing an ophthalmic condition include inserting more than 1,000,000 sustained release drug loaded biodegradable polymer based implants intraorbitally to treat or prevent the ophthalmic condition for more than 2 weeks.

The polymer composition and ratios of each implant in these collections of small implants can be varied between implants within a single dose such that an aggregate degradation profile of the collection of implants is achieved for delivery of the active agent for greater than 2 weeks, greater than 1 month, greater than 3 months, greater than 4 months, greater than 6 months, greater than 9 months and greater than 12 months.

Delivery of such implants disclosed herein include delivery through a 27 gauge needle or smaller.

In one embodied delivery method the needle is a 28 gauge, 29 gauge, 30 gauge, 31 gauge, 32 gauge, 33 gauge, or 34 gauge needle.

Further still, are disclosed pharmaceutical compositions for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer. In some embodiments polymer i) makes up between 12-32 wt% of the implant and polymer ii) makes up between 35-55 wt% of the implant with the balance of the implant being the active drug component. In some embodiments polymer i) makes up between 17-27 wt% of the implant and polymer ii) makes up between 40-50 wt% of the implant with the balance of the implant being the active drug component. In some embodiments polymer i) makes up between 19-25 wt% of the implant and polymer ii) makes up between 42-48 wt% of the implant with the balance of the implant being the active drug component. In some embodiments polymer i) makes up between 20-24 wt% of the implant and polymer ii) makes up between 42-46 wt% of the implant with the balance of the implant being the active drug component. In some embodiments polymer i) makes up between 21-23 wt% of the implant and polymer ii) makes up between 43-45 wt% of the implant with the balance of the implant being the active drug component. In some embodiments polymer i) makes up 22 +/- 0.5 wt% of the implant and polymer ii) makes up 44.5 +/-0.5 wt% of the implant with the balance of the implant being the active drug component. In some embodiments polymer i) makes up 22.1 wt% of the implant and polymer ii) makes up 44.9 wt% of the implant with the balance of the implant being the active drug component. In some embodiments polymer i) makes up 21.8 wt% of the implant and polymer ii) makes up 44.2 wt% of the implant with the balance of the implant being the active drug component.

In an embodiment, the biodegradable polymer matrix comprises: i) from about 15% to about 35% of the ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) from about 65% to about 85% of the ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer.

In another embodiment, the biodegradable polymer matrix comprises: i) from about 25% to about 35% of the ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) from about 65% to about 75% of the ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer. In another embodiment, the implant matrix comprises: i) 22 +/- 3 % of ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; ii) 45 +/- 3 % of ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer; and iii) 33 +/- 3 % active drug travoprost. In yet another embodiment, the implant matrix comprises: i) 22 +/- 0.5 % of ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; ii) 44.5 +/-0.5 % of ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer; and iii) 33 +/- 0.5 % active drug travoprost.

In a particular embodiment, the biodegradable polymer matrix comprises: i) about 33% of the ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) about 67% of the ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer.

In an aspect, the disclosure presents, a pharmaceutical composition comprising an ocular implant, wherein said ocular implant comprises: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer.

In certain embodiments, the disclosure presents a pharmaceutical composition for treating an ocular condition, comprising: a biodegradable implant comprising a polymer matrix comprising at least one polymer; and a therapeutic agent homogenously dispersed within the polymer matrix; wherein the implant comprises: a length within 10%, 7.5%, 5%, 2.5%, 2%, 1.5%, 1%, 0.5%, 0.25%, 0.1% of 3000 microns; a width within 10%, 7.5%, 5%, 2.5%, 2%, 1.5%, 1%, 0.5%, 0.25%, 0.1% of 160 microns; and a height within 10%, 7.5%, 5%, 2.5%, 2%, 1.5%, 1%, 0.5%, 0.25%, 0.1% of 180 microns. In some aspects, the implant degrades over a period not less than 90 days in the anterior chamber of the eye and releases the therapeutic agent for at least 90 days, thereby maintaining a reduction in intraocular pressure over the 90 day duration.

Another embodiment disclosed herein is a pharmaceutical composition for treating an ocular condition, comprising: a biodegradable implant comprising a polymer matrix comprising at least one polymer; and a therapeutic agent homogenously dispersed within the polymer matrix; wherein the implant comprises: a length within 10%, 7.5%, 5%, 2.5%, 2%, 1.5%, 1%, 0.5%, 0.25%, 0.1% of 1500 microns; a width within 10%, 7.5%, 5%, 2.5%, 2%, 1.5%, 1%, 0.5%, 0.25%, 0.1% of 150 microns; and a height within 10%, 7.5%, 5%, 2.5%, 2%, 1.5%, 1%, 0.5%, 0.25%, 0.1% of 150 microns. In some aspects, the implant degrades over a period not less than 90 days in the anterior chamber of the eye and releases the therapeutic agent for at least 90 days, thereby maintaining a reduction in intraocular pressure over the 90 day duration.

In another aspect, taught herein is a pharmaceutical composition for treating an ocular condition, comprising: a biodegradable implant comprising a polymer matrix comprising at least one polymer; and a therapeutic agent homogenously dispersed within the polymer matrix; wherein the implant comprises: a length within 10%, 7.5%, 5%, 2.5%, 2%, 1.5%, 1%, 0.5%, 0.25%, 0.1% of 2925 microns; a width within 10%, 7.5%, 5%, 2.5%, 2%, 1.5%, 1%, 0.5%, 0.25%, 0.1% of 225 microns; and a height within 10%, 7.5%, 5%, 2.5%, 2%, 1.5%, 1%, 0.5%, 0.25%, 0.1% of 225 microns. In some aspects, the implant degrades over a period not less than 90 days in the anterior chamber of the eye and releases the therapeutic agent for at least 90 days, thereby maintaining a reduction in intraocular pressure over the 90 day duration.

In other embodiments, the disclosure provides a pharmaceutical composition for treating an ocular condition, comprising: a biodegradable implant comprising a polymer matrix comprising at least one polymer; and a therapeutic agent homogenously dispersed within the polymer matrix; wherein the implant comprises; a therapeutic agent weight percent within 10%, 7.5%, 5%, 2.5%, 2%, 1.5%, 1%, 0.5%, 0.25%, 0.1% of 30% of the implant overall weight; and polymer matrix weight percent within 10%, 7.5%, 5%, 2.5%, 2%, 1.5%, 1%, 0.5%, 0.25%, 0.1% of 70% of the implant overall weight.

Another embodiment provided herein is a method for treating an ocular condition, comprising: implanting at least one implant into an eye of a patient having an elevated intraocular pressure, wherein each implant has a volume within 10%, 7.5%, 5%, 2.5%, 2%, 1.5%, 1%, 0.5%, 0.25%, 0.1% of 86,400,000 cubic microns.

In an aspect, one, two, three, four, five, six, seven, eight, nine, or more implants are provided in the method and are implanted. The plurality of implants may be implanted simultaneously into the eye of a patient, sequentially during the same treatment, or sequentially over a period of time during several treatments. In some aspects, a patient receives yearly implants.

Another embodiment provides a method for treating an ocular condition, comprising: implanting at least one implant into an eye of a patient having an elevated intraocular pressure, wherein each implant has a volume within 10%, 7.5%, 5%, 2.5%, 2%, 1.5%, 1%, 0.5%, 0.25%, or 0.1% of 33,750,000 cubic microns. Another embodiment provides a method for treating an ocular condition, comprising: implanting at least one implant into an eye of a patient having an elevated intraocular pressure, wherein each implant has a volume within 10%, 7.5%, 5%, 2.5%, 2%, 1.5%, 1%, 0.5%, 0.25%, or 0.1% of 40,500,000 cubic microns.

Also provided is a method for treating an ocular condition, comprising: implanting at least one implant into an eye of a patient having an elevated intraocular pressure, wherein each implant has a volume within 10%, 7.5%, 5%, 2.5%, 2%, 1.5%, 1%, 0.5%, 0.25%, or 0.1% of 148,078,125 cubic microns. Also provided is a method for treating an ocular condition, comprising: implanting at least one implant into an eye of a patient having an elevated intraocular pressure, wherein each implant has a volume within 10%, 7.5%, 5%, 2.5%, 2%, 1.5%, 1%, 0.5%, 0.25%, of 0.1% of 154,659,375 cubic microns.

Further, in one aspect, the disclosure provides a method for treating an ocular condition, comprising: reducing intraocular pressure of an eye with elevated intraocular pressure for at least 90 days following insertion into the anterior chamber of the eye of: one implant having a volume within 10%, 7.5%, 5%, 2.5%, 2%, 1.5%, 1%, 0.5%, 0.25%, or 0.1% of 154,659,375 cubic microns and therapeutic agent content between about 20% and about 45%; or two implants, each having a volume within 10%, 7.5%, 5%, 2.5%, 2%, 1.5%, 1%, 0.5%, 0.25%, or 0.1% of 86,400,000 cubic microns and therapeutic agent content between about 20% and about 40%; or three implants, each having a volume within 10%, 7.5%, 5%, 2.5%, 2%, 1.5%, 1%, 0.5%, 0.25%, or 0.1% of 40,500,000 cubic microns and therapeutic agent content between about 20% and about 40%.

In embodiments, implants may have a volume of 40,500,000 cubic microns, or 86,400,000 cubic microns, or 154,659,375 cubic microns. In some embodiments, the volume from implant to implant may vary by about 0.1% to about 10%, 0.1% to about 5%, 0.5% to about 2%, or 0.5% to about 1%. The disclosure provides for compositions comprising the implants, kits comprising the implants, and methods of utilizing the aforementioned implants with the stated cubic micron volumes.

Also provided here is a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide) homopolymer having an inherent viscosity at 25°C in 0.1% w/v CHCl₃ of 0.25 to 0.35 dl/g; and ii) an ester end-capped biodegradable poly(D,L-lactide) homopolymer having an inherent viscosity at 25°C in 0.1% w/v CHCl₃ of 1.8 to 2.2 dl/g, wherein the poly(D,L-lactide) homopolymer i) and the poly(D,L-lactide) homopolymer ii) are present in a ratio of about 1:2 to 1:3.

In certain embodiments, the aforementioned polymer matrix excludes other polymers from being present in the composition. For instance, in some aspects PEG is not present. In some embodiments, hot melt extrusion is not used to fabricate the implants. In some embodiments, in-situ gelation is not utilized to fabricate the implants. In certain embodiments, the pharmaceutical formulations exclude implants that are not of the following volumes: 148,078,125 ± 10% cubic microns, or 86,400,000 ± 10% cubic microns, or 33,750,000 ± 10% cubic microns. Some embodiments of the present pharmaceutical formulations exclude implants that are not of the following dimensions: 225 µm x 225 µm × 2,925 µm, or 180 µm × 160 µm × 3,000 µm, or 150 µm × 150 µm × 1,500 µm. In certain embodiments, the pharmaceutical formulations exclude implants that are not of the following volumes: 154,659,375 ± 10% cubic microns from 225 µm × 235 µm × 2,925 µm and 40,500,000 ± 10% cubic microns from 150 µm × 180 µm × 1,500 µm implants. Some embodiments taught herein exclude implants that are not fabricated in a mold based method, such as by, e.g. PRINT® Technology fabrication.

In some aspects, the composition comprises a two polymer matrix comprising R203S/R208S in a ratio of about 1:2 to about 1:3. In some embodiments, the composition comprises R203S in an amount of 23.29±2.0 wt% and R208S in an amount of 47.30±2.0 wt%.

In an embodiment, the polymer matrix comprises: i) from about 25% to about 35% of an ester end-capped biodegradable poly(D,L-lactide) homopolymer having an inherent viscosity at 25°C in 0.1% w/v CHCl₃ of 0.25 to 0.35 dL/g; and ii) from about 65% to about 75% of an ester end-capped biodegradable poly(D,L-lactide) homopolymer having an inherent viscosity at 25°C in 0.1% w/v CHCl₃ of 1.8 to 2.2 dL/g.

In certain embodiments, the pharmaceutical composition's polymer blend comprises: i) about 33% of R203S; and ii) about 67% of R208S.

Further provided herein is a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide-co-glycolide) copolymer having an inherent viscosity at 25°C in 0.1% w/v CHCl₃ of 0.16 to 0.24 dL/g and comprising 73-77 mol % D,L-lactide and 23-27 mol % glycolide; and ii) an ester end-capped biodegradable poly(D,L-lactide) homopolymer having an inherent viscosity at 25°C in 0.1% w/v CHCl₃ of 0.25 to 0.35 dL/g.

In certain embodiments, the aforementioned polymer matrix excludes other polymers from being present in the composition. For instance, in some aspects PEG is not present.

In some embodiments, the poly(D,L-lactide-co-glycolide) copolymer and poly(D,L-lactide) homopolymer are present in a ratio of about 1:5 to 1:6, or 1:5 to 1:7.

In some aspects, the composition comprises a two polymer matrix comprising RG752S/R203S in a ratio of about 1:5 to about 1:6. In some embodiments, the composition comprises RG752S in an amount of 10.5±2.0 wt% and R203S in an amount of 59.5±2.0 wt%.

In certain embodiments, the pharmaceutical composition's polymer blend comprises: i) about 15% of RG752S; and ii) about 85% of R203S.

Some embodiments the polymer matrix comprises from about 10% to about 20% of an ester end-capped biodegradable poly(D,L-lactide-co-glycolide) copolymer having an inherent viscosity at 25°C in 0.1% w/v CHCl₃ of 0.16 to 0.24 dL/g and comprising 73-77 mol % D,L-lactide and 23-27 mol % glycolide and from about 80% to about 90% of an ester end-capped biodegradable poly(D,L-lactide) homopolymer having an inherent viscosity at 25°C in 0.1% w/v CHCl₃ of 0.25 to 0.35 dL/g.

Another aspect of the disclosure entails a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers selected from the following: i) a polymer matrix comprising: a) a first ester end-capped biodegradable poly(D,L-lactide) homopolymer having an inherent viscosity at 25°C in 0.1% w/v CHCl₃ of 0.25 to 0.35 dl/g; and b) a second ester end-capped biodegradable poly(D,L-lactide) homopolymer having an inherent viscosity at 25°C in 0.1% w/v CHCl₃ of 1.8 to 2.2 dl/g, wherein said first and second poly(D,L-lactide) homopolymers are present in a ratio of about 1:2 to 1:3; OR, ii) a polymer matrix comprising: a) an ester end-capped biodegradable poly(D,L-lactide-co-glycolide) copolymer having an inherent viscosity at 25°C in 0.1% w/v CHCl₃ of 0.16 to 0.24 dL/g and comprising 73-77 mol % D,L-lactide and 23-27 mol % glycolide; and b) an ester end-capped biodegradable poly(D,L-lactide) homopolymer having an inherent viscosity at 25°C in 0.1% w/v CHCl₃ of 0.25 to 0.35 dL/g, wherein the poly(D,L-lactide-co-glycolide) copolymer and poly(D,L-lactide) homopolymer are present in a ratio of about 1:5 to 1:6.

In some aspects, the disclosure provides a method of treating elevated intraocular pressure in a human subject by administering, via intracameral injection, a solid, biodegradable, rod-shaped intracameral implant to the subject. In aspects, the implant is delivered directly into the anterior chamber of the subject's eye, where it resides at the inferior iridocorneal angle. In some aspects, the implant resides at the 6:00 o'clock position of the eye. In a particular aspect, the implants of the disclosure do not migrate substantially from their initial position. In other aspects, the implants may move substantially from their initial position. In embodiments of the disclosed methods, intraocular pressure in a human is controlled for 4 to 6 months following implantation, via intracameral injection, of implants having an initial therapeutic agent content ranging from about: 1 to 500 µg per eye, 1 to 400 µg per eye, 1 to 300 µg per eye, 1 to 200 µg per eye, 1 to 150 µg per eye, 1 to 140 µg per eye, 1 to 130 µg per eye, 1 to 120 µg per eye, 1 to 110 µg per eye, 1 to 100 µg per eye, 1 to 90 µg per eye, 1 to 80 µg per eye, 1 to 70 µg per eye, 1 to 60 µg per eye, 1 to 50 µg per eye, 1 to 40 µg per eye, 1 to 30 µg per eye, 1 to 20 µg per eye, or 1 to 10 µg per eye. In some embodiments, the drug is travoprost. The travoprost is released from the implant over time treating the ocular condition.

In particular embodiments, 3 implants are administered to an eye of a patient, wherein said implants each have a volume of 148,078,125 cubic microns, and thus a total travoprost dosage of about 130 µg per eye is given to the patient over the course of the treatment. In this embodiment, each implant having a volume of 148,078,125 cubic microns comprises about 43.3 µg of travoprost.

In other embodiments, 3 implants are administered to an eye of a patient, wherein said implants each have a volume of 148,078,125 cubic microns, and thus a total travoprost dosage of about 121 µg per eye is given to the patient over the course of the treatment. In this embodiment, each implant having a volume of 148,078,125 cubic microns comprises about 40.4 µg of travoprost.

In particular embodiments, 1 implant is administered to an eye of a patient, wherein said implant has a volume of 148,078,125 cubic microns, and thus a total travoprost dosage of about 40.4 µg per eye is given to the patient over the course of the treatment.

In yet other embodiments, 3 implants are administered to an eye of a patient, wherein said implants each have a volume of 33,750,000 cubic microns, and thus a total travoprost dosage of about 24 µg per eye is given to the patient over the course of the treatment. In this embodiment, each implant having a volume of 33,750,000 cubic microns comprises about 8 µg of travoprost.

In particular embodiments, 1 implant is administered to an eye of a patient, wherein said implant has a volume of 33,750,000 cubic microns, and thus a total travoprost dosage of about 8 µg per eye is given to the patient over the course of the treatment.

Also disclosed is a pharmaceutical composition, comprising: at least one ocular implant, wherein said at least one ocular implant has a volume of 33,750,000 ± 10% cubic microns; and comprises: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide-co-glycolide) copolymer having an inherent viscosity of 0.16 to 0.24 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 0.25 to 0.35 dL/g measured at 0.1% w/v CHCl₃ at 25°C measured with a Ubbelhode size 0c glass capillary viscometer.

Some embodiments entail administering one ocular implant having a volume of 33,750,000 ± 10% cubic microns to an eye. Other embodiments entail administering two ocular implants each having a volume of 33,750,000 ± 10% cubic microns to an eye. Yet other embodiments entail administering three ocular implants each having a volume of 33,750,000 ± 10% cubic microns to an eye. Yet other embodiments entail administering three or more ocular implants each having a volume of 33,750,000 ± 10% cubic microns to an eye.

In some embodiments, each of the aforementioned ocular implants having a volume of 33,750,000 ± 10% cubic microns contains a travoprost content of from 1 µg to 10 µg. In a particular embodiment, each of the aforementioned ocular implants having a volume of 33,750,000 ± 10% cubic microns contains a travoprost content of 8 µg ± 2 µg.

Further provided by the present disclosure is a pharmaceutical composition, comprising: at least one ocular implant, wherein said at least one ocular implant has a volume of 148,078,125 ± 10% cubic microns; and comprises: A) a biodegradable polymer matrix; and B) at least one therapeutic agent homogenously dispersed within the polymer matrix; wherein the biodegradable polymer matrix contains a mixture of polymers comprising: i) an ester end-capped biodegradable poly(D,L-lactide) homopolymer having an inherent viscosity of 0.25 to 0.35 dL/g measured at 0.1% w/v in CHCl₃ at 25 °C with a Ubbelhode size 0c glass capillary viscometer; and ii) an ester end-capped biodegradable poly(D,L-lactide) homopolymer having an inherent viscosity of 1.8 to 2.2 dL/g measured at 0.1% w/v in CHCl₃ at 25 °C with a Ubbelhode size 0c glass capillary viscometer.

Some embodiments entail administering one ocular implant having a volume of 148,078,125 ± 10% cubic microns to an eye. Other embodiments entail administering two ocular implants each having a volume of 148,078,125 ± 10% cubic microns to an eye. Yet other embodiments entail administering three ocular implants each having a volume of 148,078,125 ± 10% cubic microns to an eye. Yet other embodiments entail administering three or more ocular implants each having a volume of 148,078,125 ± 10% cubic microns to an eye.

In some embodiments, each of the aforementioned ocular implants having a volume of 148,078,125 ± 10% cubic microns contains a travoprost content of from 1 µg to 50 µg, or from 20 µg to 50 µg, or from 30 µg to 50 µg. In a particular embodiment, each of the aforementioned ocular implants having a volume of 148,078,125 ± 10% cubic microns contains a travoprost content of 43.3 µg ± 2 µg. In another particular embodiment, each of the aforementioned ocular implants having a volume of 148,078,125 ± 10% cubic microns contains a travoprost content of 40.4 µg ± 2 µg.

Some embodiments taught herein provide a biodegradable sustained release ocular implant, comprising: at least one therapeutic agent that is homogeneously dispersed within a biodegradable polymer matrix; wherein said biodegradable sustained release ocular implant is formulated to release a therapeutically effective amount of the at least one therapeutic agent for a period of time of at least about 180 days upon administration to a patient; and wherein said at least one biodegradable sustained release ocular implant demonstrates an in vitro release profile of from about 1% to a maximum of about 35% of the at least one therapeutic agent released during the period between day zero and day 160. In some embodiments, the biodegradable sustained release ocular implant demonstrates an in vitro release profile of less than about 30% of the at least one therapeutic agent released during the period between day zero and day 84. In some embodiments, the ocular implant comprises from about 25% to about 35% of poly(D,L-lactide) R203S and from about 65% to about 75% of poly(D,L-lactide) R208S.

Other embodiments taught herein provide a biodegradable sustained release ocular implant, comprising: at least one therapeutic agent that is homogeneously dispersed within a biodegradable polymer matrix; wherein said biodegradable sustained release ocular implant is formulated to release a therapeutically effective amount of the at least one therapeutic agent for a period of time up to about 70 to 90 days upon administration to a patient, at which point approximately 100% of the at least one therapeutic agent will have been released; and wherein said at least one biodegradable sustained release ocular implant demonstrates an in vitro release profile of from about 1% to a maximum of about 25% of the at least one therapeutic agent released during the period between day zero and day 28. In some embodiments, the sustained release ocular implant demonstrates an in vitro release profile of less than about 45% of the at least one therapeutic agent released during the period between day zero and day 56. In some embodiments, the sustained release ocular implant demonstrates an in vitro release profile of at least 50% of the at least one therapeutic agent released during the period between day 56 and day 84. In some embodiments, the ocular implant comprises from about 10% to about 20% of poly(D,L-lactide-co-glycolide) RG752S and from about 80% to about 90% of poly(D,L-lactide) R203S.

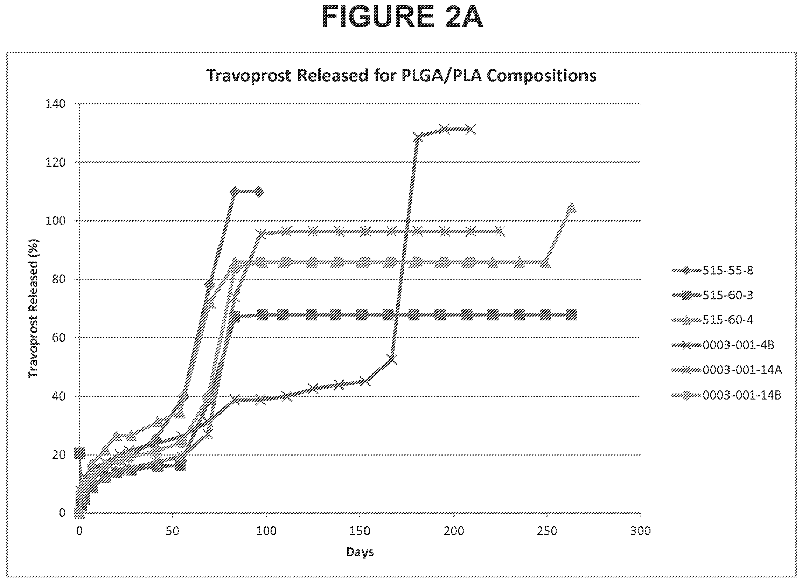

In particular embodiments, the disclosure provides for sustained release ocular implants that demonstrate an in vitro release profile corresponding to the in vitro release profiles depicted by the implants of FIG. 2A-2F Further, the disclosure provides for polymer matrix compositions that demonstrate the release profiles illustrated in any of the disclosed figures or tables, or release profiles that are mathematically derivable from the data in said figures and tables.

In some aspects, the ocular implant is formulated for treating an ocular condition characterized by an elevated intraocular pressure. In some aspects, the ocular implant is formulated for treating glaucoma. In some aspects, the ocular implant is formulated for treating ocular hypertension.

In one embodiment, the ocular implant is sized and structured to allow for implantation of the implant into the inferior iridocorneal angle of the anterior chamber of an eye.

In some aspects, the ocular implant is formulated to not increase in size by more than 5% during the entire period between initial administration to a patient and 180 days post-administration to a patient.

In some aspects, the ocular implant is sized and structured to allow for administration with a needle for delivery. In some embodiments, the needle is 27 gauge.

An important aspect of some embodiments of the present disclosure is the uniformity and control of overall size to the tolerances discussed herein to provide for use of the smallest needle gauge as possible. An implant will have between 10-50 micron clearance between overall maximum implant cross-sectional width and inside needle diameter. In other embodiments, an implant-needle clearance shall be between 20-40 micron between overall maximum implant cross-sectional width and inside needle diameter. In other embodiments, an implant-needle clearance shall be not less than 40 micron between overall maximum implant cross-sectional width and inside needle diameter. In other embodiments, an implant-needle clearance shall be not less than 30 micron between overall maximum implant cross-sectional width and inside needle diameter. In other embodiments, an implant-needle clearance shall be not less than 20 micron between overall maximum implant cross-sectional width and inside needle diameter. In other embodiments, an implant-needle clearance shall be not less than 10 micron between overall maximum implant cross-sectional width and inside needle diameter. It will be appreciated by one of ordinary skill in the art that the three-dimensional shape of the implant can be designed to maximize the volume of the inner opening of the needle or to facilitate the desired loading, insertion, tissue deposition or other parameter of the implant or treatment. In some embodiments, the molds and implants of the present disclosure are designed as cylindrical implants. In some embodiments the cylindrical implants are fabricated with a cross-sectional diameter that is not less than 30 micrometers smaller than the inner diameter of the needle. In some embodiments the implant, mold, or master from which the mold is made is fabricated utilizing additive manufacturing techniques.

In an embodiment of the present disclosure a 27 gauge ultra-thin walled needle is utilized with the 150x150x1500 micrometer implants of the present disclosure. It will be appreciated by one of ordinary skill in the art that enabling small needle size is an important feature of some embodiments of the present disclosure to minimize tissue damage.

In an embodiment, the ocular implant comprises: i) about 30% w/w of the at least one therapeutic agent; and ii) about 70% w/w of the biodegradable polymer matrix.

In a particular embodiment, the ocular implant comprises: i) the active agent travoprost (30% loading w/w); and ii) a biodegradable polymer matrix comprising: a poly(D,L-lactide) (PLA) blend of R203S and R208S (70% w/w) polymers, wherein said ocular implant has dimensions of 225 µm × 225 µm × 2,925 µm and a volume of 148,078,125 ± 5% cubic microns.

In an embodiment, the ocular implant comprises: i) the active agent travoprost (33% loading w/w); and ii) a biodegradable polymer matrix comprising: a poly(D,L-lactide) (PLA) blend of R203S (22.11% w/w) and R208S (44.89% w/w) polymers, wherein said ocular implant is molded from a mold cavity having dimensions of 225 µm × 240 µm × 2,925 µm. In another embodiment, the ocular implant comprises: i) the active agent travoprost (34% loading w/w); and ii) a biodegradable polymer matrix comprising: a poly(D,L-lactide) (PLA) blend of R203S (21.78% w/w) and R208S (44.22% w/w) polymers, wherein said ocular implant is molded from a mold cavity having dimensions of 150 µm × 180 µm × 1,500 µm.

In another embodiment, the ocular implant comprises: i) the active agent travoprost (30% loading w/w); and ii) a biodegradable polymer matrix comprising: a poly(D,L-lactide-co-glycolide) / poly(D,L-lactide) (PLGA / PLA) blend of RG752S and R203S (70% w/w) polymers, wherein said ocular implant has dimensions of 150 µm × 150 µm × 1,500 µm and a volume of 33,750,000 ± 5% cubic microns.

In a particular embodiment, the ocular implant is manufactured by a process comprising: 1) providing a mold, wherein the mold comprises a plurality of recessed areas formed therein; 2) disposing a volume of liquid material in the plurality of recessed areas; 3) forming a plurality of substantially uniform implants; and 4) harvesting the implants from the patterned template, wherein each of said implants substantially mimics the recessed areas.

Some embodiments comprise a kit for administering a biodegradable sustained release ocular implant, comprising: (a) at least one biodegradable sustained release ocular implant; wherein said at least one biodegradable sustained release ocular implant comprises at least one therapeutic agent that is homogeneously dispersed within a biodegradable polymer matrix; and (b) a single use ocular implant applicator that comprises a needle or needlelike device. In some embodiments the needle or needlelike device is 22 gauge or smaller. In an embodiment the needle like device has a needle internal diameter not less than 40 micrometers larger than the largest cross-sectional diameter of the implant. In an embodiment the needle like device has a needle internal diameter not less than 30 micrometers larger than the largest cross-sectional diameter of the implant. In an embodiment the needle like device has a needle internal diameter not less than 20 micrometers larger than the largest cross-sectional diameter of the implant.

In some aspects, the implants produced according to the present disclosure exhibit a therapeutic agent release profile that has very low inter-implant variability. The therapeutic agent release profiles exhibited by some implants of the present disclosure are consistent across implants and demonstrate variation that is not statistically significant. Consequently, the drug release profiles demonstrated by embodiments of the implants exhibit coefficients of variation that are within a confidence interval and not biologically relevant.

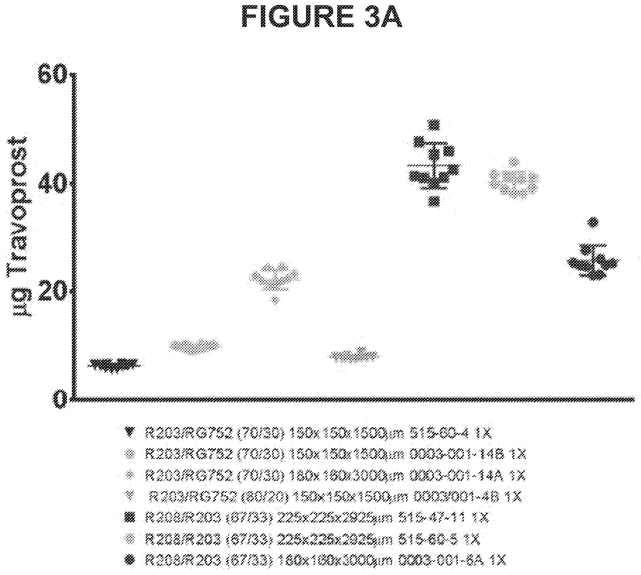

In some aspects, the therapeutic agent content amongst implants of a given configuration is highly consistent In particular embodiments, the implants of the present disclosure possess a therapeutic agent content that does not vary significantly amongst implants of a given configuration. In an embodiment, the therapeutic agent content of implants having a given configuration does not vary in a statistically significant manner from one another. In particular embodiments, the implants of the disclosure possess a therapeutic agent content variation amongst members having a given configuration, as illustrated in the therapeutic agent content uniformity graphics of FIG 3A-3C.

In certain aspects, the disclosure provides a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) 22 +/- 5% of ester end-capped biodegradable poly(D,L-lactide) homopolymer having an inherent viscosity of 0.25 to 0.35 dL/g measured at 0.1% w/v in CHCl₃ at 25°C with a Ubbelhode size 0c glass capillary viscometer; and ii) 45 +/-5% of ester end-capped biodegradable poly(D,L-lactide) homopolymer having an inherent viscosity of 1.8 to 2.2 dL/g measured at 0.1% w/v in CHCl₃ at 25°C with a Ubbelhode size 0c glass capillary viscometer.

In certain aspects, the disclosure provides a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) 22 +/- 3% of ester end-capped biodegradable poly(D,L-lactide) homopolymer having an inherent viscosity of 0.25 to 0.35 dL/g measured at 0.1% w/v in CHCl₃ at 25°C with a Ubbelhode size 0c glass capillary viscometer; and ii) 45 +/-3% of ester end-capped biodegradable poly(D,L-lactide) homopolymer having an inherent viscosity of 1.8 to 2.2 dL/g measured at 0.1% w/v in CHCl₃ at 25°C with a Ubbelhode size 0c glass capillary viscometer.

In certain aspects, the disclosure provides a pharmaceutical composition for treating an ocular condition, comprising: A) a biodegradable polymer matrix; and B) at least one therapeutic agent homogenously dispersed within the polymer matrix; wherein the biodegradable polymer matrix contains a mixture of polymers comprising: i) 22 +/- 1% of ester end-capped biodegradable poly(D,L-lactide) homopolymer having an inherent viscosity of 0.25 to 0.35 dL/g measured at 0.1% w/v in CHCl₃ at 25°C with a Ubbelhode size 0c glass capillary viscometer; and ii) 45 +/-1% of ester end-capped biodegradable poly(D,L-lactide) homopolymer having an inherent viscosity of 1.8 to 2.2 dL/g measured at 0.1% w/v in CHCl₃ at 25°C with a Ubbelhode size 0c glass capillary viscometer.

In an aspect, the pharmaceutical composition for treating an ocular condition taught herein has a biodegradable polymer matrix comprising 65% ± 5% w/w of the pharmaceutical composition, or 65% ± 3% w/w of the pharmaceutical composition, or 65% ± 1% w/w of the pharmaceutical composition.

In an aspect, the pharmaceutical composition for treating an ocular condition taught herein has at least one therapeutic agent homogenously dispersed within the polymer matrix selected from the group consisting of: a prostaglandin, a prostaglandin prodrug, a prostaglandin analogue, a prostamide, and combinations thereof.

In an aspect, the pharmaceutical composition for treating an ocular condition taught herein has at least one therapeutic agent homogenously dispersed within the polymer matrix selected from the group consisting of: latanoprost, travoprost, bimatoprost, tafluprost, unoprostone isopropyl, and combinations thereof.

In an aspect, the pharmaceutical composition for treating an ocular condition taught herein has at least travoprost homogenously dispersed within the polymer matrix.

In an aspect, the pharmaceutical composition for treating an ocular condition taught herein is fabricated as an ocular implant.

In an aspect, the pharmaceutical composition for treating an ocular condition taught herein is fabricated as an ocular implant and said fabrication does not comprise hot-melt extrusion.

In an aspect, the pharmaceutical composition for treating an ocular condition taught herein is fabricated as an ocular implant and said fabrication occurs at a temperate range of about 340°F to about 350°F. In some aspects, the fabrication of the ocular implants does not occur in the 250°F to 300°F temperature range.

In an aspect, the pharmaceutical composition for treating an ocular condition taught herein is fabricated as an ocular implant and wherein the implant degrades in not less than 90 days in the anterior chamber of a human eye and releases the therapeutic agent for more than 90 days.

In an aspect, the pharmaceutical composition for treating an ocular condition taught herein is fabricated as an ocular implant and used in a method to treat glaucoma, or elevated intraocular pressure, or ocular hypertension.

### BRIEF DESCRIPTION OF THE DRAWINGS

**FIG. 1** is a schematic of an implant placed in the iridocorneal angle of the eye and also a depiction of the aqueous humor outflow located in the iridocorneal angle of the eye.
**FIG. 2A** shows the percent travoprost released as a function of time (days) for an in vitro study.
**FIG. 2B** shows the amount of travoprost released (µg) as a function of time (days) for an in vitro study.
**FIG. 2C** shows the in vitro release rate of travoprost (ng/day) as a function of time (days).
**FIG. 2D** shows the percent travoprost released as a function of time (days) for an in vitro study.
**FIG. 2E** shows the amount of travoprost released (µg) as a function of time (days) for an in vitro study.
**FIG. 2F** shows the in vitro release rate of travoprost (ng/day) as a function of time (days).
**FIG. 3A** illustrates a therapeutic agent content uniformity graphic for the implants which are illustrated in FIGS. 2A-2F.
**FIG. 3B** illustrates a therapeutic agent content uniformity graphic for select implants utilizing an R208S/R203S polymer matrix.
**FIG. 3C** illustrates a therapeutic agent content uniformity graphic for select implants utilizing an R203S/RG752S polymer matrix.
**FIG. 4A** shows the in vitro % travoprost released over time of implants 515-55-8 (2 implants) and 515-47-11 (3 implants) over a 180 day period.
**FIG. 4B** shows the in vitro cumulative travoprost released (µg) over time of implants 515-55-8 (2 implants) and 515-47-11 (3 implants) over a 180 day period.
**FIG. 4C** shows the in vitro travoprost release rate (ng/day) over time of implants 515-55-8 (2 implants) and 515-47-11 (3 implants) over a 180 day period.
**FIG. 5** shows IOP as a function of time for 515-47-11 (3 implants) and 515-55-8 (2 implants) in normotensive beagle dogs for in vivo study PRE004 over 224 days.
**FIG. 6** shows IOP lowering effects of 515-55-8 (2 implants) in normotensive beagle dogs for in vivo study PRE004 over 84 days.
**FIG. 7** shows IOP lowering effects of implant 515-47-11 (3 implants) in normotensive beagle dogs for in vivo study PRE004 over 84 days.
**FIG. 8** shows IOP as a function of time for 515-60-4 (3 implants); 515-60-5 (1 implant); and 515-60-5 (3 implants) in normotensive beagle dogs for in vivo study PRE006 over 168 days.
**FIG. 9** shows IOP lowering effects of 515-60-4 (3 implants); 515-60-3 (1 implant); and 515-60-3 (3 implants) in normotensive beagle dogs for in vivo study PRE006 over 84 days.
**FIG. 10A** shows IOP lowering effects of 515-60-5 (1 implant) and 515-60-5 (3 implants) in normotensive beagle dogs for in vivo study PRE006 over 84 days (as unaudited exemplary data).
[001351 **FIG. 10B** shows IOP lowering effects of 515-60-5 (1 implant) and 515-60-5 (3 implants) in normotensive beagle dogs for in vivo study PRE006 over 280 days.
**FIG. 11A** shows IOP as a function of time for 003-001-6B (3 implants); 003-001-6A (3 implants); and 003-001-8A (3 implants) in normotensive beagle dogs for in vivo study PRE009 (PLA/PLA Matrix) over 126 days (as unaudited exemplary data).

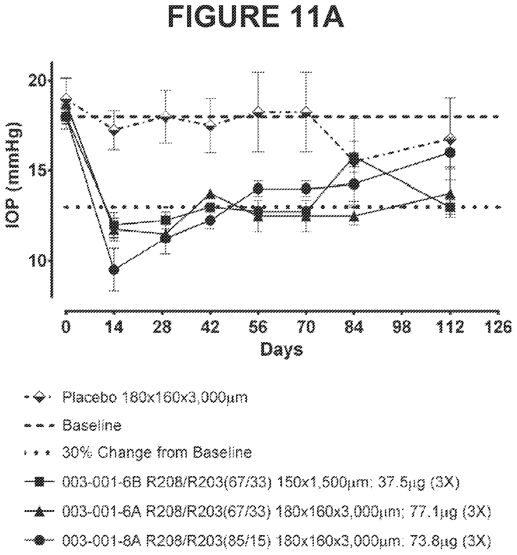

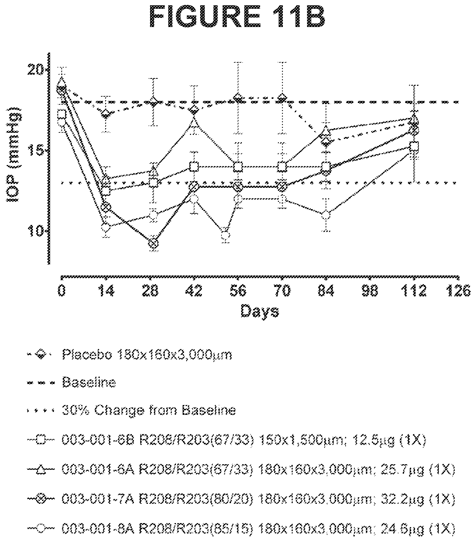

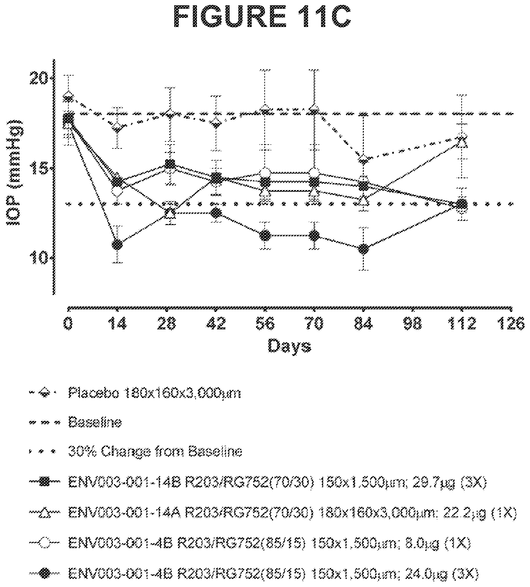

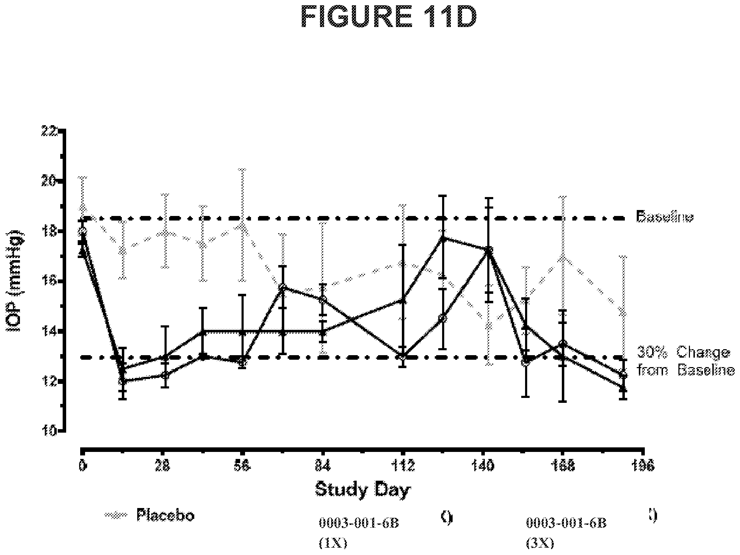

**FIG. 11****.B** shows IOP as a function of time for 003-001-6B (1 implant); 003-001-6A (1 implant); 003-001-7A (1 implant); and 003-001-8A (1 implant) in normotensive beagle dogs for in vivo study PRE009 (PLA/PLA Matrix) over 126 days (as unaudited exemplary data).
**FIG. 11C** shows IOP as a function of time for 003-001-14B (3 implants); 003-001-14A (1 implant); 003-001-4B (1 implant); and 003-001-4B (3 implants) in normotensive beagle dogs for in vivo study PRE009 (PLA/PLGA Matrix) over 126 days (as unaudited exemplary data).
**FIG. 11D** shows IOP as a function of time for 0003-001-6B (1 implant) and 0003-001-6B (3 implants) in normotensive beagle dogs for in vivo study PRE009 over 189 days.

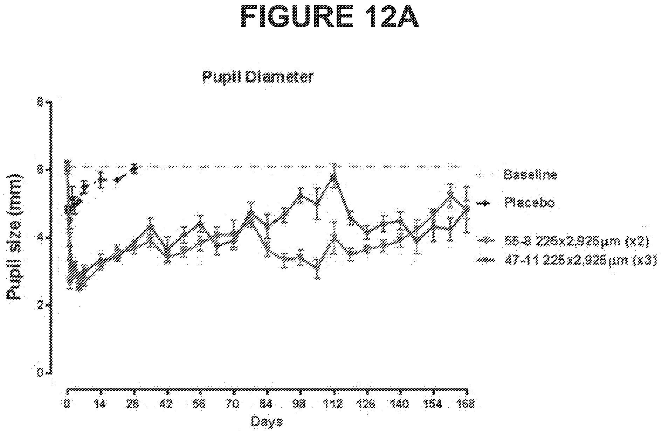

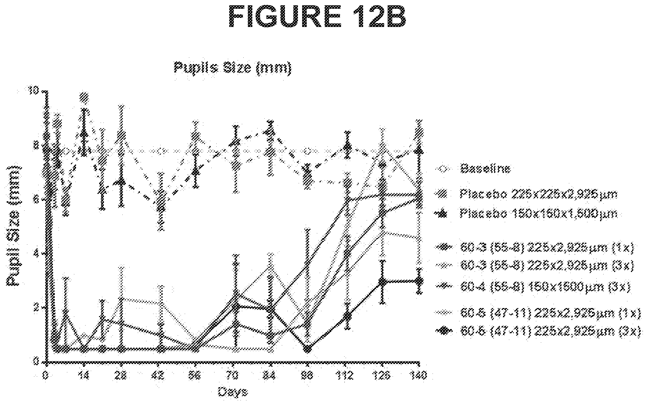

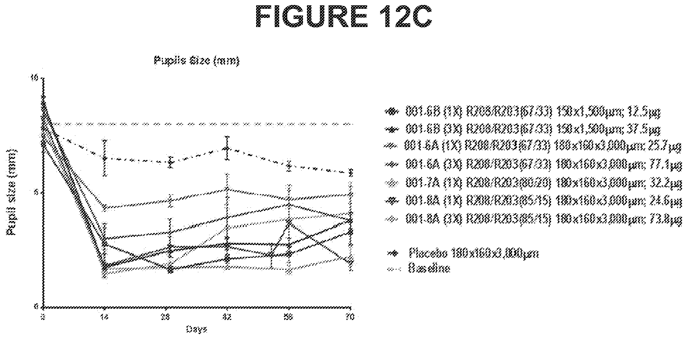

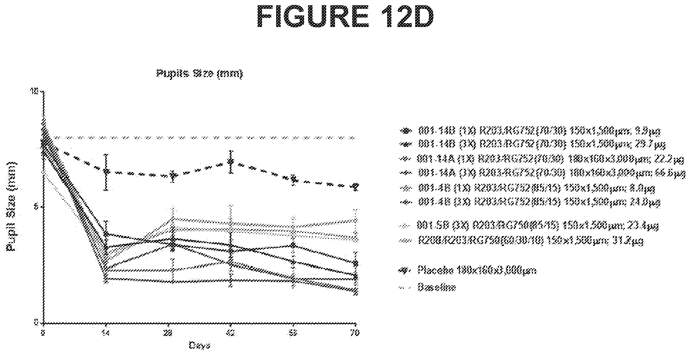

**FIG. 12** illustrates pupil miosis effects of the implants in beagle dogs. **(****FIG. 12A** (PRE004 Study), **FIG. 12B** (PRE006 Study), **FIG. 12C** and **FIG. 12D** (PRE009 Study).

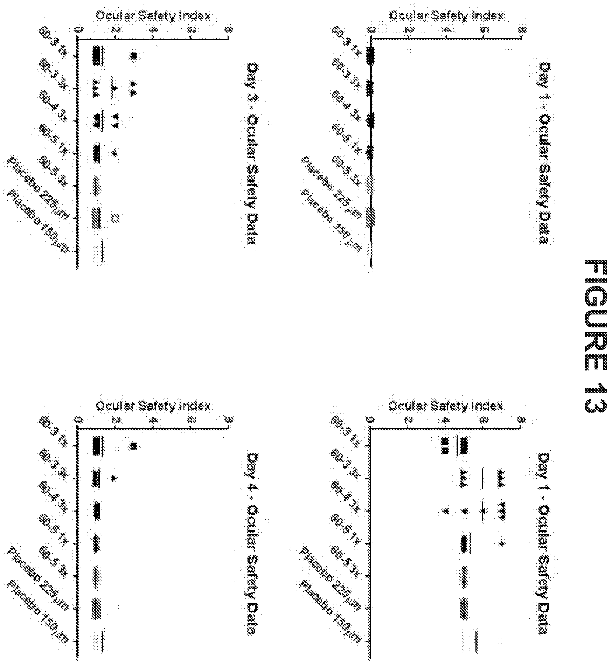

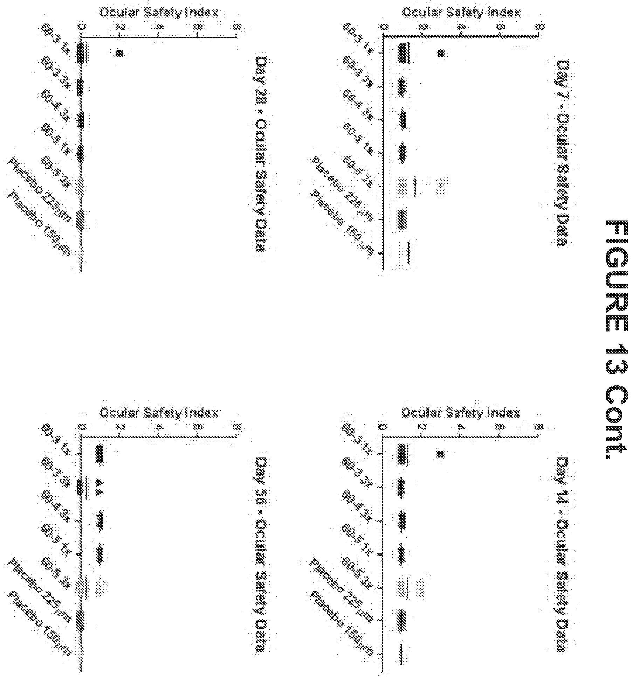

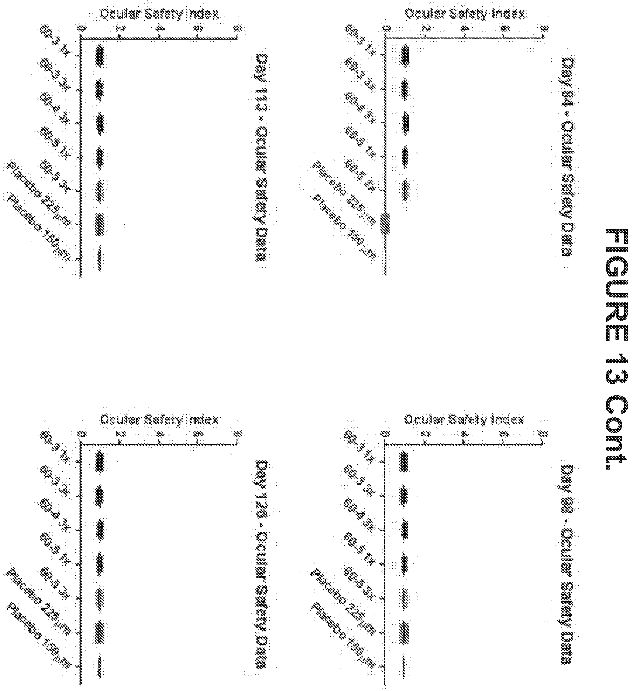

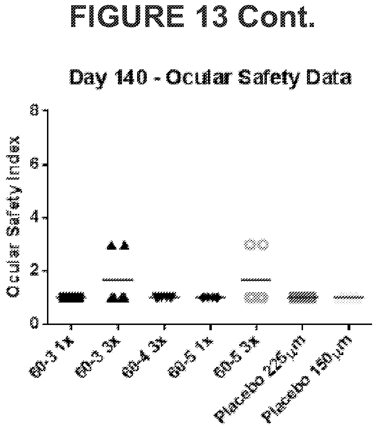

**FIG. 13** illustrates ocular safety index for travoprost implants with dog-specific reduced pupillary light reflex excluded over a 140 day period.

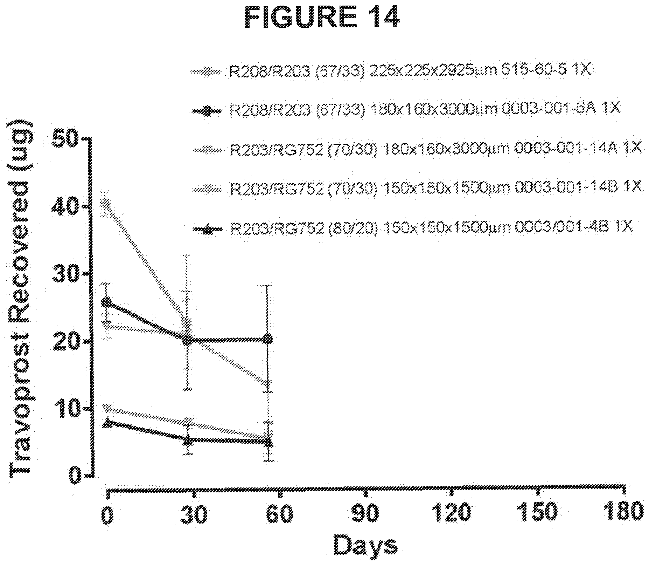

**FIG. 14** illustrates amount of travoprost recovered (µg) in vivo at days 28 and 56 post intracameral insertion.

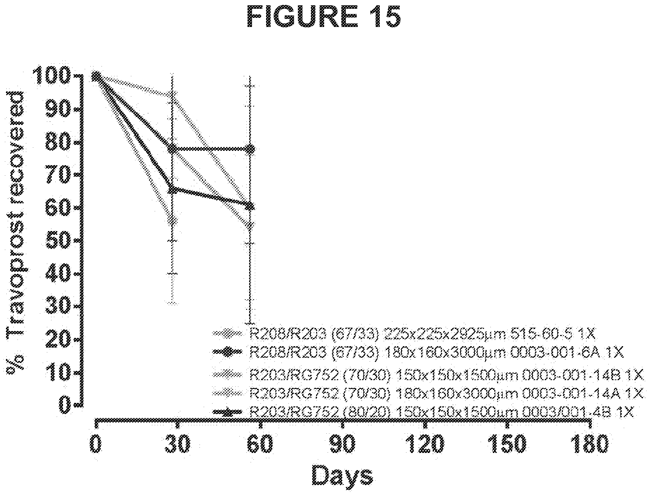

**FIG. 15** illustrates % of travoprost recovered in vivo at days 28 and 56 post intracameral insertion.

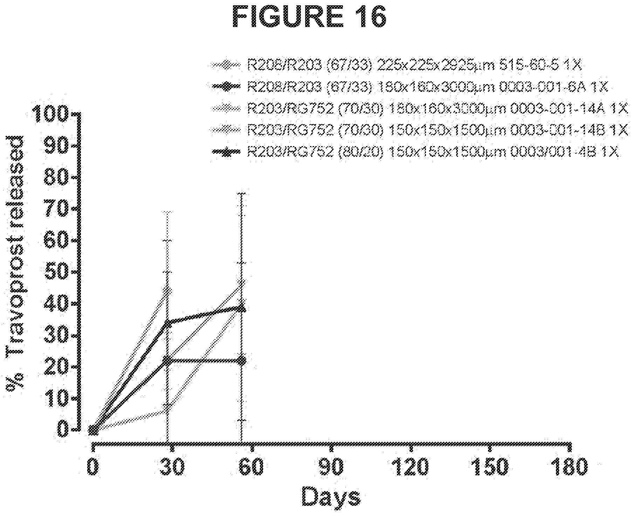

**FIG. 16** illustrates % of travoprost released in vivo at days 28 and 56 post intracameral insertion.

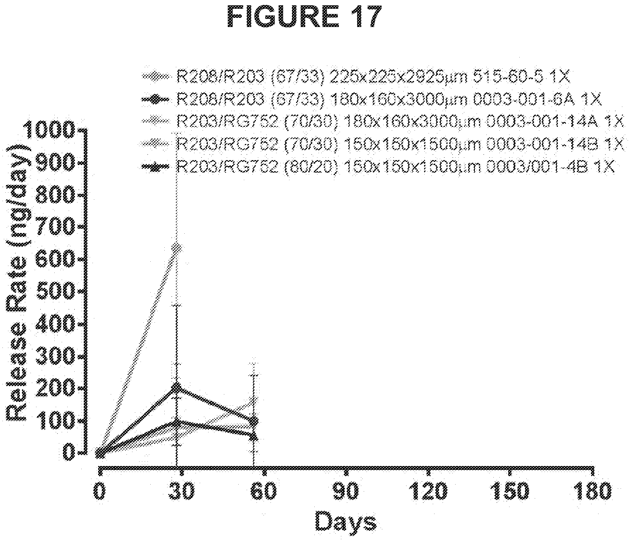

**FIG. 17** illustrates travoprost release rate (ng/day) in vivo at days 28 and 56 post intracameral insertion.

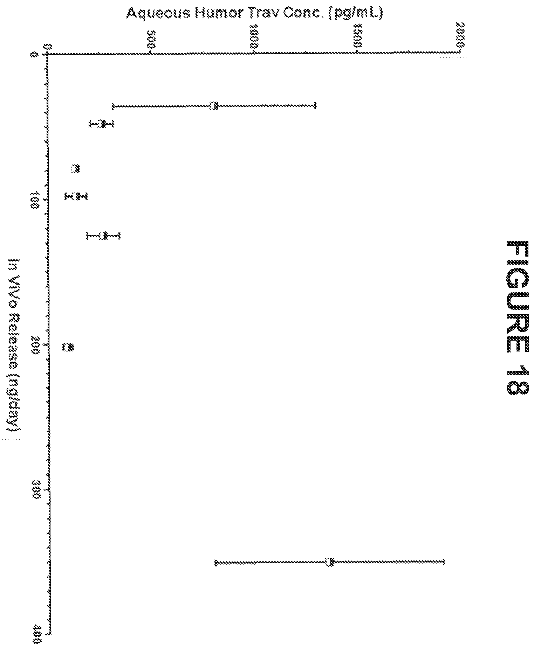

**FIG. 18** is a representation of the in vivo rate of release vs. aqueous humor concentration of travoprost 1 month post dose. The graph depicts the in vivo rate of release (ng/day) on the x-axis and the travoprost free acid concentration in the aqueous humor (pg/mL) on the y-axis.

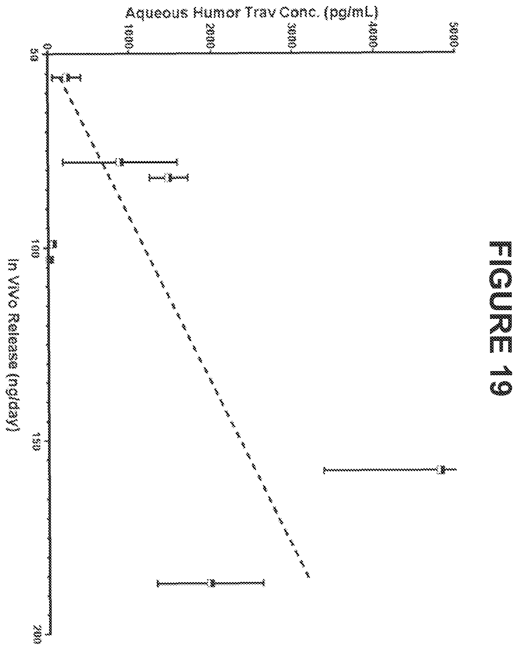

**FIG. 19** is a representation of the in vivo rate of release vs. aqueous humor concentration of travoprost 2 months post dose. The graph depicts the in vivo rate of release (ng/day) on the x-axis and the travoprost free acid concentration in the aqueous humor (pg/mL) on the y-axis.

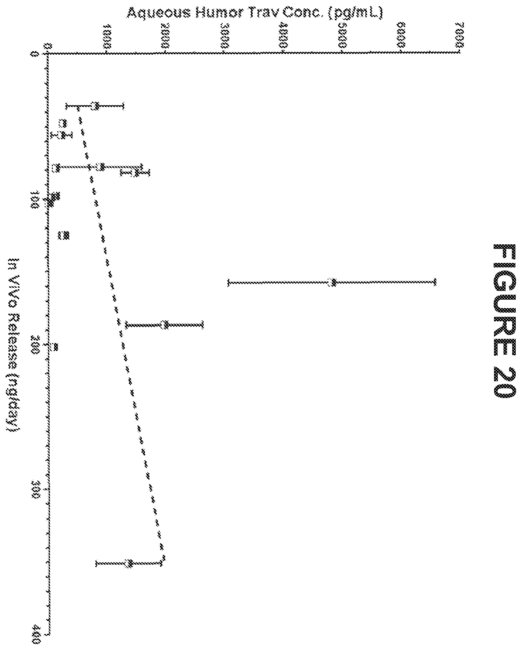

**FIG. 20** is a representation of the in vivo rate of release vs. aqueous humor concentration of travoprost combined 1 and 2 month post dose data. The graph depicts the in vivo rate of release (ng/day) on the x-axis and the travoprost free acid concentration in the aqueous humor (pg/mL) on the y-axis.

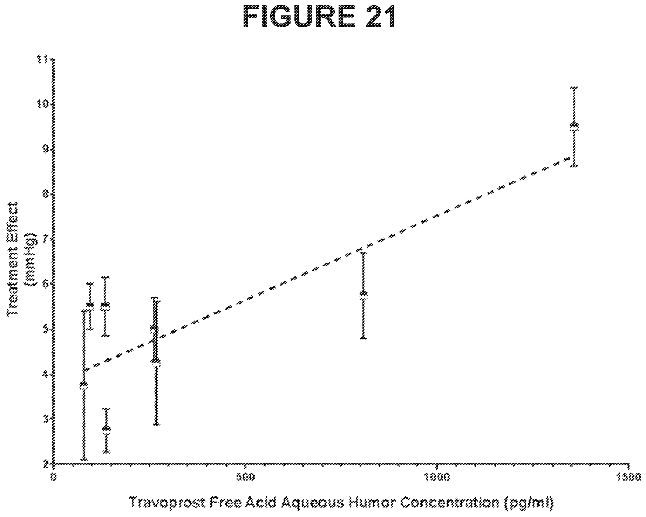

**FIG. 21** is a representation of the IOP vs. aqueous humor concentration of travoprost 1 month post dose. The graph depicts the travoprost free acid concentration in the aqueous humor (pg/mL) on the x-axis and the IOP treatment effect (mmHg) on the y-axis.

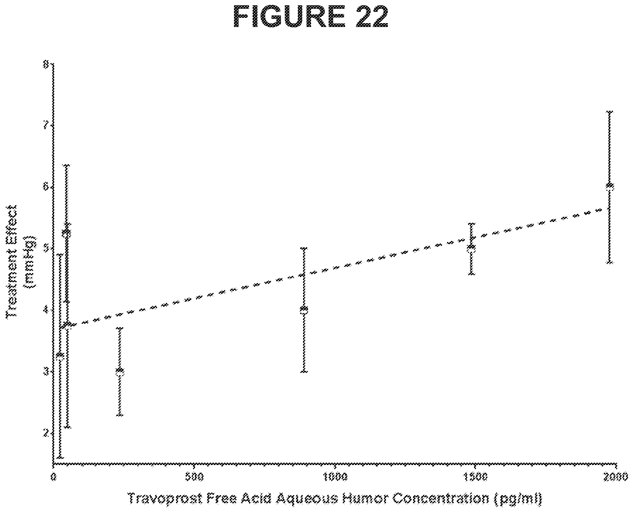

**FIG. 22** is a representation of the IOP vs. aqueous humor concentration of travoprost 2 months post dose. The graph depicts the travoprost free acid concentration in the aqueous humor (pg/mL) on the x-axis and the IOP treatment effect (mmHg) on the y-axis.

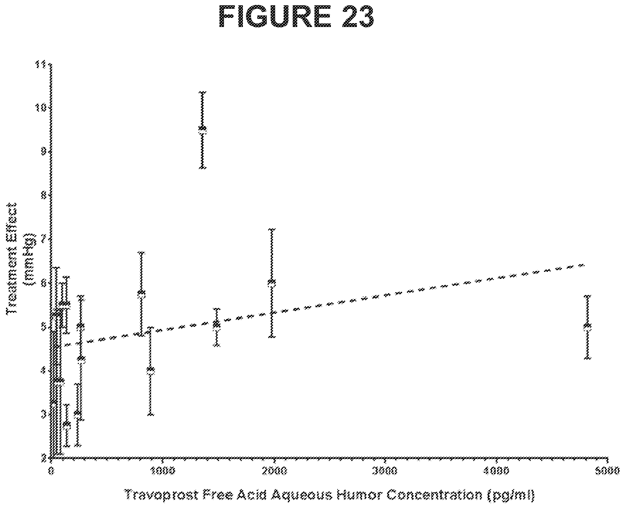

**FIG. 23** is a representation of the IOP vs. aqueous humor concentration of travoprost combined 1 and 2 months post dose data. The graph depicts the travoprost free acid concentration in the aqueous humor (pg/mL) on the x-axis and the IOP treatment effect (mmHg) on the y-axis.

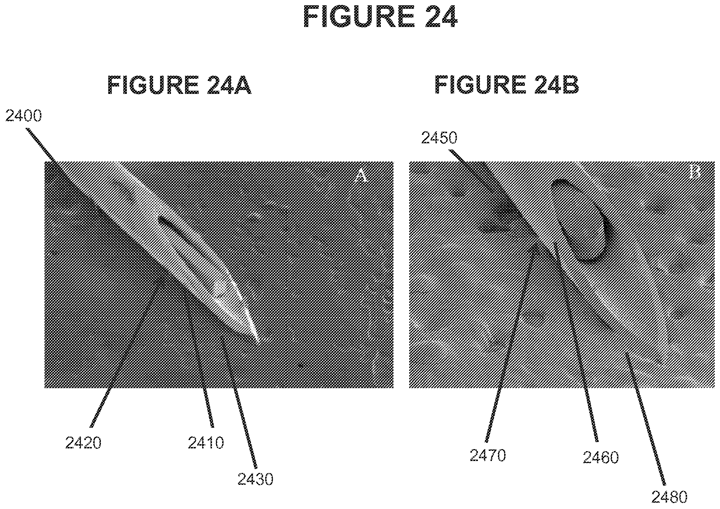

**FIG. 24A** is an electron micrograph illustrating a 150 µm x 150 µm x 1,500 µm implant in a 27G thin-walled needle. **FIG. 24B** is an electron micrograph illustrating a 225 µm x 225 µm x 2,925 µm implant in a 27G ultra thin-walled needle.

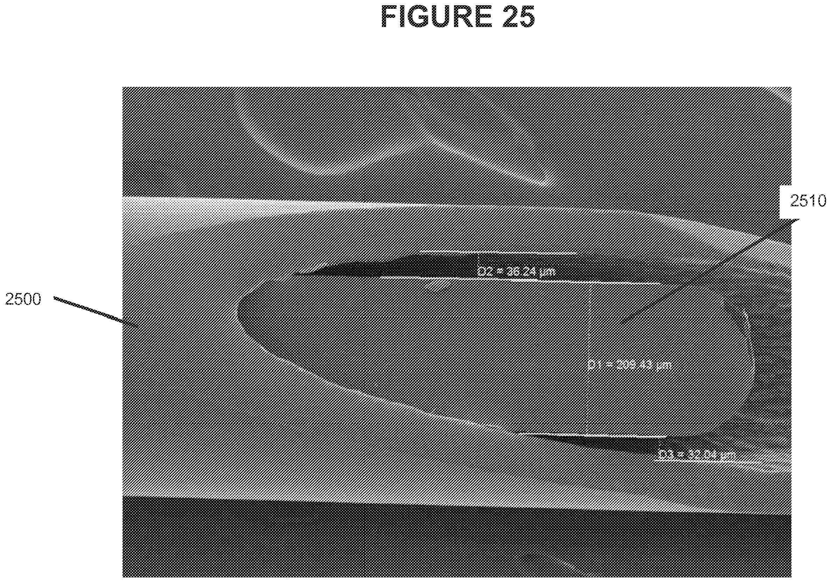

**FIG. 25** shows an image of an implant loaded into a 27 gauge ultra thin walled needle and clearance between the inner diameter of the needle and the implant.

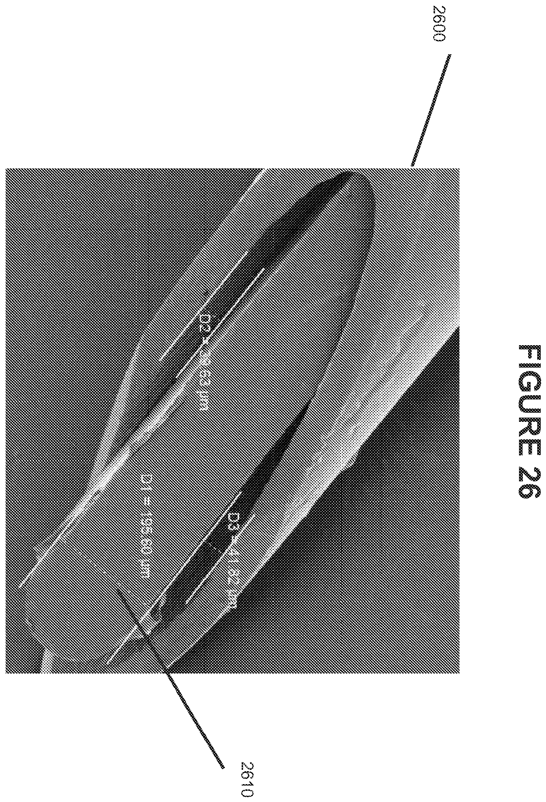

**FIG. 26** shows another image of an implant loaded into a 27 gauge ultra thin walled needle and clearance between the inner diameter of the needle and the implant.

### DETAILED DESCRIPTION

Provided herein are pharmaceutical compositions for treating an ocular condition. In embodiments, the pharmaceutical composition comprises: a biodegradable polymer matrix and a therapeutic agent, which is included in the polymer matrix. In embodiments, the therapeutic agent is dispersed homogeneously throughout the polymer matrix.

As described herein, multiple pharmaceutical compositions have been fabricated and/or contemplated in the form of an implant, resulting in highly effective pharmaceutically active products including ocular therapeutic treatments including sustained release ocular implants.

In various embodiments, these pharmaceutical compositions include a therapeutic agent dispersed throughout a polymer matrix formed into an ocular implant.

In a particular embodiment, the pharmaceutical composition of the present disclosure comprises: i) a biodegradable polymer or blend of biodegradable polymers, and ii) a therapeutic agent such as, for example, a drug effective for use in the treatment of an ocular condition, such as elevated intraocular pressure (IOP).

### Definitions

"About" means plus or minus a percent (e.g., ±5%) of the number, parameter, or characteristic so qualified, which would be understood as appropriate by a skilled artisan to the scientific context in which the term is utilized. Furthermore, since all numbers, values, and expressions referring to quantities used herein, are subject to the various uncertainties of measurement encountered in the art, and then unless otherwise indicated, all presented values may be understood as modified by the term "about."

As used herein, the articles "a," "an," and "the" may include plural referents unless otherwise expressly limited to one-referent, or if it would be obvious to a skilled artisan from the context of the sentence that the article referred to a singular referent.

Where a numerical range is disclosed herein, then such a range is continuous, inclusive of both the minimum and maximum values of the range, as well as every value between such minimum and maximum values. Still further, where a range refers to integers, every integer between the minimum and maximum values of such range is included. In addition, where multiple ranges are provided to describe a feature or characteristic, such ranges can be combined. That is to say that, unless otherwise indicated, all ranges disclosed herein are to be understood to encompass any and all subranges subsumed therein. For example, a stated range of from "1 to 10" should be considered to include any and all subranges between the minimum value of 1 and the maximum value of 10. Exemplary subranges of the range "1 to 10" include, but are not limited to, 1 to 6.1, 3.5 to 7.8, and 5.5 to 10.

As used herein, the term "polymer" is meant to encompass both homopolymers (polymers having only one type of repeating unit) and copolymers (a polymer having more than one type of repeating unit).

"Biodegradable polymer" means a polymer or polymers, which degrade in vivo, under physiological conditions. The release of the therapeutic agent occurs concurrent with, or subsequent to, the degradation of a biodegradable polymer over time.

The terms "biodegradable" and "bioerodible" are used interchangeably herein. A biodegradable polymer may be a homopolymer, a copolymer, or a polymer comprising more than two different polymeric units.

As used herein, the term "polymer matrix" refers to a homogeneous mixture of polymers. In other words, the matrix does not include a mixture wherein one portion thereof is different from the other portion by ingredient, density, and etc. For example, the matrix does not include a composition containing a core and one or more outer layers, nor a composition containing a drug reservoir and one or more portions surrounding the drug reservoir. The mixture of polymers may be of the same type, e.g. two different PLA polymers, or of different types, e.g. PLA polymers combined with PLGA polymers.

"Ocular condition" means a disease, ailment, or condition, which affects or involves the ocular region.

The term "hot-melt extrusion" or "hot-melt extruded" is used herein to describe a process, whereby a blended composition is heated and/or compressed to a molten (or softened) state and subsequently forced through an orifice, where the extruded product (extrudate) is formed into its final shape, in which it solidifies upon cooling.

The term "non-extruded implant" or "non-hot melt extruded implant" refers to an implant that was not manufactured in a process that utilizes an extrusion step, for example, through molding in a mold cavity.

"Sustained release" or "controlled release" refers to the release of at least one therapeutic agent, or drug, from an implant at a sustained rate. Sustained release implies that the therapeutic agent is not released from the implant sporadically, in an unpredictable fashion. The term "sustained release" may include a partial "burst phenomenon" associated with deployment. In some example embodiments, an initial burst of at least one therapeutic agent may be desirable, followed by a more gradual release thereafter. The release rate may be steady state (commonly referred to as "timed release" or zero order kinetics), that is the at least one therapeutic agent is released in even amounts over a predetermined time (with or without an initial burst phase), or may be a gradient release. For example, sustained release can have substantially constant release over a given time period or as compared to topical administration.

"Therapeutically effective amount" means a level or amount of a therapeutic agent needed to treat an ocular condition; the level or amount of a therapeutic agent that produces a therapeutic response or desired effect in the subject to which the therapeutic agent was administered. Thus, a therapeutically effective amount of a therapeutic agent, such as a travoprost, is an amount that is effective in reducing at least one symptom of an ocular condition.

### Ocular Anatomy

In particular embodiments, the implants described herein are intracameral implants manufactured for placement at or into the iridocorneal angle of the human eye.

In these embodiments, the sustained release of therapeutic agent from the implant achieves a concentration of drug in the aqueous humor of the patient's eye that significantly lowers IOP over the period of sustained release. Furthermore, in embodiments, the intracameral implant placed at or into the iridocorneal angle of a patient's eye achieves a drug concentration in the aqueous humor that does not fluctuate below a therapeutic level for any consecutive period of 48 hours or more over the sustained release period of the implant and thus overcomes an inherent problem associated with a topical administration paradigm and prior art implants.

The anterior and posterior chambers of the eye are filled with aqueous humor, a fluid predominantly secreted by the ciliary body with an ionic composition similar to the blood. The function of the aqueous humor is: a) to supply nutrients to the avascular structures of the eye, e.g. the lens and cornea, and b) maintain IOP.

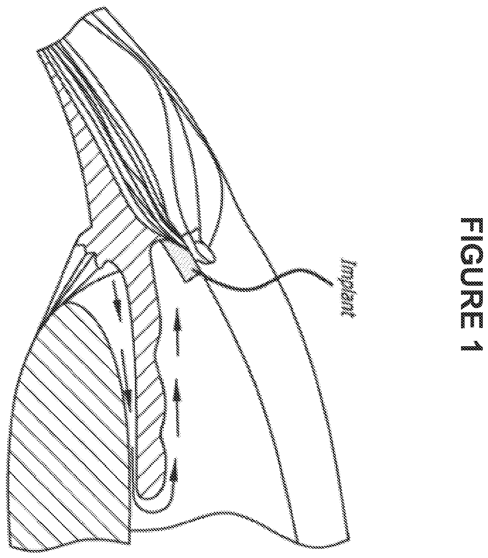

Aqueous humor is predominantly secreted to the posterior chamber of the eye by the ciliary processes of the ciliary body and a minor mechanism of aqueous humor production is through ultrafiltration from arterial blood. Aqueous humor reaches the anterior chamber by crossing the pupil and there are convection currents where the flow of aqueous humor adjacent to the iris is upwards, and the flow of aqueous humor adjacent to the cornea flows downwards **(****FIG. 1****).**

There are two different pathways of aqueous humor outflow, both located in the iridocorneal angle of the eye **(****FIG. 1****).** The uveoscleral, or nonconventional pathway, refers to the aqueous humor leaving the anterior chamber by diffusion through intercellular spaces among ciliary muscle fibers. Although this seems to be a minority outflow pathway in humans, the uveoscleral pathway is the target of specific anti-hypertensive drugs, such as the hypotensive lipids.

The aqueous humor drains 360° into the trabecular meshwork that initially has pore size diameters ranging from 10 to under 30 microns in humans. Aqueous humor drains through Schlemm's canal and exits the eye through 25 to 30 collector channels into the aqueous veins, and eventually into the episcleral vasculature and veins of the orbit.

Therapeutic agent eluting from an implant as described herein enters the aqueous humor of the anterior chamber via convection currents. The therapeutic agent is then dispersed throughout the anterior chamber and enters the target tissues such as the trabecular meshwork and the ciliary body region through the iris root region.

Both in the aforementioned trabecular meshwork and in the uveoscleral tissue, various prostanoid receptors have been found, which indicates that prostanoids are involved in the regulation of aqueous humor production and/or drainage and thereby influence the intraocular pressure. In the trabecular network, genes encoding the EP, FP, IP, DP and TP receptor families are expressed, whereas the EP and FP receptor families are dominant in the uveoscleral tissue (Toris et al., Surv Ophthalmol. 2008; 53, Suppl. 1, S107-S120).

Prostanoids are physiological fatty acid derivatives representing a subclass of eicosanoids. They comprise prostaglandins, prostamides, thromboxanes, and prostacyclins, all of which compounds are mediators involved in numerous physiological processes. Natural prostaglandins such as PGF₂ₐ, PGE₂, PGD₂, and PGI₂ exhibit a particular affinity to their respective receptors (FP, EP, DP, IP), but also have some non-selective affinity for other prostaglandin receptors. Prostaglandins also have direct effects on matrix metalloproteinases. These are neutral proteinases expressed in the trabecular meshwork, which play a role in controlling humor outflow resistance by degrading the extracellular matrix.

Several prostaglandin analogues have been found effective as topically administered medicines in reducing the intraocular pressure, such as latanoprost, bimatoprost, tafluprost, travoprost, and unoprostone. By some experts, bimatoprost is understood as a prostamide rather than prostaglandin derivative. Latanoprost, travoprost, tafluprost, and probably also bimatoprost, are potent and selective PGF₂ₐ agonists. Their net effect is a reduction of intraocular pressure, which is predominantly caused by a substantial increase in aqueous humor drainage, via the uveoscleral pathway. Probably they also increase the trabecular outflow to some degree. Unoprostone is sometimes also classified as a PGF₂ₐ analogue even though its potency and selectivity are much lower than in the case of the previously mentioned compounds. It is most closely related to a pulmonary metabolite of PGF₂ₐ. It is also capable of reducing the intraocular pressure, but appears to act predominantly by stimulating the trabecular drainage pathway, whereas it has little effect on the uveoscleral outflow.

An advantage of injection and intracameral placement of a biodegradable implant described herein is that the anterior chamber is an immune privileged site in the body and less likely to react to foreign material, such as polymeric therapeutic agent delivery systems.

### Biodegradable Polymers

In certain embodiments, the implants described herein are engineered in size, shape, composition, and combinations thereof, to provide maximal approximation of the implant to the iridocorneal angle of a human eye. In certain embodiments, the implants are made of polymeric materials.

In embodiments, the polymer materials used to form the implants described herein are biodegradable. In embodiments, the polymer materials may be any combination of polylactic acid, glycolic acid, and co-polymers thereof that provides sustained-release of the therapeutic agent into the eye over time.

Suitable polymeric materials or compositions for use in the implants include those materials which are compatible, that is biocompatible, with the eye so as to cause no substantial interference with the functioning or physiology of the eye. Such polymeric materials may be biodegradable, bioerodible or both biodegradable and bioerodible.

In particular embodiments, examples of useful polymeric materials include, without limitation, such materials derived from and/or including organic esters and organic ethers, which when degraded result in physiologically acceptable degradation products. Also, polymeric materials derived from and/or including, anhydrides, amides, orthoesters and the like, by themselves or in combination with other monomers, may also find use in the present disclosure. The polymeric materials may be addition or condensation polymers. The polymeric materials may be cross-linked or non-cross-linked,. For some embodiments, besides carbon and hydrogen, the polymers may include at least one of oxygen and nitrogen. The oxygen may be present as oxy, e.g. hydroxy or ether, carbonyl, e.g. non-oxo-carbonyl, such as carboxylic acid ester, and the like. The nitrogen may be present as amide, cyano and amino.

In one embodiment, polymers of hydroxyaliphatic carboxylic acids, either homopolymers or copolymers, and polysaccharides are useful in the implants. Polyesters can include polymers of D-lactic acid, L-lactic acid, racemic lactic acid, glycolic acid, polycaprolactone, co-polymers thereof, and combinations thereof.

Some characteristics of the polymers or polymeric materials for use in embodiments of the present disclosure may include biocompatibility, compatibility with the selected therapeutic agent; ease of use of the polymer in making the therapeutic agent delivery systems described herein, a desired half-life in the physiological environment, and hydrophilicity.

In one embodiment, the biodegradable polymer matrix used to manufacture the implant is a synthetic aliphatic polyester, for example, a polymer of lactic acid and/or glycolic acid, and includes poly-(D,L-lactide) (PLA), poly-(D-lactide), poly-(L-lactide), polyglycolic acid (PGA), and/or the copolymer poly-(D, L-lactide-co-glycolide) (PLGA).

PLGA and PLA polymers are known to degrade via backbone hydrolysis (bulk erosion) and the final degradation products are lactic and glycolic acids, which are non-toxic and considered natural metabolic compounds. Lactic and glycolic acids are eliminated safely via the Krebs cycle by conversion to carbon dioxide and water.

PLGA is synthesized through random ring-opening co-polymerization of the cyclic dimers of glycolic acid and lactic acid. Successive monomeric units of glycolic or lactic acid are linked together by ester linkages. The ratio of lactide to glycolide can be varied, altering the biodegradation characteristics of the product. By altering the ratio it is possible to tailor the polymer degradation time. Importantly, drug release characteristics are affected by the rate of biodegradation, molecular weight, and degree of crystallinity in drug delivery systems. By altering and customizing the biodegradable polymer matrix, the drug delivery profile can be changed.

PLA, PGA, and PLGA are cleaved predominantly by non-enzymatic hydrolysis of its ester linkages throughout the polymer matrix, in the presence of water in the surrounding tissues. PLA, PGA, and PLGA polymers are biocompatible, because they undergo hydrolysis in the body to produce the original monomers, lactic acid and/or glycolic acid. Lactic and glycolic acids are nontoxic and eliminated safely via the Krebs cycle by conversion to carbon dioxide and water. The biocompatibility of PLA, PGA and PLGA polymers has been further examined in both non-ocular and ocular tissues of animals and humans. The findings indicate that the polymers are well tolerated.

Examples of PLA polymers, which may be utilized in an embodiment of the disclosure, include the RESOMER® Product line available from Evonik Industries identified as, but are not limited to, R 207 S, R 202 S, R 202 H, R 203 S, R 203 H, R 205 S, R 208, R 206, and R 104. Examples of suitable PLA polymers include both acid and ester terminated polymers with inherent viscosities ranging from approximately 0.15 to approximately 2.2 dL/g when measured at 0.1% w/v in CHCl₃ at 25°C with an Ubbelhode size 0c glass capillary viscometer.,.

The synthesis of various molecular weights of PLA is possible. In one embodiment, PLA, such as RESOMER® R208S, with an inherent viscosity of approximately 1.8 to approximately 2.2 dl/g, can be used. In another embodiment, PLA, such as RESOMER® R203S, with an inherent viscosity of approximately 0.25 to approximately 0.35 dl/g can be used

Resomer's R203S and R208S are poly(D,L-lactide) or PLA ester-terminated polymers with the general structure (1):

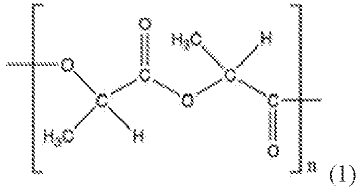

Examples of PLGA polymers, which may be utilized in an embodiment of the disclosure, include the RESOMER® Product line from Evonik Industries identified as, but are not limited to, RG 502, RG 502 H, RG 503, RG 503 H, RG 504, RG 504 H, RG 505, RG 506, RG 653 H, RG 752 H, RG 752 S, RG 753 H, RG 753 S, RG 755, RG 755 S, RG 756, RG 756 S, RG 757 S, RG 750 S, RG 858, and RG 858 S. Such PLGA polymers include both acid and ester terminated polymers with inherent viscosities ranging from approximately 0.14 to approximately 1.7 dl/g when measured at 0.1% w/v in CHCl₃ at 25°C with an Ubbelhode size 0c glass capillary viscometer. Example polymers used in various embodiments of the disclosure may include variation in the mole ratio of D,L-lactide to glycolide from approximately 50:50 to approximately 85:15, including, but not limited to, 50:50, 65:35, 75:25, and 85:15.

The synthesis of various molecular weights of PLGA with various D,L-lactide-glycolide ratios is possible. In one embodiment, PLGA, such as RESOMER® RG752S, with an inherent viscosity of approximately 0.16 to approximately 0.24 dl/g can be used

Resomer RG752S is a poly(D,L-lactide-co-glycolide) or ester-terminated PLGA copolymer (lactide:glycolide ratio of 75:25) with the general structure (2):

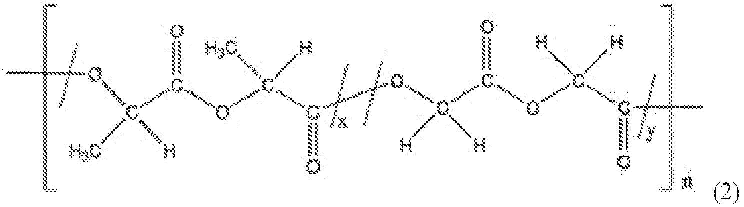

The polymers used to form the implants of the disclosure have independent properties associated with them that when combined provide the properties needed to provide sustained release of a therapeutically effective amount of a therapeutic agent.

A few of the primary polymer characteristics that control therapeutic agent release rates are the molecular weight distribution, polymer endgroup (i.e., acid or ester), and the ratio of polymers and/or copolymers in the polymer matrix. The present disclosure provides examples of polymer matrices that possess desirable therapeutic agent release characteristics by manipulating one or more of the aforementioned properties to develop a suitable ocular implant.

The biodegradable polymeric materials which are included to form the implant's polymeric matrix are often subject to enzymatic or hydrolytic instability. Water soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the implants. Different molecular weights of the same or different polymeric compositions may be included to modulate the release profile of the at least one therapeutic agent.

In an embodiment of the present disclosure, the polymers of the present implants are selected from biodegradable polymers, disclosed herein, that do not substantially swell when in the presence of the aqueous humor. By way of example but not limitation, PLGA polymers swell when used as the matrix material of drug delivery implants whereas PLA based polymer blends do not appreciably swell in the presence of the aqueous humor. Therefore, PLA polymer matrix materials are polymer matrix materials in embodiments of the present disclosure.

### Drug Release Profile Manipulation

The rate of drug release from biodegradable implants depends on several factors. For example, the surface area of the implant, therapeutic agent content, and water solubility of the therapeutic agent, and speed of polymer degradation. For a homopolymer such as PLA, the drug release is also determined by (a) the lactide stereoisomeric composition (i.e. the amound of L- vs. D,L-lactide) and (b) molecular weight. Three additional factors that determine the degradation rate of PLGA copolymers are: (a) the lactide:glycolide ratio, (b) the lactide stereoisomeric composition (*i.e.,* the amount of L- vs. DL-lactide), and (c) molecular weight.

The lactide:glycolide ratio and stereoisomeric composition are generally considered most important for PLGA degradation, as they determine polymer hydrophilicity and crystallinity. For instance, PLGA with a 1:1 ratio of lactic acid to glycolic acid degrades faster than PLA or PGA, and the degradation rate can be decreased by increasing the content of either lactide or glycolide. Polymers with degradation times ranging from weeks to years can be manufactured simply by customizing the lactide:glycolide ratio and lactide stereoisomeric composition.

The versatility of PGA, PLA, and PLGA allows for construction of delivery systems to tailor the drug release for treating a variety of ocular diseases.

When the versatility of PGA, PLA, and PLGA polymers are combined with the manufacturing techniques of the present disclosure, *i.e.* PRINT® technology (Envisia Therapeutics Inc.) particle fabrication, then a host of custom tailored and highly consistent and predictable drug release profiles can be created, which were not possible based upon the technology of the prior art, such as for example extrusion.

That is, with the present mold based particle fabrication technology, implants can be manufactured that exhibit a drug release profile that has highly reproducible characteristics from implant to implant. The drug release profiles exhibited by various implants of the present disclosure are consistent implant to implant and demonstrate variation that is not statistically significant Consequently, the drug release profiles demonstrated by embodiments of the implants exhibit coefficients of variation that are within a confidence interval and does not impact the therapeutic delivery. The ability to produce implants that demonstrate such a high degree of consistent drug loading or release is an advancement over the state of the art.

### Drug Release Kinetics

Drug release from PLA- and PLGA-based polymer matrix drug delivery systems generally follows pseudo first-order or square root kinetics.

Drug release is influenced by many factors including: polymer composition, therapeutic agent content, implant morphology, porosity, tortuosity, surface area, method of manufacture, and deviation from sink conditions, just to name a few. The present mold based manufacturing techniques-utilized in embodiments of the disclosure-are able to manipulate implant morphology, porosity, tortuosity, and surface area in ways that the prior art methods were incapable of doing. For instance, the highly consistent drug release profiles, highly consistent implant morphologies, and highly consistent homogeneous drug dispersions achievable by the present methods, were not available to prior art practitioners relegated to utilizing an extrusion based method of manufacture.

In general, therapeutic agent release occurs in 3 phases: (a) an initial burst release of therapeutic agent from the surface, (b) followed by a period of diffusional release, which is governed by the inherent dissolution of therapeutic agent (diffusion through internal pores into the surrounding media) and lastly, (c) therapeutic agent release associated with biodegradation of the polymer matrix. The rapid achievement of high therapeutic agent concentrations, followed by a longer period of continuous lower-dose release, makes such delivery systems ideally suited for acute-onset diseases that require a loading dose of therapeutic agent followed by tapering doses over a 1-day to 3-month period.

More recent advancements in PLGA-based drug delivery systems have allowed for biphasic release characteristics with an initial high (burst) rate of therapeutic agent release followed by substantially sustained zero-order (linear) kinetic release (*i.e.,* therapeutic agent release rate from the polymer matrix is steady and independent of the therapeutic agent concentration in the surrounding milieu) over longer periods. In addition, when desired for treating chronic diseases such as elevated IOP, these therapeutic agent delivery systems can be designed to have substantially steady state release following zero order kinetics from the onset.

### Therapeutic Agents

Suitable therapeutic agents for use in various embodiments of the disclosure may be found in the Orange Book published by the Food and Drug Administration, which lists therapeutic agents approved for treating ocular diseases including glaucoma and/or lowering IOP.

In some embodiments, the therapeutic agents that can be used according to the disclosure include: prostaglandins, prostaglandin prodrugs, prostaglandin analogues, prostamides, pharmaceutically acceptable salts thereof, and combinations thereof.

Examples include prostaglandin receptor agonists, including prostaglandin E₁ (alprostadil), prostaglandin E₂ (dinoprostone), latanoprost, and travoprost. Latanoprost and travoprost are prostaglandin prodrugs (*i.e.* I-isopropyl esters of a prostaglandin); however, they are referred to as prostaglandins, because they act on the prostaglandin F receptor, after being hydrolyzed to the 1-carboxylic acid.

A prostamide (also called a prostaglandin-ethanolamide) is a prostaglandin analogue, which is pharmacologically unique from a prostaglandin (*i.e.* because prostamides act on a different cell receptor [the prostamide receptor] than do prostaglandins), and is a neutral lipid formed a as product of cyclo-oxygenase-2 ("COX-2") enzyme oxygenation of an endocannabinoid (such as anandamide). Additionally, prostamides do not hydrolyze *in situ* to the 1-carboxylic acid. Examples of prostamides are bimatoprost (the synthetically made ethyl amide of 17-phenyl prostaglandin F_{2α}) and prostamide F_{2α}. Other prostaglandin analogues that can be used as therapeutic agents include, but are not limited to, unoprostone, and EP₂/EP₄ receptor agonists.

Prostaglandins as used herein also include one or more types of prostaglandin derivatives, prostaglandin analogues including prostamides and prostamide derivatives, prodrugs, salts thereof, and mixtures thereof.

Suitable examples of the aforementioned drugs include, but are not limited to, latanoprost, travoprost, bimatoprost, tafluprost, and unoprostone isopropyl.

In one embodiment, the disclosure utilizes travoprost, latanoprost, and bimatoprost. In another embodiment, the disclosure utilizes travoprost and latanoprost.

In a particular embodiment, the disclosure utilizes travoprost. Travoprost has a molecular formula of C₂₆H₃₅F₃O₆ and a molecular weight of 500.548 g/mol.

The chemical structure (3) of travoprost is illustrated below:

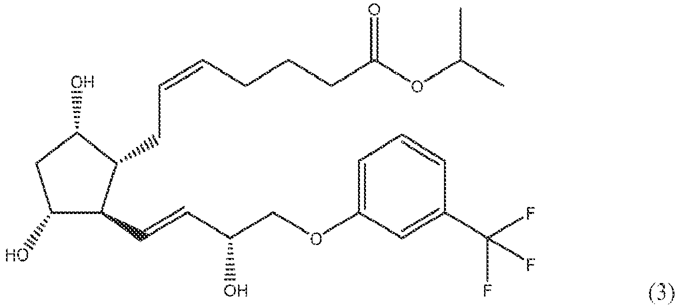

### IUPAC Name: propan-2-yl 7-[3,5-dihydroxy-2-[3-hydroxy-4-[3-(trifluoromethyl) phenoxy]-but-1-enyl]-cyclopentyl]hept-5-enoate

Travoprost, a prostaglandin analogue ester prodrug of the active moiety (+)-fluprostenol, is currently marketed as a 0.004% sterile, preserved, or preservative free, isotonic, multidose ophthalmic solution using well-known excipients. The formulations contain 40 µg of travoprost per mL of solution and is administered as a once a day drop with approximately 1 µg travoprost per day in patients with primary open-angle glaucoma or ocular hypertension to reduce intraocular pressure (TRAVATAN Z®, travoprost ophthalmic solution, Package Insert. Alcon Laboratories, Inc. Fort Worth, TX 2004; and TRAVATAN®, travoprost ophthalmic solution, Package Insert. Alcon Laboratories, Inc. Fort Worth, TX. 2013). Travoprost was first approved by the FDA as topical eye drops in 2001 under the tradename TRAVATAN® and more recently in 2006 under the tradename TRAVATAN Z®.

Travoprost is a synthetic prostaglandin analogue and is an isopropyl ester prodrug of its free-acid active form, a selective and potent full agonist of the prostaglandin FP receptor with an EC₅₀ of 3.2 nM (Sharif NA, Kelly CR, Crider JY. "Agonist Activity of Bimatoprost, Travoprost, Latanoprost, Unoprostone Isopropyl Ester and Other Prostaglandin Analogs at the Cloned Human Ciliary Body FP Prostaglandin Receptor," J Ocul Pharmacol Ther. 2002;18:313-324).

When dosed as topical eye drops, travoprost is hydrolyzed and appears in the aqueous humor as the free acid. Travoprost is believed to lower IOP by enhancing the uveoscleral outflow of aqueous humor and has been studied for this effect in several animal models including monkey, dog, and cat (Gelatt KN, MacKay EO. "Effect of different dose schedules of travoprost on intraocular pressure and pupil size in the glaucomatous Beagle," Vet Ophthalmol. 2004;7(1):53-57; and Bean GW, Camras CB. "Commercially available prostaglandin analogs for the reduction of intraocular pressure: similarities and differences," Surv Ophthalmol. 2008;53 Suppl 1:S69-S84).

In ocular tissues, travoprost is known to rapidly hydrolyze to the free acid. Travoprost free acid is highly potent and selective for the FP receptor and is amongst the most potent in its class. *See, Supra,* Sharif *et al.*

A relative comparison of potency of parent and free acid for different members of the prostaglandin analogue class is presented in **Table 1.**

**Table 1: Agonist Activity of Prostaglandin Analogues at the Cloned Human Ciliary Body FP Prostaglandin Receptor**

| Compound | Functional Potency, EC₅₀ |
|---|---|
| Travoprost acid | EC₅₀ = 3.2 ± 0.6 nM |
| Bimatoprost acid | EC₅₀ = 5.8 ± 2.6 nM |
| Latanoprost acid | EC₅₀ = 54.6 ± 12.4 nM |
| Travoprost | EC₅₀ = 42.3 ± 6.7 nM |
| Bimatoprost | EC₅₀ = 694 ± 293 nM |
| Latanoprost | EC₅₀ = 126 ± 34.2 nM |

### Pharmaceutical Compositions

In embodiments, the pharmaceutical composition is comprised of the biodegradable polymer matrix and at least one therapeutic agent.

The biodegradable polymer matrix is comprised of polymers meeting the desired characteristics. For example, desired characteristics may include a specific therapeutic agent release rate or a specific duration of action. The biodegradable polymer matrix may be comprised of one polymer, two polymers, or many polymers, such as three, four, five polymers, or more polymers.

In some embodiments, the compositions may comprise polymers utilizing the same monomer, such as compositions comprising various poly(D,L-lactide) homopolymers, or compositions comprising various poly(D,L-lactide-co-glycolide) copolymers. However, even if the polymers of the composition utilize the same monomer, the polymers may differ in other characteristics, such as, for example, inherent viscosity or mole ratio of D,L-lactide to glycolide.

In other embodiments, the compositions may comprise polymers utilizing different monomers, such as compositions comprising a poly(D, L-lactide-co-glycolide) copolymer and a poly(D,L-lactide) homopolymer. However, even if the polymers of the compositions utilize different monomers, the polymers may be similar in other characteristics, such as for example, inherent viscosity.

In one embodiment, the pharmaceutical composition comprises a biodegradable polymer matrix and at least one therapeutic agent homogeneously dispersed throughout the polymer matrix. For example, the polymer matrix contains a mixture of polymers comprising an ester end-capped biodegradable poly(D,L-lactide-co-glycolide) copolymer having an inherent viscosity at 25°C in 0.1% w/v CHCl₃ of approximately 0.16 to approximately 0.24 dL/g and an ester end-capped biodegradable poly(D,L-lactide) homopolymer having an inherent viscosity at 25°C in 0.1% w/v CHCl₃ of approximately 0.25 to approximately 0.35 dL/g. In this embodiment, the ratio of D,L-lactide to glycolide can be, for example, approximately 73-77 mole percent D,L-lactide and 23-27 mole percent glycolide. In this embodiment, the ratio of poly(D,L-lactide-co-glycolide) to poly(D,L-lactide) in the polymer matrix can vary from approximately 15:85 to approximately 30:70. Further, the presently discussed pharmaceutical composition comprising a biodegradable polymer matrix and at least one therapeutic agent, may, in certain embodiments, also exclude other polymers. That is, in some embodiments, the aforementioned polymer matrix only includes the poly(D,L-lactide-co-glycolide) copolymer and poly(D,L-lactide) homopolymer described above and no other polymer.

In a second embodiment, the pharmaceutical composition comprises a biodegradable polymer matrix and at least one therapeutic agent homogeneously dispersed throughout the polymer matrix. For example, the polymer matrix contains a mixture of polymers comprising an ester end-capped biodegradable poly(D,L-lactide) homopolymer having an inherent viscosity at 25°C in 0.1% w/v CHCl₃ of approximately 0.25 to approximately 0.35 dL/g and an ester end-capped biodegradable poly(D,L-lactide) homopolymer having an inherent viscosity at 25°C in 0.1% w/v CHCl₃ of approximately 1.8 to approximately 2.2 dL/g. The ratio of the homopolymers in the polymer matrix can vary from approximately 15:85 to approximately 35:65 (lower inherent viscosity to higher inherent viscosity). Further, the presently discussed pharmaceutical composition comprising a biodegradable polymer matrix and at least one therapeutic agent, may, in certain embodiments, also exclude other polymers. That is, in some embodiments, the aforementioned polymer matrix only includes the two poly(D,L-lactide) homopolymers described above and no other polymer.

In an embodiment, the pharmaceutical composition comprises a biodegradable polymer matrix and at least one therapeutic agent homogeneously dispersed throughout the polymer matrix. For example, the polymer matrix contains a mixture of R203S and R208S. The ratio of the homopolymers in the polymer matrix can vary from approximately 15:85 to approximately 35:65 (lower inherent viscosity to higher inherent viscosity). Further, the presently discussed pharmaceutical composition comprising a biodegradable polymer matrix and at least one therapeutic agent, may, in certain embodiments, also exclude other polymers. In an embodiment the polymer matrix only includes R203S and R208S. In an embodiment, the ocular implant comprises: i) the active agent travoprost (33% loading w/w); and ii) a biodegradable polymer matrix comprising: a poly(D,L-lactide) (PLA) blend of R203S (22.11% w/w) and R208S (44.89% w/w) polymers, wherein said ocular implant is molded from a mold cavity having dimensions of 225 µm × 240 µm × 2,925 µm. In another embodiment, the ocular implant comprises: i) the active agent travoprost (34% loading w/w); and ii) a biodegradable polymer matrix comprising: a poly(D,L-lactide) (PLA) blend of R203S (21.78% w/w) and R208S (44.22% w/w) polymers, wherein said ocular implant is molded from a mold cavity having dimensions of 150 µm × 180 µm × 1,500 µm.

The aforementioned mold cavities used to fabricate the ocular implants may vary from the recited dimensions by ± 50 µm, or ± 40 µm, or ± 30 µm, or ± 20 µm, or ± 10 µm, or ± 5 µm, in various aspects.

In embodiments, the therapeutic agent is blended with the biodegradable polymer matrix to form the pharmaceutical composition. The amount of therapeutic agent used in the pharmaceutical composition depends on several factors such as: biodegradable polymer matrix selection, therapeutic agent selection, rate of release, duration of release desired, configuration of pharmaceutical composition, and ocular PK, to name a few.

For example, the therapeutic agent content of the overall implant may comprise approximately 0.1 to approximately 60.0 weight percent of the total implants pharmaceutical composition. In some embodiments, the therapeutic agent comprises approximately 10.0 to approximately 50.0 weight percent of the pharmaceutical composition. In other embodiments, the therapeutic agent comprises approximately 20.0 to approximately 40.0 weight percent of the pharmaceutical composition. In other embodiments, the therapeutic agent comprises approximately 30.0 to approximately 40.0 weight percent of the pharmaceutical composition. In yet other embodiments, the therapeutic agent comprises approximately 30.0 to approximately 35.0 weight percent of the pharmaceutical composition. In yet still other embodiments, the therapeutic agent comprises approximately 30.0 weight percent of the pharmaceutical composition. Or in other embodiments the therapeutic agent comprises approximately 33.0 weight percent of the pharmaceutical composition.

In embodiments, the pharmaceutical composition is prepared by dissolving the polymer or polymers and the therapeutic agent in a suitable solvent to create a homogeneous solution. For example, acetone, alcohol, acetonitrile, tetrahydrofuran, chloroform, and ethyl acetate may be used as solvents. Other solvents known in the art are also contemplated. The solvent is then allowed to evaporate, leaving behind a homogeneous film. The solution can be aseptically filtered prior to evaporation of the solvent.

### Fabrication of an Ocular Implant

Various methods may be used to produce the implants. Methods include, but are not limited to, solvent casting, phase separation, interfacial methods, molding, compression molding, injection molding, extrusion, co-extrusion, heat extrusion, die cutting, heat compression, and combinations thereof. In certain embodiments, the implants are molded, preferably in polymeric molds.

In particular embodiments, the implants of the present disclosure are fabricated through the PRINT® Technology (Liquidia Technologies, Inc.) particle fabrication. In particular, the implants are made by molding the materials intended to make up the implants in mold cavities.

The molds can be polymer-based molds and the mold cavities can be formed into any desired shape and dimension. Uniquely, as the implants are formed in the cavities of the mold, the implants are highly uniform with respect to shape, size, and composition. Due to the consistency among the physical and compositional makeup of each implant of the present pharmaceutical compositions, the pharmaceutical compositions of the present disclosure provide highly uniform release rates and dosing ranges. The methods and materials for fabricating the implants of the present disclosure are further described and disclosed in the Applicant's issued patents and co-pending patent applications, each of which are incorporated herein by reference in their entirety: U.S. Pat. Nos. 8,518,316; 8,444,907; 8,420,124; 8,268,446; 8,263,129; 8,158,728; 8,128,393; 7,976,759; U.S. Pat. Application Publications Nos. 2013-0249138, 2013-0241107, 2013-0228950, 2013-0202729, 2013-0011618, 2013-0256354, 2012-0189728, 2010-0003291, 2009-0165320, 2008-0131692; and pending U.S. Application Nos. 13/852,683 filed March 28, 2013 and 13/950,447 filed July 25, 2013.

The mold cavities can be formed into various shapes and sizes. For example, the cavities may be shaped as a prism, rectangular prism, triangular prism, pyramid, square pyramid, triangular pyramid, cone, cylinder, torus, or rod. The cavities within a mold may have the same shape or may have different shapes. In certain aspects of the disclosure, the shapes of the implants are a cylinder, rectangular prism, or a rod. In a particular embodiment, the implant is a rod.

The mold cavities can be dimensioned from nanometer to micrometer to millimeter dimensions and larger. For certain embodiments of the disclosure, mold cavities are dimensioned in the micrometer and millimeter range. For example, cavities may have a smallest dimension of between approximately 50 nanometers and approximately 750 µm. In some aspects, the smallest mold cavity dimension may be between approximately 100 µm and approximately 300 µm. In other aspects, the smallest mold cavity dimension may be between approximately 125 µm and approximately 250 µm. The mold cavities may also have a largest dimension of between approximately 750 µm and approximately 10,000 µm. In other aspects, the largest mold cavity dimension may be between approximately 1,000 µm and approximately 5000 µm. In other aspects, the largest mold cavity dimension may be between approximately 1,000 µm and approximately 3,500 µm.

In one embodiment, a mold cavity having generally a rod shape with dimensions of 225 µm × 240 µm × 2,925 µm (W × H x L) is utilized to fabricate the implants of the present disclosure.

In another embodiment, a mold cavity having generally a rod shape with dimensions of 150 µm × 180 µm × 1,500 µm (W × H x L) is used to fabricate the implants of the present disclosure.

In a further embodiment, a mold cavity having a rod shape with dimensions of 180 µm × 160 µm × 3,000 µm (W × H x L) is used to fabricate the implants of the present disclosure.

Once fabricated, the implants may remain on an array for storage, or may be harvested immediately for storage and/or utilization. Implants may be fabricated using sterile processes, or may be sterilized after fabrication. Thus, the present disclosure contemplates kits that include a storage array that has fabricated implants attached thereon. These storage array/implant kits provide a convenient method for mass shipping and distribution of the manufactured implants.

In other embodiments, the implants can be fabricated through the application of additive manufacturing techniques. Additive manufacturing, such as disclosed in US published application US 2013/0295212 and the like can be utilized to either make the master template used in the PRINT process, utilized to make the mold used into the PRINT process otherwise disclosed herein or utilized to fabricate the implants directly.

In a particular embodiment, the implants are fabricated through the process of i) dissolving the polymer and active agent in a solvent, for example acetone; ii) casting the solution into a thin film; iii) drying the film; iv) folding the thin film onto itself; v) heating the folded thin film on a substrate to form a substrate; vi) positioning the thin film on the substrate onto a mold having mold cavities; vii) applying pressure, and in some embodiments heat, to the mold-thin film-substrate combination such that the thin film enters the mold cavities; ix) cooling; x) removing the substrate from the mold to provide implants that substantially mimic the size and shape of the mold cavities.

### Delivery Devices

In embodiments, a delivery device may be used to insert the implant into the eye or eyes for treatment of ocular diseases.

Suitable devices can include a needle or needle-like applicator. In some embodiments, the smallest dimension of an implant may range from approximately 50 µm to approximately 750 µm, and therefore a needle or needle-like applicator with a gauge ranging from approximately 22 to approximately 30 may be utilized. The delivery implant may be a syringe with an appropriately sized needle or may be a syringe-like implant with a needle-like applicator. In an embodiment, the device uses a 27 gauge ultra thin wall needle having an inner diameter of 300 +/- 10 micrometers. As shown in **FIG. 24A** implant 2410 is loaded into needle 2400. Implant 2410 of **FIG. 24A** is 150 µm x 150 µm x 1,500 µm and has clearance between implant 2410 and inner diameter of needle 2400 of 50 µm or more in all directions. In another embodiment, shown in **FIG. 24B****,** 27 gauge needle 2450 includes tip 2480 and implant 2460 loaded into the inner opening of needle 2450. Implant 2460 is 225 µm x 225 µm x 2,925 µm and has clearance between implant 2460 and inner diameter of needle 2450 of 30 +/- 10 µm. Importantly, it is found that implant inner diameter clearance less than 10 micrometers causes damage to the implant that can cause delivery of a different drug dosage to patient or other negative events. **FIG. 25** shows another implant 2510 loaded into needle 2500 and having 35 +/- 5 micrometer clearance between the perimeter of implant 2510 and inner diameter of needle 2500. **FIG. 26** shows yet another implant 2610 loaded into needle 2600 and having 40 +/- 2 micrometer clearance between the perimeter of implant 2610 and inner diameter of needle 2600.

Delivery routes include punctual, intravitreal, subconjunctival, lens, intrascleral, fornix, anterior sub-Tenon's, suprachoroidal, posterior sub-Tenon's, subretinal, anterior chamber, and posterior chamber, to name a few.

In embodiments, an implant or implants are delivered to the anterior chamber of a patient's eye to treat glaucoma and/or elevated intraocular pressure.

### Kits

In embodiments, the implant and delivery device may be combined and presented as a kit for use.

The implant may be packaged separately from the delivery device and loaded into the delivery device just prior to use.

Alternatively, the implant may be loaded into the delivery implant prior to packaging. In this case, once the kit is opened, the delivery implant is ready for use.

Components may be sterilized individually and combined into a kit, or may be sterilized after being combined into a kit.

Further, as aforementioned, a kit may include an array with implants bound thereon.

### Use of Ocular Implant for Treatment

In one aspect of the disclosure, there is presented a method of treating glaucoma and/or elevated IOP. The method comprises placing a biodegradable implant in an eye, degrading the implant, releasing a therapeutic agent which is effective to lower IOP, and thereby treating glaucoma and/or ocular hypertension.

In aspects of the disclosure, the eye is that of an animal. For example, a dog, cat, horse, cow (or any agricultural livestock), or human.

### Course of Treatment

Over the course of treatment, the biodegradable polymer matrix degrades releasing the therapeutic agent. Once the therapeutic agent has been completely released, the polymer matrix is expected to be gone. Complete polymer matrix degradation may take longer than the complete release of the therapeutic agent. Polymer matrix degradation may occur at the same rate as the release of the therapeutic agent.

Current treatments for glaucoma and/or elevated intraocular pressure require the patient to place drops in their eyes each day. The pharmaceutical composition of the disclosure is designed for sustained release of an effective amount of therapeutic agent, thus eliminating the need for daily drops.

For example, the pharmaceutical composition may be designed to release an effective amount of therapeutic agent for approximately one month, two months, three months, four months, five months, six months, seven months, eight months, nine months, ten months, eleven months, twelve months, or longer. In aspects, the pharmaceutical composition is designed to release an effective amount of therapeutic agent for one month, two months, three months, four months, five months, or six months. In other aspects, the pharmaceutical composition is designed to release an effective amount of therapeutic agent for three months, four months, five months, or six months.

In an embodiment, the pharmaceutical composition is dosed in a repetitive manner. The dosing regimen provides a second dose of the pharmaceutical composition implants is dosed following the first dose releases its drug cargo. The dosing regimen also provides that a fourth dose of the pharmaceutical composition implants is not dosed until the polymer matrix of the implants of the first dosing are sufficiently degraded. In an embodiment the implant of the first dose fully degrade before the third dosing is administered.

The following non-limiting examples illustrate certain aspects of the present disclosure.

### EXAMPLES

### Example 1: Preparation of Polymer Matrix/Therapeutic Agent Blends

A series of polymer matrix/therapeutic agent blends were prepared prior to fabrication of implants. Acetone was used to dissolve the polymers and therapeutic agent to create a homogeneous mixture. All blends contained travoprost as the therapeutic agent. The resulting solution was aseptically filtered. After filtering, the acetone was evaporated leaving a thin film of homogeneous material. **Table 2** details the composition of the various blends.

**Table 2: Polymer Matrix/Therapeutic Agent Blend Ratios**

| ID | Polymer Matrix Blend | R 208 S (PLA) wt% | R 203 S (PLA) wt % | RG 752 S (PLGA) wt % | Travoprost wt % |
|---|---|---|---|---|---|
| 515-47-11 | R203S/R208S 33%/67% | 47.302 | 23.298 | 0.0 | 29.4 |
| 515-55-8 | RG752S/R203S 30%/70% | 0.0 | 49.42 | 21.18 | 29.4 |
| 515-60-3 | RG752S/R203S 30%/70% | 0.0 | 49.42 | 21.18 | 29.4 |
| 515-60-4 | RG752S/R203S 30%/70% | 0.0 | 49.42 | 21.18 | 29.4 |
| 515-60-5 | R203S/R208S 33%/67% | 47.302 | 23.298 | 0.0 | 29.4 |
| 0003-001-4B | RG752S/R203S 15%/85% | 0.0 | 59.5 | 10.5 | 31.8 |
| 0003-001-6A | R203S/R208S 33%/67% | 54.56 | 13.64 | 0.0 | 31.8 |
| 0003-001-6B | R203S/R208S 33%/67% | 45.694 | 22.506 | 0.0 | 31.8 |
| 0003-001-7A | R203S/R208S 20%/80% | 54.56 | 13.64 | 0.0 | 31.8 |
| 0003-001-8A | R203S/R208S 15%/85% | 57.97 | 10.23 | 0.0 | 31.8 |
| 0003-001-14A | RG752S/R203S 30%/70% | 0.0 | 47.74 | 20.46 | 31.8 |
| 0003-001-14B | RG752S/R203S 30%/70% | 0.0 | 47.74 | 20.46 | 31.8 |
| 515-1-G-14015-A | R203S/R208S 33%/67% | 44.86 | 22.14 | 0.0 | 33.00 |
| 515-3-G-14014-A | R203S/R208S 33%/67% | 44.21 | 21.83 | 0.0 | 33.96 |

### Example 2: Fabrication of Molds

A series of molds of various dimensions were acquired from Liquidia Technologies, Inc, North Carolina.

Molds utilized included: a) a rod shape with dimensions of 225 µm × 225 µm × 2,925 µm, b) a rod shape with dimensions of 150 µm × 150 µm × 1,500 µm, and c) a rod shape with dimensions of 180 µm × 160 nm × 3,000 nm.

### Example 3: Implant Fabrication

A series of implants were fabricated utilizing the polymer matrix/therapeutic agent blends of Example 1 and the molds of Example 2. Under aseptic conditions, a portion of polymer matrix/therapeutic agent blend was spread over a PET sheet and was heated for approximately 30 to 90 seconds until fluid. Once heated, the blend was covered with the mold of Example 2 which had the desired dimensions. Light pressure was applied using a roller to spread the blend over the mold area. The mold/blend laminate was then passed through a commercially available thermal laminator using the parameters in **Table 3** below. The blend flowed into the mold cavities and assumed the shape of the mold cavities. The blend was allowed to cool to room temperature and created individual implants in the mold cavities. The mold was then removed leaving a two-dimensional array of implants resting on the film. Individual implants were removed from the PET film utilizing forceps.

**Table 3: Implant Fabrication Conditions**

| Process Parameter | R 203 S / R 208 S Matrix | RG 752 S / R 203 S Matrix |
|---|---|---|
| Hot Plate Temperature, °C | 120-140 | 40-50 |
| Hot Plate Time, seconds | 30-90 | 30-90 |
| Laminator Temperature, °F | 320-360 | 100-150 |
| Laminator Speed, ft/min | 0.1-1.0 | 0.1-1.0 |
| Laminator Pressure, psi | 60-80 | 60-80 |
| Number of Passes | 5-8 | 1-3 |

**Table 4** details the implants that were produced with the blends of Example 1 and the molds of Example 2 using the fabrication process of Example 3. The same ID used for the blend was also used for the resulting implant.

**Table 4: Implant Configurations**

| ID | Implant Dimensions |
|---|---|
| 515-47-11 | 225 µm X 225 µm X 2,925 µm |
| 515-55-8 | 225 µm X 225 µm X 2,925 µm |
| 515-60-3 | 225 µm X 225 µm X 2,925 µm |
| 515-60-4 | 150 µm X 150 µm X 1,500 µm |
| 515-60-5 | 225 µm X 225 µm X 2,925 µm |
| 0003-001-4B | 150 µm X 150 µm X 1,500 µm |
| 0003-001-6A | 180 µm X 160 µm X 3,000 µm |
| 0003-001-6B | 150 µm X 150 µm X 1,500 µm |
| 0003-001-7A | 180 µm X 160 µm X 3,000 µm |
| 0003-001-8A | 180 µm X 160 µm X 3,000 µm |
| 0003-001-14A | 180 µm X 160 µm X 3,000 µm |
| 0003-001-14B | 150 µm X 150 µm X 1,500 µm |

### Example 4: Analysis of Travoprost Content

Individual implants were placed into 2 mL HPLC vials and acetonitrile was added to achieve an approximate concentration of 200 µg/mL travoprost based on the calculated travoprost content. The solution was diluted with an equal volume of water, reducing the concentration of travoprost to approximately 100 µg/mL in 50/50 v/v acetonitrile/water. The travoprost concentration was determined via detection of travoprost-isopropyl ester using HPLC as described in Example 5 below. A total of ten implants were analyzed for each configuration. **Table 5** details the test results.

**Table 5: Travoprost Content for Implants**

| ID | Average µg | STDEV µg | RSD % | Min µg | Max µg | Range µg |
|---|---|---|---|---|---|---|
| 515-47-11 | 43.3 | 4.1 | 9.5 | 36.8 | 50.8 | 14.0 |
| 515-55-8 | 40.2 | 1.7 | 4.2 | 36.1 | 42.3 | 6.2 |
| 515-60-3 | 30.4 | 7.1 | 23.3 | 19.9 | 37.2 | 17.3 |
| 515-60-4 | 6.4 | 0.3 | 5.4 | 5.8 | 6.8 | 1.0 |
| 515-60-5 | 40.4 | 1.8 | 4.5 | 38.2 | 44.0 | 5.8 |
| 0003-001-4B | 8.0 | 0.3 | 4.3 | 7.6 | 8.9 | 1.3 |
| 0003-001-6A | 25.7 | 2.8 | 10.8 | 23.0 | 32.7 | 9.7 |
| 0003-001-6B | 12.5 | 2.5 | 20.3 | 8.3 | 17.8 | 9.5 |
| 0003-001-7A | 32.3 | 3.0 | 9.2 | 25.0 | 34.6 | 9.6 |
| 0003-001-8A | 24.6 | 2.9 | 12.0 | 19.0 | 29.0 | 10.0 |
| 0003-001-14A | 22.2 | 1.8 | 8.0 | 18.5 | 24.6 | 6.1 |
| 0003-001-14B | 9.9 | 0.4 | 3.9 | 9.3 | 10.4 | 1.1 |

### Example 5: HPLC Method for Determination of Travoprost-isopropyl Ester

Mobile phase A was prepared by combining approximately 900 mL water, approximately 100 mL acetonitrile, and approximately 1 mL trifluoroacetic acid. Mobile phase B was prepared by combining approximately 900 mL acetonitrile, approximately 100 mL water, and approximately 1 mL trifluoroacetic acid. A series of standards were prepared by diluting a USP standard travoprost. The standard was supplied at approximately 0.5 mg/mL in 70/30 water/acetonitrile, so it was diluted using 30/70 acetonitrile/water to achieve dilutions in 50/50 acetonitrile/water. A suitable range for standards was from approximately 0.1 µg/mL to approximately 100 µg/mL.

For analysis conditions, the column was a Waters Symmetry C18, 4.6 X 75 mm, 3.5 µm, flow rate was 1.5 mL per minute, wavelength was 210 nm, temperature was 30°C, injection volume was 80 µL, and the run time was 5 minutes. The gradient is detailed in **Table 6.** The vial prefetch was enabled at 2.95 minutes.

**Table 6: HPLC Gradient for Travoprost Analysis**

| Time, minutes | Mobile Phase A, % | Mobile Phase B, % |
|---|---|---|
| 0 | 40 | 60 |
| 2.2 | 40 | 60 |
| 2.3 | 5 | 95 |
| 2.7 | 5 | 95 |
| 2.8 | 40 | 60 |

To determine therapeutic agent content in an implant, the measured µg/mL determined from the response associated with the standard curve was multiplied by the volume used to dissolve the implant (total volume of acetonitrile and water).

### Example 6: In Vitro Travoprost Release Studies

In vitro release of travoprost was determined for the implants of Example 3. In this study, single implants were placed into 2 mL HPLC vials and were incubated in 0.75 mL of 1X PBS (phosphate buffered saline) with 0.1% w/w Triton X-100 surfactant media at 37°C. At each time point of interest, the media was removed for analysis. The media was then replaced with 0.75 mL of fresh media. The media that was removed was analyzed for travoprost released via the HPLC method of Example 5.

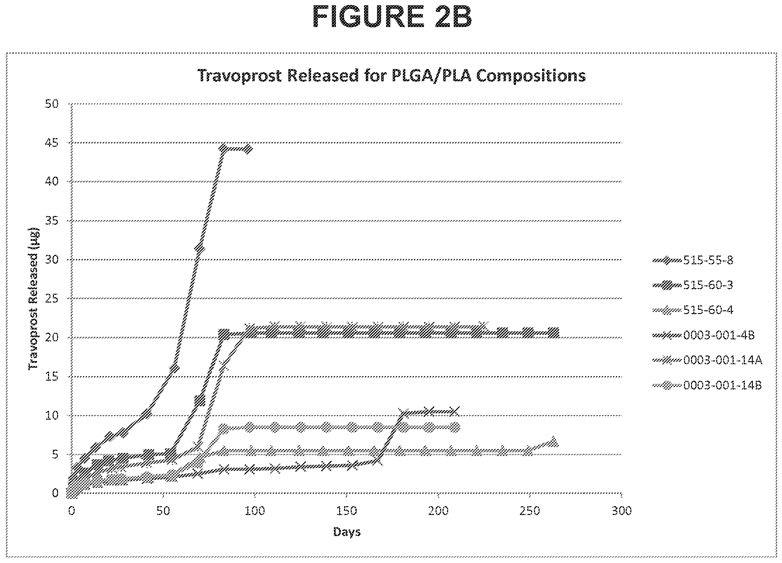

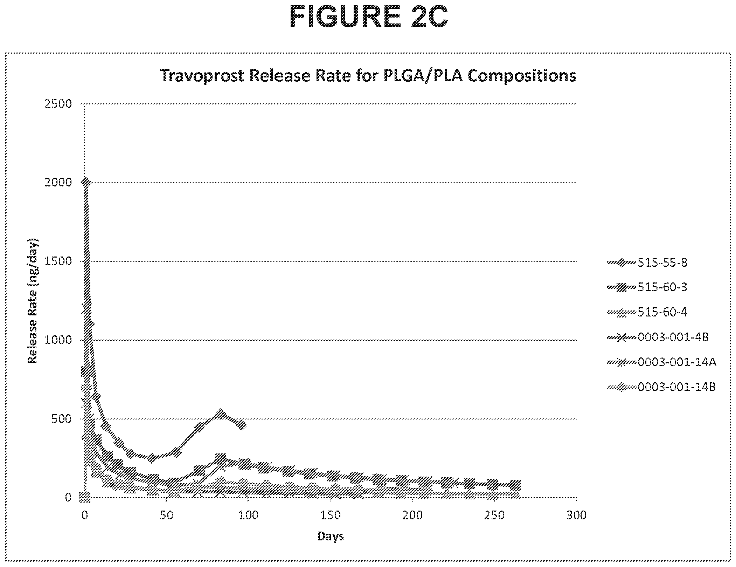

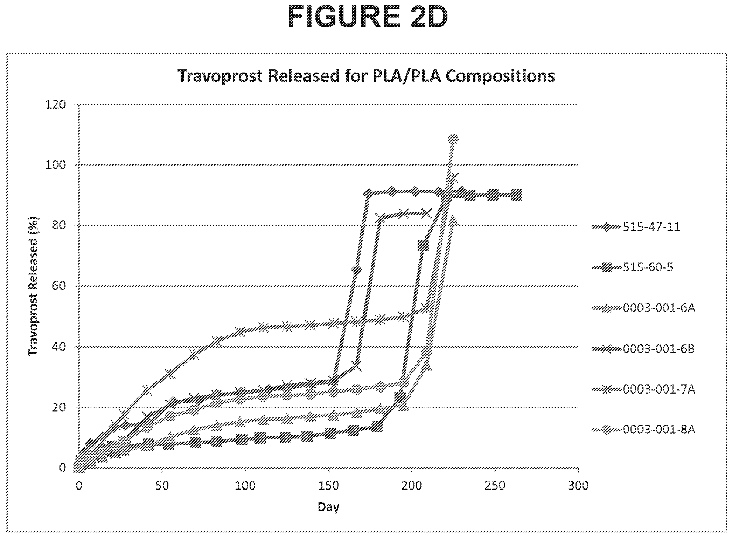

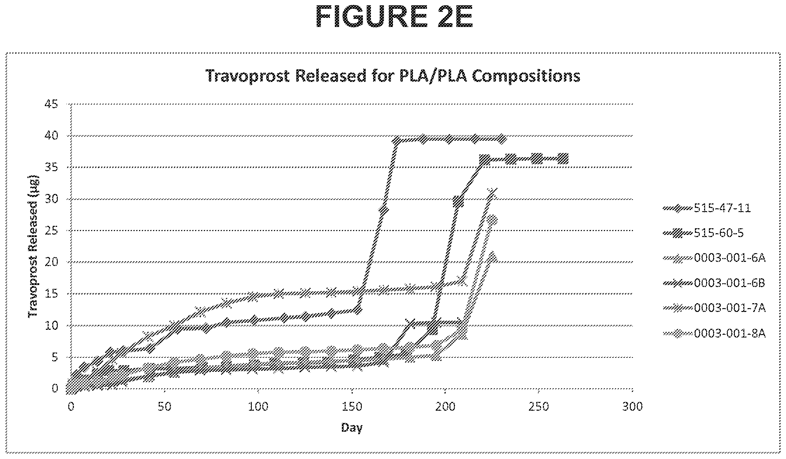

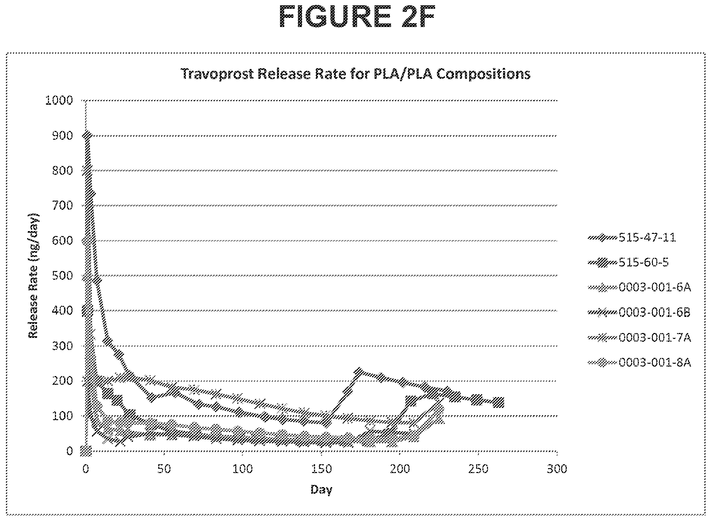

**FIGS. 2A****,** **2B****,** and **2C** detail particular results from the study for implants utilizing a PLGA/PLA polymer matrix. **FIGS. 2D****,** **2E****,** and **2F** detail particular results from the study for implants utilizing a PLA/PLA polymer matrix.

**FIG. 2A** shows the percent travoprost released (%) as a function of time (days) **FIG. 2B** shows the amount of travoprost released (µg) as a function of time (days) **FIG. 2C** shows the release rate of travoprost (ng/day) as a function of time (days).

Further details of the study is shown in **FIGS. 2D****,** **2E****,** and **2F****.**

**FIG. 2D** shows the percent of travoprost released (%) as a function of time (days). **FIG. 2E** shows the amount of travoprost released (µg) over time (days). **FIG. 2F** shows the release rate of travoprost (ng/day) as a function of time (days) .

The data demonstrates that for the PLA/PLGA blends under investigation, for example 0003-001-14A and 0003-001-14B, a smaller implant releases the therapeutic agent more rapidly on a percentage basis when compared to a larger implant. Also, the data demonstrates that as the PLGA content increases, the rate of release of the therapeutic agent is increased (see, e.g., 0003-001-4B and 0003-001-14B). Comparing the PLA/PLGA matrix to the PLA/PLA matrix, for the same approximate therapeutic agent load and the same implant configuration, the PLA/PLGA matrix releases the therapeutic agent more rapidly (see, e.g., 0003-001-6A and 0003-001-14A). The data demonstrates that the release rate for the therapeutic agent can be adjusted.

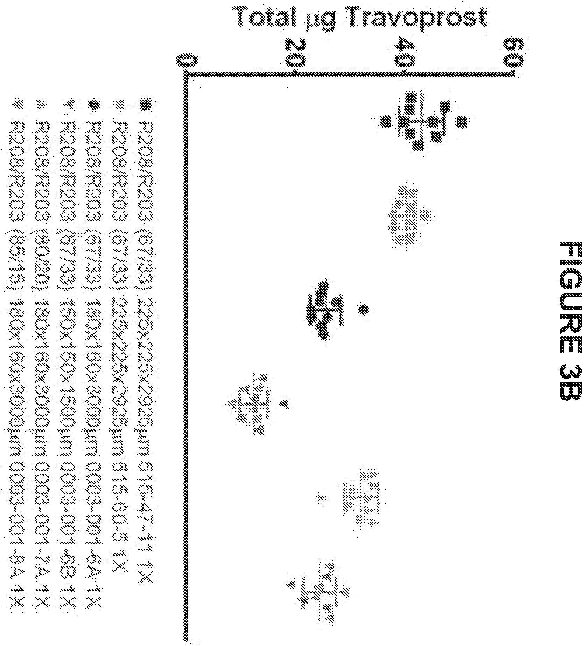

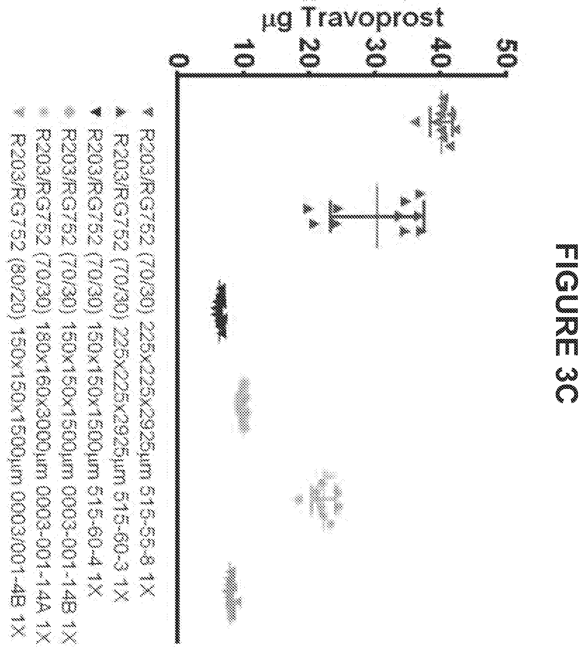

Further details of the study can be gained from **FIGS. 3A****,** **3B****,** and **3C****.**

**FIGS. 3A-3C** shows the therapeutic agent dose content uniformity graph of the implants from previous **FIGS. 2A-2F****.** The data in **FIG. 3A** demonstrates the highly consistent therapeutic agent content (low variability) achievable with the implants of the disclosure. **FIGS. 3B** and **3C** demonstrate the therapeutic agent content uniformity graphs for other select implants. These graphs demonstrate the high degree of therapeutic agent content consistency of the present implants across both implant size and polymer matrix composition.

Also, **Table 7** illustrates the travoprost released (µg), % travoprost released, and travoprost release rate (ng/day) in vitro over a 263 day period for select implants. Further still, **Table 8** illustrates the travoprost releases (µg), % travoprost released, and travoprost release rate (ng/day) in vitro for select implants over a 263 day period.

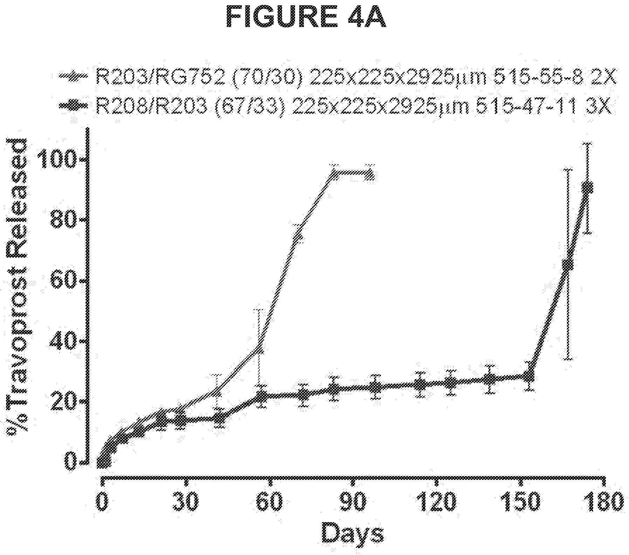

Further release rate data can be ascertained from **FIGS. 4A-4C****.**

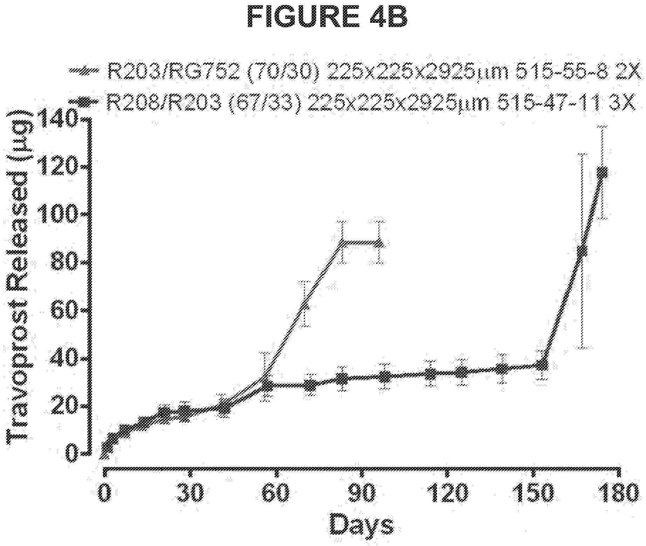

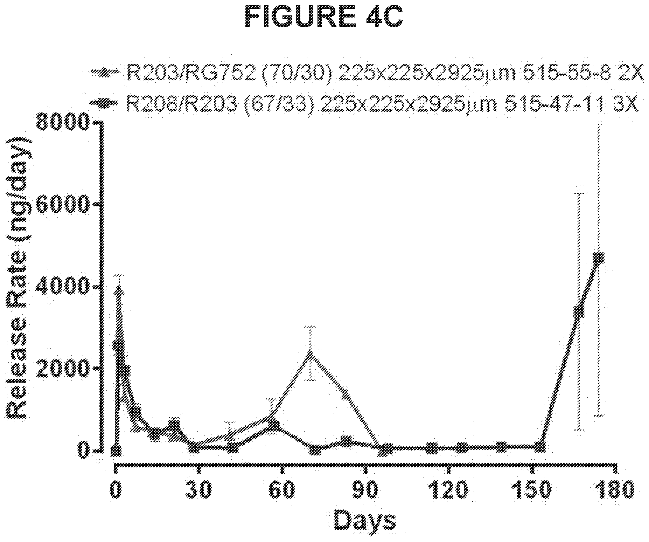

**FIG. 4A** demonstrates the in vitro % travoprost released over time of implants 515-55-8 (2 implants) and 515-47-11 (3 implants) over a 180 day period. **FIG. 4B** demonstrates the in vitro cumulative travoprost released over time of implants 515-55-8 (2 implants) and 515-47-11 (3 implants) over a 180 day period. **FIG. 4C** demonstrates the in vitro travoprost release rate over time of implants 515-55-8 (2 implants) and 515-47-11 (3 implants) over a 180 day period. It is apparent that the R203S/RG752S polymer matrix of the 515-55-8 implants achieves drug release much more rapidly than the R208S/R203S 515-47-11 implants.

**Table 7: Travoprost Released In Vitro Over a 263 Day Period**

| | | | | | | | | |
|---|---|---|---|---|---|---|---|---|
| 515-55-8 | T=0 ug | Day | 0 | 1 | 3 | 7 | 13 | 21 |
| | 40.2 | ug | 0.0 | 2.0 | 3.3 | 4.5 | 5.9 | 7.3 |
| | | % | 0.0% | 5.0% | 8.2% | 11.2% | 14.7% | 18.2% |
| | | ng/day | NA | 2000.0 | 1100.0 | 642.9 | 453.8 | 347.6 |
| 515-60-3 | T=0 ug | Day | 0 | 1 | 3 | 7 | 14 | 20 |
| | 30.4 | ug | 0.0 | 0.8 | 1.4 | 2.6 | 3.7 | 4.2 |
| | | % | 0.0% | 2.6% | 4.6% | 8.6% | 12.2% | 13.8% |
| | | n/day | NA | 800.0 | 466.7 | 371.4 | 264.3 | 210.0 |
| 515-60-4 | T=0 ug | Day | 0 | 1 | 3 | 7 | 14 | 20 |
| | 6.4 | ug | 0.0 | 0.4 | 0.7 | 1.1 | 1.4 | 1.7 |
| | | % | 0.0% | 6.3% | 10.9% | 17.2% | 21.9% | 26.6% |
| | | ng/day | NA | 400.0 | 233.3 | 157.1 | 100.0 | 85.0 |
| 515-001-4B | T=0 ug | Day | 0 | 1 | 3 | 7 | 14 | 22 |
| | 8.0 | ug | 0.0 | 0.6 | 1.0 | 1.2 | 1.4 | 1.6 |
| | | % | 0.0% | 7.5% | 12.5% | 15.0% | 17.5% | 20.0% |
| | | ng/day | NA | 600.0 | 333.3 | 171.4 | 100.0 | 72.7 |
| 0003-001-14A | T=0 ug | Day | 0 | 1 | 3 | 7 | 14 | 22 |
| | 22.2 | ug | 0.0 | 1.2 | 1.5 | 2.0 | 2.7 | 3.2 |
| | | % | 0.0% | 5.4% | 6.8% | 9.0% | 12.2% | 14.4% |
| | | ng/day | NA | 1200.0 | 500.0 | 285.7 | 192.9 | 145.5 |
| 0003-001-14B | T=0 ug | Day | 0 | 1 | 3 | 7 | 14 | 22 |
| | 9.9 | ug | 0.0 | 0.7 | 1.0 | 1.3 | 1.6 | 1.8 |
| | | % | 0.0% | 7.1% | 10.1% | 13.1% | 16.2% | 18.2% |
| | | ng/day | NA | 700.0 | 333.3 | 185.7 | 114.3 | 81.8 |
| 515-55-8 | T=0 ug | Day | 28 | 41 | 56 | 70 | 83 | 96 |
| | 40.2 | ug | 7.8 | 10.2 | 16.1 | 31.4 | 44.2 | 44.2 |
| | | % | 19.4% | 25.4% | 40.0% | 78.1% | 110.0% | 110.0% |
| | | ng/day | 278.6 | 248.8 | 287.5 | 448.6 | 532.5 | 460.4 |
| 515-60-3 | T=0 ug | Day | 28 | 42 | 54 | 70 | 83 | 98 |
| | 30.4 | ug | 4.5 | 4.9 | 5.0 | 11.9 | 20.4 | 20.6 |
| | | % | 14.8% | 16.1% | 16.4% | 39.1% | 67.1% | 67.8% |
| | | ng/day | 160.7 | 116.7 | 92.6 | 170.0 | 245.8 | 210.2 |
| 515-60-4 | T=0 ug | Day | 28 | 42 | 54 | 70 | 83 | 98 |
| | 6.4 | ug | 1.7 | 2.0 | 2.2 | 4.6 | 5.5 | 5.5 |
| | | % | 26.6% | 31.3% | 34.4% | 71.9% | 85.9% | 85.9% |
| | | ng/day | 60.7 | 47.6 | 40.7 | 65.7 | 66.3 | 56.1 |
| 515-001-4B | T=0 ug | Day | 27 | 41 | 55 | 69 | 83 | 97 |
| | 8.0 | ug | 1.7 | 1.9 | 2.1 | 2.5 | 3.1 | 3.1 |
| | | % | 21.3% | 23.8% | 26.3% | 31.3% | 38.8% | 38.8% |
| | | ng/day | 63.0 | 46.3 | 38.2 | 36.2 | 37.3 | 32.0 |
| 0003-001-14A | T=0 ug | Day | 27 | 41 | 55 | 69 | 83 | 97 |
| | 22.2 | ug | 3.4 | 3.9 | 4.3 | 6.0 | 16.4 | 21.2 |
| | | % | 15.3% | 17.6% | 19.4% | 27.0% | 73.9% | 95.5% |
| | | ng/day | 125.9 | 95.1 | 78.2 | 87.0 | 197.6 | 218.6 |
| 0003-001-14B | T=0 ug | Day | 27 | 41 | 55 | 69 | 83 | 97 |
| | 9.9 | ug | 1.9 | 2.1 | 2.4 | 3.9 | 8.3 | 8.5 |
| | | % | 19.2% | 21.2% | 24.2% | 39.4% | 83.8% | 85.9% |
| | | ng/day | 70.4 | 51.2 | 43.6 | 56.5 | 100.0 | 87.6 |
| 515-60-3 | T=0 ug | Day | 109 | 124 | 137 | 151 | 165 | 179 |
| | 30.4 | ug | 20.6 | 20.6 | 20.6 | 20.6 | 20.6 | 20.6 |
| | | % | 67.8% | 67.8% | 67.8% | 67.8% | 67.8% | 67.8% |
| | | ng/day | 189.0 | 166.1 | 150.4 | 136.4 | 124.8 | 115.1 |
| 515-60-4 | T=0 ug | Day | 109 | 124 | 137 | 151 | 165 | 179 |
| | 6.4 | ug | 5.5 | 5.5 | 5.5 | 5.5 | 5.5 | 5.5 |
| | | % | 85.9% | 85.9% | 85.9% | 85.9% | 85.9% | 85.9% |
| | | ng/day | 50.5 | 44.4 | 40.1 | 36.4 | 33.3 | 30.7 |
| 515-001-4B | T=0 ug | Day | 111 | 125 | 139 | 153 | 167 | 181 |
| | 8.0 | ug | 3.2 | 3.4 | 3.5 | 3.6 | 4.2 | 10.3 |
| | | % | 40.0% | 42.5% | 43.8% | 45.0% | 52.5% | 128.8% |
| | | ng/day | 28.8 | 27.2 | 25.2 | 23.5 | 25.1 | 56.9 |
| 0003-001-14A | T=0 ug | Day | 111 | 125 | 139 | 153 | 167 | 181 |
| | 22.2 | ug | 21.4 | 21.4 | 21.4 | 21.4 | 21.4 | 21.4 |
| | | % | 96.4% | 96.4% | 96.4% | 96.4% | 96.4% | 96.4% |
| | | ng/day | 192.8 | 171.2 | 154.0 | 139.9 | 128.1 | 118.2 |
| 0003-001-14B | T=0 ug | Day | 111 | 125 | 139 | 153 | 167 | 181 |
| | 9.9 | ug | 8.5 | 8.5 | 8.5 | 8.5 | 8.5 | 8.5 |
| | | % | 85.9% | 85.9% | 85.9% | 85.9% | 85.9% | 85.9% |
| | | ng/day | 76.6 | 68.0 | 61.2 | 55.6 | 50.9 | 47.0 |
| 515-60-3 | T=0 ug | Day | 193 | 207 | 221 | 235 | 249 | 263 |
| | 30.4 | ug | 20.6 | 20.6 | 20.6 | 20.6 | 20.6 | 20.6 |
| | | % | 67.8% | 67.8% | 67.8% | 67.8% | 67.8% | 67.8% |
| | | ng/day | 106.7 | 99.5 | 93.2 | 87.7 | 82.7 | 78.3 |
| 515-60-4 | T=0 ug | Day | 193 | 207 | 221 | 235 | 249 | 263 |
| | 6.4 | ug | 5.5 | 5.5 | 5.5 | 5.5 | 5.5 | 6.7 |
| | | % | 85.9% | 85.9% | 85.9% | 85.9% | 85.9% | 104.7% |
| | | ng/day | 28.5 | 26.6 | 24.9 | 23.4 | 22.1 | 25.5 |
| 515-001-4B | T=0 ug | Day | 195 | 209 | | | | |
| | 8.0 | ug | 10.5 | 10.5 | | | | |
| | | % | 131.3% | 131.3% | | | | |
| | | ng/day | 53.8 | 50.2 | | | | |
| 0003-001-14A | T=0 ug | Day | 195 | 209 | 225 | | | |
| | 22.2 | ug | 21.4 | 21.4 | 21.4 | | | |
| | | % | 96.4% | 96.4% | 96.4% | | | |
| | | ng/day | 109.7 | 102.4 | 95.1 | | | |
| 0003-001-14B | T=0 ug | Day | 195 | 209 | | | | |
| | 9.9 | ug | 8.5 | 8.5 | | | | |
| | | % | 85.9% | 85.9% | | | | |
| | | ng/day | 43.6 | 40.7 | | | | |

**Table 8: Travoprost Released In Vitro Over a 263 Day Period**

| | | | | | | | | |
|---|---|---|---|---|---|---|---|---|
| 515-47-11 | T=0 ug | Day | 0 | 1 | 3 | 7 | 14 | 21 |
| | 43.3 | ug | 0.0 | 0.9 | 2.2 | 3.4 | 4.4 | 5.8 |
| | | % | 0.0% | 2.1% | 5.1% | 7.9% | 10.2% | 13.4% |
| | | ng/day | NA | 900.0 | 733.3 | 485.7 | 314.3 | 276.2 |
| 515-60-5 | T=0 ug | Day | 0 | 1 | 3 | 7 | 14 | 20 |
| | 40.4 | ug | 0.0 | 0.4 | 0.6 | 1.4 | 2.3 | 2.9 |
| | | % | 0.0% | 1.0% | 1.5% | 3.5% | 5.7% | 7.2% |
| | | ng/day | NA | 400.0 | 200.0 | 200.0 | 164.3 | 145.0 |
| 0003-001-6A | T=0 ug | Day | 0 | 1 | 3 | 7 | 14 | 22 |
| | 25.7 | ug | 0.0 | 0.5 | 0.6 | 0.6 | 0.9 | 1.3 |
| | | % | 0.0% | 1.9% | 2.3% | 2.3% | 3.5% | 5.1% |
| | | ng/day | NA | 500.0 | 200.0 | 85.7 | 64.3 | 59.1 |
| 0003-001-6B | T=0 ug | Day | 0 | 1 | 3 | 7 | 14 | 22 |
| | 12.5 | ug | 0.0 | 0.2 | 0.3 | 0.4 | 0.5 | 0.6 |
| | | % | 0.0% | 1.6% | 2.4% | 3.2% | 4.0% | 4.8% |
| | | ng/day | NA | 200.0 | 100.0 | 57.1 | 35.7 | 27.3 |
| 0003-001-7A | T=0 ug | Day | 0 | 1 | 3 | 7 | 14 | 22 |
| | 32.3 | ug | 0.0 | 0.8 | 1.0 | 1.4 | 2.8 | 4.6 |
| | | % | 0.0% | 2.5% | 3.1% | 4.3% | 8.7% | 14.2% |
| | | ng/day | NA | 800.0 | 333.3 | 200.0 | 200.0 | 209.1 |
| 0003-001-8A | T=0 ug | Day | 0 | 1 | 3 | 7 | 14 | 22 |
| | 24.6 | ug | 0.0 | 0.6 | 0.8 | 0.9 | 1.2 | 1.8 |
| | | % | 0.0% | 2.4% | 3.3% | 3.7% | 4.9% | 7.3% |
| | | ng/day | NA | 600.0 | 266.7 | 128.6 | 85.7 | 81.8 |
| 515-47-11 | T=0 ug | Day | 28 | 42 | 57 | 72 | 83 | 98 |
| | 43.3 | ug | 6.0 | 6.4 | 9.5 | 9.6 | 10.5 | 10.8 |
| | | % | 13.9% | 14.8% | 21.9% | 22.2% | 24.2% | 24.9% |
| | | ng/day | 214.3 | 152.4 | 166.7 | 133.3 | 126.5 | 110.2 |
| 515-60-5 | T=0 ug | Day | 28 | 42 | 54 | 70 | 83 | 98 |
| | 40.4 | ug | 2.9 | 3.2 | 3.2 | 3.4 | 3.5 | 3.8 |
| | | % | 7.2% | 7.9% | 7.9% | 8.4% | 8.7% | 9.4% |
| | | ng/day | 103.6 | 76.2 | 59.3 | 48.6 | 42.2 | 38.8 |
| 0003-001-6A | T=0 ug | Day | 27 | 41 | 55 | 69 | 83 | 97 |
| | 25.7 | ug | 1.5 | 1.9 | 2.6 | 3.2 | 3.6 | 3.9 |
| | | % | 5.8% | 7.4% | 10.1% | 12.5% | 14.0% | 15.2% |
| | | ng/day | 55.6 | 46.3 | 47.3 | 46.4 | 43.4 | 40.2 |
| 0003-001-6B | T=0 ug | Day | 27 | 41 | 55 | 69 | 83 | 97 |
| | 12.5 | ug | 1.1 | 2.1 | 2.6 | 2.9 | 3.0 | 3.1 |
| | | % | 8.8% | 16.8% | 20.8% | 23.2% | 24.0% | 24.8% |
| | | ng/day | 40.7 | 51.2 | 47.3 | 42.0 | 36.1 | 32.0 |
| 0003-001-7A | T=0 ug | Day | 27 | 41 | 55 | 69 | 83 | 97 |
| | 32.3 | ug | 5.7 | 8.3 | 10.0 | 12.1 | 13.5 | 14.5 |
| | | % | 17.6% | 25.7% | 31.0% | 37.5% | 41.8% | 44.9% |
| | | ng/day | 211.1 | 202.4 | 181.8 | 175.4 | 162.7 | 149.5 |
| 0003-001-8A | T=0 ug | Day | 27 | 41 | 55 | 69 | 83 | 97 |
| | 24.6 | ug | 2.2 | 3.3 | 4.2 | 4.7 | 5.2 | 5.6 |
| | | % | 8.9% | 13.4% | 17.1% | 19.1% | 21.1% | 22.8% |
| | | ng/day | 81.5 | 80.5 | 76.4 | 68.1 | 62.7 | 57.7 |
| 515-47-11 | T=0 ug | Day | 114 | 125 | 139 | 153 | 167 | 174 |
| | 43.3 | ug | 11.2 | 11.4 | 11.9 | 12.4 | 28.3 | 39.2 |
| | | % | 25.9% | 26.3% | 27.5% | 28.6% | 65.4% | 90.5% |
| | | ng/day | 98.2 | 91.2 | 85.6 | 81.0 | 169.5 | 225.3 |
| 515-60-5 | T=0 ug | Day | 109 | 124 | 137 | 151 | 165 | 179 |
| | 40.4 | ug | 4.0 | 4.1 | 4.2 | 4.6 | 5.0 | 5.5 |
| | | % | 9.9% | 10.1% | 10.4% | 11.4% | 12.4% | 13.6% |
| | | ng/day | 36.7 | 33.1 | 30.7 | 30.5 | 30.3 | 30.7 |
| 0003-001-6A | T=0 ug | Day | 111 | 125 | 139 | 153 | 167 | 181 |
| | 25.7 | ug | 4.1 | 4.2 | 4.4 | 4.5 | 4.7 | 5.0 |
| | | % | 16.0% | 16.3% | 17.1% | 17.5% | 18.3% | 19.5% |
| | | ng/day | 36.9 | 33.6 | 31.7 | 29.4 | 28.1 | 27.6 |
| 0003-001-6B | T=0 ug | Day | 111 | 125 | 139 | 153 | 167 | 181 |
| | 12.5 | ug | 3.2 | 3.4 | 3.5 | 3.6 | 4.2 | 10.3 |
| | | % | 25.6% | 27.2% | 28.0% | 28.8% | 33.6% | 82.4% |
| | | ng/day | 28.8 | 27.2 | 25.2 | 23.5 | 25.1 | 56.9 |
| 0003-001-7A | T=0 ug | Day | 111 | 125 | 139 | 153 | 167 | 181 |
| | 32.3 | ug | 15.0 | 15.1 | 15.2 | 15.4 | 15.6 | 15.8 |
| | | % | 46.4% | 46.7% | 47.1% | 47.7% | 48.3% | 48.9% |
| | | ng/day | 135.1 | 120.8 | 109.4 | 100.7 | 93.4 | 87.3 |
| 0003-001-8A | T=0 ug | Day | 111 | 125 | 139 | 153 | 167 | 181 |
| | 24.6 | ug | 5.8 | 5.9 | 6.0 | 6.2 | 6.4 | 6.6 |
| | | % | 23.6% | 24.0% | 24.4% | 25.2% | 26.0% | 26.8% |
| | | ng/day | 52.3 | 47.2 | 43.2 | 40.5 | 38.3 | 36.5 |
| 515-47-11 | T=0 ug | Day | 188 | 202 | 216 | 230 | | |
| | 43.3 | ug | 39.5 | 39.5 | 39.5 | 39.5 | | |
| | | % | 91.2% | 91.2% | 91.2% | 91.2% | | |
| | | ng/day | 210.1 | 195.5 | 182.9 | 171.7 | | |
| 515-60-5 | T=0 ug | Day | 193 | 207 | 221 | 235 | 249 | 263 |
| | 40.4 | ug | 9.4 | 29.6 | 36.2 | 36.3 | 36.4 | 36.4 |
| | | % | 23.3% | 73.3% | 89.6% | 89.9% | 90.1% | 90.1% |
| | | ng/day | 48.7 | 143.0 | 163.8 | 154.5 | 146.2 | 138.4 |
| 0003-001-6A | T=0 ug | Day | 195 | 209 | 225 | | | |
| | 25.7 | ug | 5.3 | 8.7 | 21.0 | | | |
| | | % | 20.6% | 33.9% | 81.7% | | | |
| | | ng/day | 27.2 | 41.6 | 93.3 | | | |
| 0003-001-6B | T=0 ug | Day | 195 | 209 | | | | |
| | 12.5 | ug | 10.5 | 10.5 | | | | |
| | | % | 84.0% | 84.0% | | | | |
| | | ng/day | 53.8 | 50.2 | | | | |
| 0003-001-7A | T=0 ug | Day | 195 | 209 | 225 | | | |
| | 32.3 | ug | 16.1 | 17.0 | 30.9 | | | |
| | | % | 49.8% | 52.6% | 95.7% | | | |
| | | ng/day | 82.6 | 81.3 | 137.3 | | | |
| 0003-001-8A | T=0 ug | Day | 195 | 209 | 225 | | | |
| | 24.6 | ug | 6.9 | 9.4 | 26.7 | | | |
| | | % | 28.0% | 38.2% | 108.5% | | | |
| | | ng/day | 35.4 | 45.0 | 118.7 | | | |

### Example 7: Implants Utilized for In Vivo Studies

A series of in vivo studies were conducted to determine the effect on intraocular pressure. **Table 9** details the IDs of the implants, number of implants utilized for dosing, and number of eyes dosed for the studies. Tables in the previous examples provide information as to composition and dimension for the implants.

**Table 9: In Vivo Implants**

| ID | Number of Implants Dosed | Number of Eyes Dosed | Total Travoprost Dose µg | Average Travoprost Content Per Implant µg | Study |
|---|---|---|---|---|---|
| 515-47-11 | 3 | 6 | 130 | 43.3 | PRE004 |
| 515-55-8 | 2 | 6 | 80 | 40.2 | PRE004 |
| 515-60-3 | 1 and 3 (2 test cases) | 4 per test case | 30.4 and 91.2 | 30.4 | PRE006 |
| 515-60-4 | 3 | 4 | 19 | 6.4 | PRE006 |
| 515-60-5 | 1 and 3 (2 test cases) | 6 per test case | 40.4 and 121 | 40.4 | PRE006 |
| 0003-001-4B | 1 and 3 (2 test cases) | 4 per test case | 8 and 24 | 8.0 | PRE009 |
| 0003-001-6A | 1 and 3 (2 test cases) | 4 per test case | 25.7 and 77.1 | 25.7 | PRE009 |
| 0003-001-6B | 1 and 3 (2 test cases) | 4 per test case | 12.5 and 37.5 | 12.5 | PRE009 |
| 0003-001-7A | 1 | 4 | 32.3 | 32.3 | PRE009 |
| 0003-001-8A | 1 and 3 (2 test cases) | 4 per test case | 24.6 and 73.8 | 24.6 | PRE009 |
| 0003-001-14A | 1 and 3 (2 test cases) | 4 per test case | 22.2 and 66.6 | 22.2 | PRE009 |
| 0003-001-14B | 1 and 3 (2 test cases) | 4 per test case | 9.9 and 29.7 | 9.9 | PRE009 |

### Example 8: In Vivo Studies in Normotensive Beagle Dogs

Three separate non-GLP studies were conducted to evaluate various pharmaceutical compositions. Purebred normotensive beagle dogs were utilized in each study. The implant(s) were inserted into the anterior chamber utilizing an appropriately sized needle ranging from 22-gauge to 27-gauge. The intraocular pressure was monitored periodically. The number of implants was varied and was one, two, or three implants.

### Example 8A: In Vivo Study PRE004

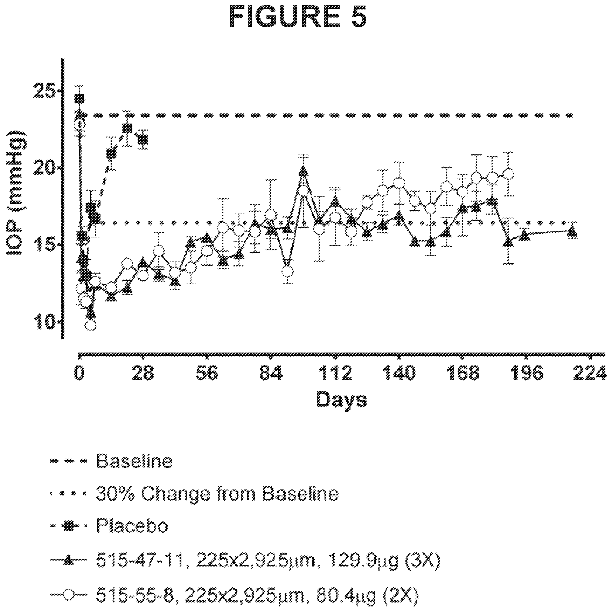

**FIG. 5** depicts the IOP as a function of time for implants 515-47-11 (3 implants) and 515-55-8 (2 implants) over the course of 224 Days. This data demonstrates that the paracentesis of the anterior chamber with a 22-gauge to 27-gauge needle alone resulted in IOP-lowering effects that returned to baseline by Day 28 following the procedure (Placebo in plot). Both pharmaceutical compositions provided IOP-lowering effects of greater than 30% from baseline for approximately 84 days. 515-47-11 provided IOP-lowering effects of approximately 30%, within standard deviation, for 224 days (over 7 months).

Because paracentesis of the anterior chamber with the application needle resulted in IOP lowering effects that returned to baseline by Day 28, the IOP treatment effects were calculated as the average IOP change from baseline between Day 28 and Day 84, or in the case of the above **FIG. 5****,** change from baseline between Day 28 and Day 224.

The treatment effect observed from 515-55-8 (2 implants) and 515-47-11 (3 implants) over a six month period can be seen in below **Table 10.**

**Table 10: IOP Treatment Effect Data from Normotensive Beagle Dog Model**

| Month | Treatment Effect | | | |
|---|---|---|---|---|
| | 515-55-8 | | 515-47-11 | |
| | | | | |
| | mm Hg | % | mm Hg | % |
| 0.5 | -10.6 | 46 | -12.6 | 54 |
| 1 | -9.3 | 41 | -9.7 | 42 |
| 2 | -8.2 | 36 | -8.5 | 36 |
| 3 | -5.9 | 26 | -8.3 | 36 |
| 4 | -6.1 | 27 | -6.1 | 26 |
| 5 | -3.8 | 17 | -6.9 | 30 |
| 6 | -4.4 | 19 | -5.7 | 24 |
| Average | -6.3 | 28 | -7.5 | 32 |

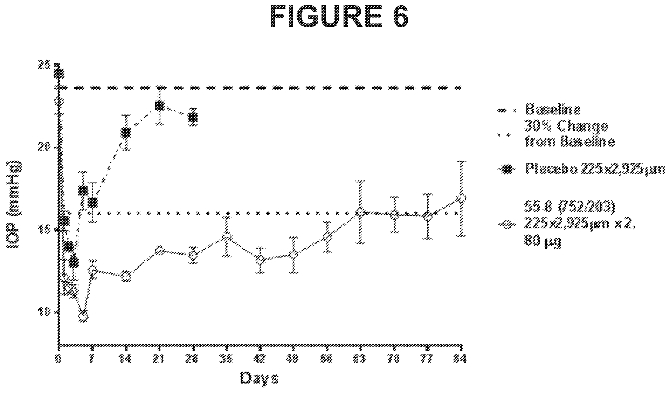

Further, as illustrated in **FIG. 6****,** a single intracameral administration of a dose of 80 µg of travoprost contained within two implants of 225 µm × 225 µm ×2,925 µm in size resulted in a robust IOP-lowering effect. The data in **FIG. 6** demonstrates that 515-55-8, with a travoprost dose of 80 µg, decreased the IOP by 7.9±1.4 mmHg, over 84 days.

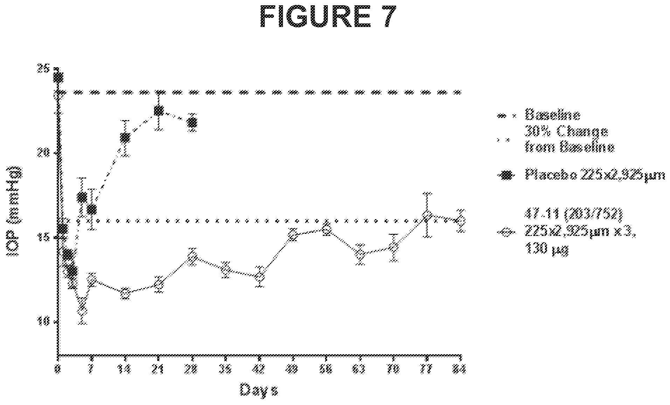

Further, **FIG. 7** demonstrates that 515-47-11 with a travoprost dose of 130 µg (3 implants) resulted in IOP reduction by 8.8±1.3 mmHg, over 84 days.

### Example 8B: In Vivo Study PRE006

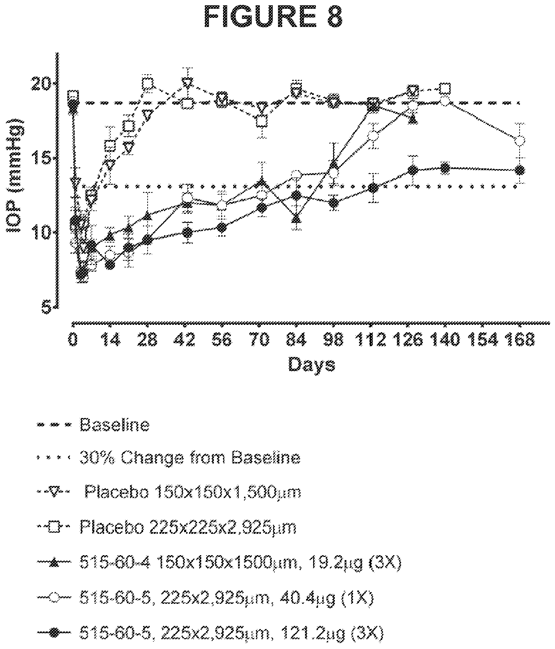

**FIG. 8** depicts the IOP as a function of time for implants 515-60-5 (1 implant), 515-60-5 (3 implants), and 515-60-4 (3 implants). Two placebo implants comprising 20% w/w lactose and 80% w/w RG 752 S with dimensions of 150 µm X 150 µm X 1,500 µm and 225 µm X 225 µm X 2,925 µm were also implanted as controls. As in the first study, the transient lowering of the IOP with the procedure with therapeutic agent free implants returns to baseline in approximately 28 days. The IOP for both placebos maintains at baseline through the data collected (126-140 days). This data demonstrates all three configurations under investigation provided IOP-lowering effects of greater than 30% of baseline through approximately 84 days. After approximately 84 days, both 515-60-4 and 515-60-5 (1 implant) appear to have reduced efficacy, with transient excursions to baseline. 515-60-5 (3 implants) appears to maintain IOP-lowering effect of approximately 30% from baseline through the collected data of 168 days (over 5 months).

The treatment effect observed from 515-60-4 (3 implants); 515-60-5 (1 implant); and 515-60-5 (3 implants), along with additionally tested implants 515-60-3 (1 and 3 implants), over a five month period can be seen in below **Table 11.**

**Table 11: IOP Treatment Effect Data from Normotensive Beagle Dog Model**

| Month | Treatment Effect | | | | | | | | | |
|---|---|---|---|---|---|---|---|---|---|---|
| | 515-60-3 | | 515-60-3 | | 515-60-4 | | 515-60-5 | | 515-60-5 | |
| | 1 implant | | 3 implants | | 3 implants | | 1 implant | | 3 implants | |
| | mm Hg | % | mm Hg | % | mm Hg | % | mm Hg | % | mm Hg | % |
| 0.5 | -8.4 | 45 | -7.7 | 41 | -8.5 | 45 | -10.3 | 55 | -10.8 | 58 |
| 1 | -7.7 | 41 | -9.9 | 53 | -7.2 | 38 | -9.3 | 50 | -9.1 | 49 |
| 2 | -5.7 | 31 | -8.7 | 47 | -6.5 | 35 | -7.0 | 37 | -8.3 | 44 |
| 3 | -8.4 | 45 | -8.4 | 45 | -6.3 | 34 | -6.5 | 34 | -8.6 | 46 |
| 4 | -3.2 | 17 | -3.9 | 21 | 0.2 | -1 | -2.3 | 12 | -5.6 | 30 |
| 5 | 0.5 | -3 | -5.2 | 28 | -0.3 | 2 | -0.1 | 1 | -4.3 | 23 |
| Average | -4.9 | 26 | -7.2 | 39 | -4.0 | 22 | -5.0 | 27 | -7.2 | 38 |

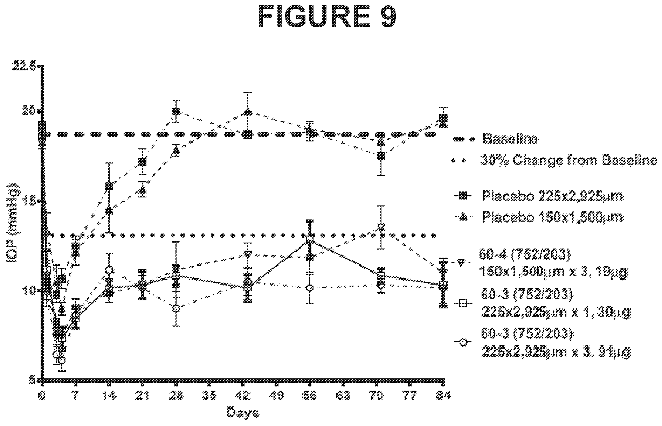

Further, **FIG. 9** demonstrates that: 515-60-4 with a total travoprost dose of 19 µg (3 implants) decreased the IOP by 6.4±1.0 mmHg; and 515-60-3 with a travoprost dose of 30 µg (1 implant) decreased the IOP by 7.6±1.1 mmHg; and 515-60-3 with a total travoprost dose of 91 µg (3 implants) decreased the IOP by 8.9±0.6 mmHg, over 84 days.

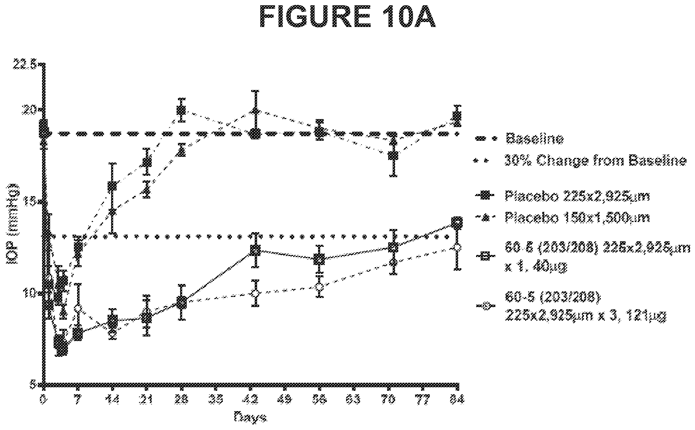

Also, **FIG. 10A** demonstrates that: 515-60-5 with a travoprost dose of 40 µg (1 implant) decreased the IOP by 6.8±1.6 mmHg, and 515-60-5 with a total travoprost dose of 121 µg (3 implants) decreased the IOP by 7.8±1.2 mmHg, over 84 days.

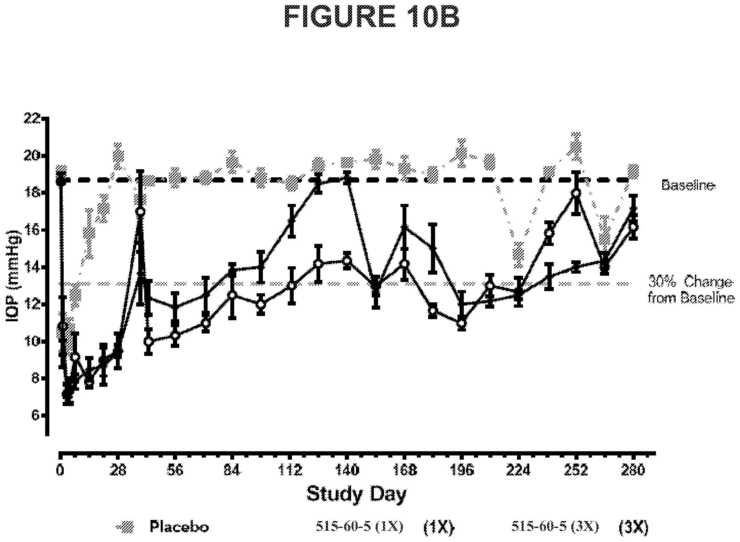

**FIG. 10B** demonstrates animals dosed with one or three implants per eye displayed an average decrease in IOP from baseline of 26.0% and 34.5%, respectively, through 8 months following a single dose administration. The placebo administration resulted in a transient decrease in IOP related to the injection procedure followed by IOP return to baseline by day 28.

### Example 8C: In Vivo Study PRE009

In this study a number of pharmaceutical compositions were evaluated. One placebo implant comprising 7% w/w lactose and 93% w/w polymer matrix (70% R203S/30% RG752S) with dimensions of 180 µm X 160 µm X 3,000 µm was also implanted as a control.

**FIG. 11A** depicts the IOP as a function of time for implants 0003-001-6A (3 implants), 0003-001-6B (3 implants), and 0003-001-8A (3 implants). This data demonstrates that the use of multiple implants containing the same total therapeutic agent content as a single implant, as shown in previous examples, may provide equivalent IOP-lowering effects.

**FIG. 11B** depicts the IOP as a function of time for implants 0003-001-6A (1 implant), 0003-001-6B (1 implant), 0003-001-7A (1 implant), and 0003-001-8A (1 implant). This data demonstrates that increasing the PLA content in the PLGA/PLA blend for a given shape and approximate therapeutic agent content increases the IOP-lowering effect (comparing 0003-001-8A to 0003-001-7A to 0003-001-6A). However, at a given PLGA/PLA blend, a smaller shape with a lower therapeutic agent content may be as effective or more effective in lowering IOP (comparing 0003-001-6A to 0003-001-6B).

The treatment effect observed from the implants of **FIG. 11A** and **11B****,** along with additionally tested implants, over a 2.5 month period can be seen in below **Table 12.**

**Table 12: IOP Treatment Effect Data from Normotensive Beagle Dog Model**

| Month | Treatment Effect | | | | | | | | | | | | | |
|---|---|---|---|---|---|---|---|---|---|---|---|---|---|---|
| | 0003-001-6B | | 0003-001-6B | | 0003-001-6A | | 0003-001-6A | | 0003-001-7A | | 0003-001-8A | | 0003-001-8A | |
| | 1 implant | | 3 implants | | 1 implant | | 3 implants | | 1 implant | | 1 implant | | **3** implants | |
| | mm Hg | % | mm Hg | % | mm H g | % | mm Hg | % | mm Hg | % | mm H g | % | mm Hg | % |
| 0.5 | -4.8 | 26 | -6.0 | 33 | -6.0 | 33 | -7.0 | 38 | -7.3 | 40 | -6.5 | 36 | -8.5 | 47 |
| 1 | -4.3 | 23 | -5.8 | 32 | -5.5 | 30 | -7.3 | 40 | -9.5 | 52 | -5.8 | 32 | -6.8 | 37 |
| 1.5 | -3.3 | 18 | -5.0 | 27 | -2.5 | 14 | -5.0 | 27 | -6.0 | 33 | -4.8 | 26 | -5.8 | 32 |
| 2 | -3.3 | 18 | -5.3 | 29 | -5.3 | 29 | -6.3 | 34 | -6.0 | 33 | -10.8 | 59 | -4.0 | 22 |
| 2.5 | -3.0 | 16 | -2.3 | 13 | -2.8 | 16 | -6.3 | 35 | -5.0 | 27 | -11.2 | 61 | -4.0 | 22 |
| Average | -3.5 | 19 | -4.6 | 25 | -4.0 | 22 | -6.2 | 34 | -6.6 | 36 | -8.2 | 45 | -5.2 | 28 |

**FIG. 11C** depicts the IOP as a function of time for implants 0003-001-4B (1 implant), 0003-001-4B (3 implants), 0003-001-14A (1 implant), and 0003-001-14B (3 implants). This data demonstrates that utilizing 1 implant or 3 implants, to deliver a given amount of therapeutic agent, may be equally effective, at least through approximately 98 days (comparing 0003-001-14A and 0003-001-14B).

The treatment effect observed from the implants of **FIG. 11C****,** along with additionally tested implants, over a 2.5 month period can be seen in below **Table 13.**

**Table 13: IOP Treatment Effect Data from Normotensive Beagle Dog Model**

| Month | Treatment Effect | | | | | | | | | | | | | |
|---|---|---|---|---|---|---|---|---|---|---|---|---|---|---|
| | 0003-001-14B | | 0003-001-14B | | 0003-001-14A | | 0003-001-14A | | 0003-001-4B | | 0003-001-4B | | 0003-001-5B | |
| | 1 implant | | 3 implants | | 1 implant | | 3 implants | | 1 implant | | 3 implants | | 3 implants | |
| | mm Hg | % | mm Hg | % | mm Hg | % | mm Hg | % | mm Hg | % | mm Hg | % | mm Hg | % |
| 0.5 | -2.5 | 14 | -3.5 | 20 | -3.0 | 17 | -5.0 | 28 | -4.0 | 22 | -6.8 | 38 | -4.8 | 27 |
| 1 | -5.5 | 31 | -2.5 | 14 | -5.0 | 28 | -6.5 | 37 | -2.8 | 15 | -5.0 | 28 | -3.8 | 21 |
| 1.5 | -4.8 | 27 | -3.3 | 18 | -3.0 | 17 | -6.3 | 35 | -3.5 | 20 | -5.0 | 28 | -2.8 | 15 |
| 2 | -5.0 | 28 | -3.5 | 20 | -3.8 | 21 | -6.0 | 34 | -3.0 | 17 | -6.3 | 35 | -2.0 | 11 |
| 2.5 | -4.6 | 26 | -4.3 | 24 | -4.3 | 24 | -4.3 | 24 | -3.9 | 22 | -4.0 | 22 | -4.4 | 25 |
| Average | -5.0 | 28 | -3.4 | 19 | -4.0 | 23 | -5.8 | 33 | -3.3 | 19 | -5.1 | 28 | -3.3 | 18 |

**FIG. 11D** depicts the IOP as a function of time for implants 0003-001-6B (1 implant) and 0003-001-6B (3 implants). Animals dosed with one or three implants per eye demonstrated an average decrease in IOP from baseline of 19.8% and 27.8%, respectively, for seven months following a single dose.

### Example 9: Pupil Miosis in Normotensive Beagle Dogs

The pharmacologic effect of travoprost on the pupil, resulting in pupil miosis, has been described previously and was observed for the present formulations, as illustrated in **FIG.12A-12D****.**

The safety profile of the presently disclosed travoprost intracameral implant was evaluated in non-GLP assessments using a normotensive canine model. The beagle dog is an acceptable model for assessing the safety of intracameral implants due to the size of the eye, the anatomy and physiology of the anterior chamber and angle, and the pharmacologically similar response to this class of agents (Dorairaj S, Liebmann JM, Ritch R. "Quantitative evaluation of anterior segment parameters in the era of imaging," Trans Am Ophthalmol Soc. 2007;105:99-108; discussion 108-10; and Tsai S, Almazan A, Lee SS, Li H, Conforti P, Burke J, Miller PE, Robinson MR. "The effect of topical latanoprost on anterior segment anatomic relationships in normal dogs," Vet Ophthalmol. 2013;16(5):370-376).

Both eyes of each animal were examined using a hand-held slit lamp and indirect ophthalmoscope according to the modified microscopic ocular grading system (Hackett RB, McDonald TO. "Ophthalmic Toxicology and Assessing Ocular Irritation," Dermatoxicology, 5th Edition. Ed. F.N. Marzulli and H.I. Maibach. Washington, DC: Hemisphere Publishing Corporation. 1996, 299-305 and 557-566; and Draize JH. "Appraisal of the safety of chemicals in foods, drugs, and cosmetics," Association of Food and Drug Officials of the United States, Austin, TX, 1959:46-59; and also McDonald TO, Shadduck JA. "Eye irritation in dermatoxicology," Marzulli FN, Hemisphere Publishing corp. New York, NY, 1977:579-582).

Composite ocular tolerability score was assessed using utilized the McDonald-Shadduck Scoring System. The ocular tolerability was used to provide an initial assessment of ocular safety and tolerability is a summation of all scores across all domains. A "perfect score" is equal to 0, however, a score that does not equal zero does not indicate a clinically unacceptable pharmaceutical composition.

The safety and tolerability of various formulations were evaluated in normotensive beagle dogs according to the Ocular Safety Index as explained above. Intracameral travoprost implant formulations were administered via a single insertion of one to three implants into the anterior chamber (studies PRE004, PRE006, and PRE009) with doses and rates of release explained above for the PRE004, PRE006, and PRE009 studies. Placebo implant formulations were used as controls and 22 to 27-gauge needles were used to administer implants intracamerally.

It has been shown that travoprost and other PGAs elicit species-specific iris miosis in dogs, which is not present in human subjects. This phenomenon was also shown with the present implants (see **FIG. 12A** (PRE004 Study), **12B** (PRE006 Study), **12C** & **12D** (PRE009 Study)).

The dog-specific pupil miosis consequently leads to reduced pupillary light reflex, graded as abnormality in the modified microscopic ocular grading system. For this reason, the safety data is presented as the composite index without pupillary light reflex scores, excluding the dog-specific miosis that consequently reduces the pupillary light reflex **(****FIG. 13****).**

In the PRE006 study in normotensive Beagle dogs, the travoprost doses ranged from 19 to 121 µg of travoprost and were formulated into RG752S/R203S and R203S/R208S implant formulations. The implants demonstrated overall good ocular safety and tolerability, with peak ocular irritation scores occurring equally for placebo and travoprost implants immediately after implant insertion via paracentesis into the anterior chamber on Days 1 and 3 (**FIG. 13****,** Study PRE006). The composite ocular safety and tolerability scores for travoprost implants remained low and generally equal or comparable to placebo implant scores across the duration of the study. The safety data from the PRE004 study resulted in similar safety and tolerability profiles (data not shown). The data for PRE009 study is also not illustrated, but demonstrated similar tolerability scores as the PRE006.

The most common findings were conjunctival congestion, conjunctival swelling, and impaired pupillary light reflex due to miosis. The conjunctival congestion and swelling were comparable between travoprost doses and placebo implants. The reduced pupillary light reflex was notable for travoprost implants. As discussed above, the latter effect is directly due to the expected pharmacology of travoprost in the canine eye. Based on the previous studies of travoprost and other prostaglandin analogues, these findings are considered to be transient pharmacological responses rather than toxic events.

In summary, travoprost intracameral implants inserted into the anterior chamber of beagle dogs appear to be well tolerated over three months. A transient increase in the ocular safety score was observed immediately following the implant insertion, likely caused by the paracentesis procedure alone.

### Example 11: Various Implant Formulations

Besides the aforementioned implant formulations utilized in the various experiments, there were also a host of other formulations derived and analyzed, as depicted in **Table 17.**

**Table 14: Various Implant Formulations**

| Sample ID | Implant Design | Travoprost loading w/w % | Polymer loading w/w % | Resomer Polymer (Polymer Ratio) | Total Mass (µg) (STDEV,µg) |
|---|---|---|---|---|---|
| 515-20-8 | 250 x 250 x 1,500 µm | 30% | 70% | R203S/R208S (50/50) | |
| 515-57-2 | 225 x 225 x 2,925µm | 20.7% | 79.3% | RG752S/RG755S (63/37) | |
| 515-57-3 | 225 x 225 x 2,925µm | 20.7% | 79.3% | R202H/RG755S (63/37) | |
| 515-47-2 | 225 x 225 x 2,925µm | 29.4% | 70.6% | RG503S | |
| 515-47-9 | 225 x 225 x 2,925µm | 29.4% | 70.6% | RG752S/R208S (15/85) | |
| 515-47-10 | 225 x 225 x 2,925µm | 29.4% | 70.6% | RG755S/R208S (15/85) | |
| 515-47-11 | 225 x 225 x 2,925µm | 29.4% | 70.6% | R203S/R208S (33/67) | |
| 515-47-12 | 225 x 225 x 2,925µm | 29.4% | 70.6% | R203S/R208S (15/85) | |
| 515-47-13 | 225 x 225 x 2,925µm | 29.4% | 70.6% | R205S/R208S (33/67) | |
| 515-47-14 | 225 x 225 x 2,925µm | 29.4% | 70.6% | R205S/R208S (15/85) | |
| 515-49-2 | 225 x 225 x 2,925µm | 29.4% | 70.6% | RG752S | |
| 515-53-9 | 150 x 150 x 1,500µm | 29.4% | 70.6% | RG752S/R208S (15/85) | |
| 515-53-10 | 150 x 150 x 1,500µm | 29.4% | 70.6% | RG755S/R208S (15/85) | |
| 515-53-11 | 150 x 150 x 1,500µm | 29.4% | 70.6% | R203S/R208S (33/67) | |
| 515-53-12 | 150 x 150 x 1,500µm | 29.4% | 70.6% | R203S/R208S (15/85) | |
| 515-53-13 | 150 x 150 x 1,500µm | 29.4% | 70.6% | R205S/R208S (33/67) | |
| 515-53-14 | 150 x 150 x 1,500µm | 29.4% | 70.6% | R205S/R208S (15/85) | |
| 515-65-9 | 150 x 150 x 5,150µm | 40% | 60% | R203S/R205S (33/67) | |
| 515-65-10 | 150 x 150 x 5,150µm | 40% | 60% | R203S/R205S (15/85) | |
| 515-65-13 | 150 x 150 x 5,150µm | 40% | 60% | R205S/R208S (33/67) | |
| 515-65-14 | 150 x 150 x 5,150µm | 40% | 60% | R205S/R208S (15/85) | |
| 515-65-17 | 150 x 150 x 5,150µm | 40% | 60% | RG757S/R207S (33/67) | |
| 515-65-18 | 150 x 150 x 5,150µm | 40% | 60% | RG757S/R207S (15/85) | |
| 515-65-19 | 150 x 150 x 5,150µm | 50% | 50% | RG757S/R207S (15/85) | |
| 515-66-1 | 150 x 150 x 5,150µm | 30% | 70% | R205S | |
| 515-66-6 | 150 x 150 x 5,150µm | 30% | 70% | R203S/R208S (33/67) | |
| 515-66-7 | 150 x 150 x 5,150µm | 30% | 70% | R203S/R208S (15/85) | |
| 515-66-8 | 150 x 150 x 5,150µm | 30% | 70% | R205S/R208S (15/85) | |
| 515-66-9 | 250 x 250 x 1,500µm | 30% | 70% | R208S/RG750S (85/15) | |
| 003-001-1A | 180x 160 x 3,000µm | 31.8% | 68.2% | RG750S | 89.7 µg (2.8 µg) |
| 003-001-2A | 180 x 160 x 3,000µm | 31.8% | 68.2% | R203S | 64.2 µg (2.3 µg) |
| 003-001-3A | 180 x 160 x 3,000µm | 31.8% | 68.2% | RG752S/R203S (33/67) | 109.3 µg (4.8 µg) |
| 003-001-4A | 180 x 160 x 3,000µm | 31.8% | 68.2% | RG752S/R203S (15/85) | N/A |
| 003-001-5A | 180 x 160 x 3,000µm | 31.8% | 68.2% | RG750/R203 (85/15) | 70.8 µg (2.0 µg) |
| 003-001-6A | 180 x 160 x 3,000µm | 31.8% | 68.2% | R203S/R208S (33/67) | 80.2 µg (6.7 µg) |
| 003-001-7A | 180 x 160 x 3,000µm | 31.8% | 68.2% | R203S/R208S (20/80) | 94.8 µg (2.1 µg) |
| 003-001-8A | 180 x 160 x 3,000µm | 31.8% | 68.2% | R203S/R208S (15/85) | 89.8 µg (5.5 µg) |
| 003-001-9A | 180 x 160 x 3,000µm | 31.8% | 68.2% | RG750S/R208S (15/85) | N/A |
| 003-001-10A | 180 x 160 x 3,000µm | 31.8% | 68.2% | RG750S/R203S/ R208S (10/30/60) | 90.4 µg (3.0 µg) |
| 003-001-11A | 180 x 160 x 3,000µm | 31.8% | 68.2% | RG750S/R203S/ R208 (10/60/30) | 77.5 µg (1.3 µg) |
| 003-001-12A | 180 x 160 x 3,000µm | 31.8% | 68.2% | RG752S/R203S/ R208S (10/30/60) | 86.2 µg (1.8 µg) |
| 003-001-13A | 180 x 160 x 3,000µm | 31.8% | 68.2% | RG750S/R205S/ R207S (10/60/30) | 78.7 µg (4.2 µg) |
| 003-001-14A | 180 x 160 x 3,000µm | 31.8% | 68.2% | RG752S/R203S (30/70) | 94.4 µg (3.9 µg) |
| 003-001-1B | 150 x 150 x 1,500µm | 31.8% | 68.2% | RG750S | 40.1 µg (1.2 µg) |
| 003-001-2B | 150 x 150 x 1,500µm | 31.8% | 68.2% | R203S | 32.5 µg (1.5 µg) |
| 003-001-3B | 150 x 150 x 1,500µm | 31.8% | 68.2% | RG752S/R203S (33/67) | 32.8 µg (3.3 µg) |
| 003-001-4B | 150 x 150 x 1,500µm | 31.8% | 68.2% | RG752S/R203S (15/85) | 41.2 µg (2.0 µg) |
| 003-001-5B | 150 x 150 x 1,500µm | 31.8% | 68.2% | RG750/R203 (85/15) | 38.3 µg (1.6 µg) |
| 003-001-6B | 150 x 150 x 1,500µm | 31.8% | 68.2% | R203S/R208S (33/67) | 39.9 µg (1.3 µg) |
| 003-001-7B | 150 x 150 x 1,500µm | 31.8% | 68.2% | R203S/R208S (20/80) | 37.1 µg (2.5 µg) |
| 003-001-8B | 150 x 150 x 1,500µm | 31.8% | 68.2% | R203S/R208S (15/85) | N/A |
| 003-001-9B | 150 x 150 x 1,500µm | 31.8% | 68.2% | RG750S/R208S (15/85) | 37.6 µg (1.3 µg) |
| 003-001-10B | 150 x 150 x 1,500µm | 31.8% | 68.2% | RG750S/R203S/ R208S (10/30/60) | 40.6 µg (1.9 µg) |
| 003-001-11B | 150 x 150 x 1,500µm | 31.8% | 68.2% | RG750S/R203S/ R208 (10/60/30) | 39.5 µg (1.1 µg) |
| 003-001-12B | 150 x 150 x 1,500µm | 31.8% | 68.2% | RG752S/R203S/ R208S (10/30/60) | 40.5 µg (1.6 µg) |
| 003-001-13B | 150 x 150 x 1,500µm | 31.8% | 68.2% | RG750S/R205S/ R207S (10/60/30) | 37.0 µg (2.0 µg) |
| 003-001-14B | 150 x 150 x 1,500µm | 31.8% | 68.2% | RG752S/R203S (30/70) | 39.6 µg (2.2 µg) |

### Example 12: In Vivo Release Profiles

An in vivo study PRE008 was undertaken to directly measure the travoprost release profiles of the disclosed implants.

The implants were dosed intracamerally in New Zealand White (NZW) rabbits and removed at different intervals for the analysis of the residual travoprost contained within the recovered implant.

The amount of released travoprost was calculated as the initial amount of travoprost contained within the implants minus the amount of travoprost contained in the recovered implants.

**Table 15** illustrates the travoprost recovered in vivo during the experiment at day 28 and day 56 for select implants. **FIG. 14** shows the µg travoprost recovered over time. **FIG. 15** shows the % travoprost recovered over time. **FIG. 16** shows the % travoprost released over time. **FIG. 17** shows the release rate over time.

**Table 15: Travoprost Recovered In Vivo**

| | | | | | |
|---|---|---|---|---|---|
| 515-60-5 | T=0 ug | Day | 0 | 28 | 56 |
| | 40.4 | ug recover | 40.4 | 22.6 | |
| | | % (recover) | 100.0% | 55.9% | 0.0% |
| | | ug (released) | 0.0 | 17.8 | 40.4 |
| | | % (released) | 0.0% | 44.1% | 100.0% |
| | | ng/day released | NA | 635.7 | 721.4 |
| 0003-001-6A | T=0 ug | Day | 0 | 28 | 56 |
| | 25.7 | ug (recover) | 25.7 | 20.0 | 20.1 |
| | | % (recover) | 100.0% | 77.8% | 78.2% |
| | | ug (released) | 0.0 | 5.7 | 5.6 |
| | | % (released) | 0.0% | 22.2% | 21.8% |
| | | ng/day released | NA | 203.6 | 100.0 |
| 0003-001-14A | T=0 ug | Day | 0 | 28 | 56 |
| | 22.2 | ug (recover) | 22.2 | 20.9 | 13.3 |
| | | % (recover) | 100.0% | 94.1% | 59.9% |
| | | ug (released) | 0.0 | 1.3 | 8.9 |
| | | % (released) | 0.0% | 5.9% | 40.1% |
| | | ng/day released | NA | 46.4 | 158.9 |
| 0003-001-14B | T=0 mg | Dav | 0 | 28 | 56 |
| | 9.9 | ug (recover) | 9.9 | 7.7 | 5.3 |
| | | % (recover) | 100.0% | 77.8% | 53.5% |
| | | ug (released) | 0.0 | 2.2 | 4.6 |
| | | % (released) | 0.0% | 22.2% | 46.5% |
| | | ng/day released | NA | 78.6 | 82.1 |
| 0003-001-4B | T=0 ug | Day | 0 | 28 | 56 |
| | 8.0 | ug (recover) | 8.0 | 5.3 | 4.9 |
| | | % (recover) | 100.0% | 66.3% | 61.3% |
| | | ug (released) | 0.0 | 2.7 | 3.1 |
| | | % (released) | 0.0% | 33.8% | 38.8% |
| | | ng/day released | NA | 96.4 | 55.4 |

### Example 15: Pharmacokinetic Post Dose In Vivo and In Vitro Correlations

Data from the aforementioned in vivo and in vitro experiments was statistically analyzed to determine correlations among the implants pharmacokinetic behavior.

**FIG. 18** is a representation of the in vivo rate of release vs. aqueous humor concentration of travoprost 1 month post dose. The graph depicts the in vivo rate of release (ng/day) on the x-axis and the travoprost free acid concentration in the aqueous humor (pg/mL) on the y-axis.

**FIG. 19** is a representation of the in vivo rate of release vs. aqueous humor concentration of travoprost 2 months post dose. The graph depicts the in vivo rate of release (ng/day) on the x-axis and the travoprost free acid concentration in the aqueous humor (pg/mL) on the y-axis.

**FIG. 20** is a representation of the in vivo rate of release vs. aqueous humor concentration of travoprost combined 1 and 2 month post dose data. The graph depicts the in vivo rate of release (ng/day) on the x-axis and the travoprost free acid concentration in the aqueous humor (pg/mL) on the y-axis.

**FIG. 21** is a representation of the IOP vs. aqueous humor concentration of travoprost 1 month post dose. The graph depicts the travoprost free acid concentration in the aqueous humor (pg/mL) on the x-axis and the IOP treatment effect (mmHg) on the y-axis.

**FIG. 22** is a representation of the IOP vs. aqueous humor concentration of travoprost 2 months post dose. The graph depicts the travoprost free acid concentration in the aqueous humor (pg/mL) on the x-axis and the IOP treatment effect (mmHg) on the y-axis.

**FIG. 23** is a representation of the IOP vs. aqueous humor concentration of travoprost combined 1 and 2 months post dose data. The graph depicts the travoprost free acid concentration in the aqueous humor (pg/mL) on the x-axis and the IOP treatment effect (mmHg) on the y-axis.

### Example 15: Aqueous Humor Pharmacokinetic Data From In Vivo Study

Aqueous humor data from in vivo beagle dog experiment PRE006B was gathered and analyzed.
**Table 16** illustrates aqueous humor (ester + acid) data results obtained on days 14, 28, and 60.

**Table 16: Beagle Dog Aqueous Humor PK Results, Travoprost Ester + Acid**

| Time Days | Beagle Dog Aqueous Humor PK Results, Travoprost Acid | | | | | | | |
|---|---|---|---|---|---|---|---|---|
| | 515-66-6 | | 515-66-1 | | 515-65-14 | | 515-67-1 (Placebo) | |
| | AVE, pg | SD, pg | AVE, pg | SD, pg | AVE, pg | SD, pg | AVE, pg | SD, pg |
| 14 | 123.5 | 77.1 | 288.7 | 103.2 | 114.2 | 25.0 | 0.0 | 0.0 |
| 28 | 72.7 | 13.8 | 268.3 | 31.0 | 257.5 | 14.8 | 0.0 | 0.0 |
| 60 | 47.7 | 3.3 | 118.3 | 16.2 | 489.7 | 223.8 | 0.0 | 0.0 |

| Time Days | Beagle Dog Aqueous Humor PK Results, Travoprost Ester | | | | | | | |
|---|---|---|---|---|---|---|---|---|
| | 515-66-6 | | 515-66-1 | | 515-65-14 | | 515-67-1 (Placebo) | |
| | AVE, pg | SD, pg | AVE, pg | SD, pg | AVE, pg | SD, pg | AVE, pg | SD, pg |
| 14 | 14.5 | 8.8 | 0.3 | 0.6 | 0.7 | 1.2 | 0.0 | 0.0 |
| 28 | 6.6 | 5.5 | 6.7 | 5.9 | 13.0 | 17.0 | 0.0 | 0.0 |
| 60 | 0.0 | 0.0 | 0.0 | 0.0 | 16.7 | 15.6 | 0.0 | 0.0 |

| Time Days | Beagle Dog Aqueous Humor PK Results, Travoprost Acid + Ester | | | | | | | |
|---|---|---|---|---|---|---|---|---|
| | 515-66-6 | | 515-66-1 | | 515-65-14 | | 515-67-1 (Placebo) | |
| | AVE, pg | SD, pg | AVE, pg | SD, pg | AVE, pg | SD, pg | AVE, pg | SD, pg |
| 14 | 138.0 | 68.3 | 285.0 | 99.3 | 112.9 | 24.9 | 0.0 | 0.0 |
| 28 | 121.8 | 74.0 | 271.7 | 21.2 | 270.5 | 31.8 | 0.0 | 0.0 |
| 60 | 43.0 | 0.4 | 108.2 | 16.7 | 497.3 | 214.3 | 0.0 | 0.0 |

### Example 16: Intracameral Implant Administration

The aforementioned experiments utilized an appropriately sized needle ranging from 23-gauge to 27-gauge. **FIG. 24A** is an electron micrograph illustrating a 150 µm × 150 µm × 1,500 µm implant in a 27G thin-walled needle. **FIG. 24B** is an electron micrograph illustrating a 225 µm × 225 µm × 2,925 µm implant in a 27G ultra thin-walled needle.

It was observed that 225µm × 225µm × 2,925 µm implants tightly fit in 27G ultra thin-walled (UTW) needles.

Also, 150 µm × 150 µm × 1,500 µm implants fit in 27G thin-walled (TW) needles and loosely fit in 27G UTW needles.

Further, 150 µm × 150 µm × 1,500 µm implants do not fit in 30G UTW needles.

Thus, in certain embodiments, implant design with critical dimensions less than ∼200 µm may fit in a custom-made 28G or 29G UTW needle.

In some embodiments, implant designs with critical dimensions more than ∼200 µm would likely not fit in a custom-made 28G or 29G UTW needle.

Example embodiments have been described herein. As may be noted elsewhere, these embodiments have been described for illustrative purposes only and are not limiting. Other embodiments are possible and are covered by the disclosure, which will be apparent from the teachings contained herein. Thus, the breadth and scope of the disclosure should not be limited by any of the above-described embodiments, but should be defined only in accordance with features and claims supported by the present disclosure and their equivalents. Moreover, embodiments of the subject disclosure may include formulations, compounds, methods, systems, and devices which may further include any and all elements/features from any other disclosed formulations, compounds, methods, systems, and devices, including the manufacture and use thereof. In other words, features from one and/or another disclosed embodiment may be interchangeable with features from other disclosed embodiments, which, in turn, correspond to yet other embodiments. One or more features/elements of disclosed embodiments may be removed and still result in patentable subject matter (and thus, resulting in yet more embodiments of the subject disclosure). Furthermore, some embodiments of the present disclosure may be distinguishable from the prior art by specifically lacking one and/or another feature, functionality, ingredient or structure, which is included in the prior art (*i.e.,* claims directed to such embodiments may include "negative limitations" or "negative provisos").

### INCORPORATION BY REFERENCE

All references, articles, publications, patents, patent publications, and patent applications cited herein are incorporated by reference in their entireties for all purposes. However, mention of any reference, article, publication, patent, patent publication, and patent application cited herein is not, and should not be taken as, an acknowledgment or any form of suggestion that they constitute valid prior art or form part of the common general knowledge in any country in the world.

## Claims

1. A pharmaceutical composition for treating an ocular condition, comprising
A) a biodegradable polymer matrix; and
B) at least one therapeutic agent homogenously dispersed within the polymer matrix;
wherein the biodegradable polymer matrix contains a mixture of polymers comprising
i) an ester end-capped biodegradable poly(D,L-lactide-co-glycolide) copolymer having an inherent viscosity of 0.16 to 0.24 dL/g measured at 0.1% w/v in CHCl₃ at 25°C with an Ubbelohde size 0c glass capillary viscometer; and
ii) an ester end-capped biodegradable poly(D,L-lactide) homopolymer having an inherent viscosity of 0.25 to 0.35 dL/g measured at 0.1% w/v in CHCl₃ at 25°C with an Ubbelohde size 0c glass capillary viscometer.

2. A pharmaceutical composition according to claim 1, wherein the poly(D,L-lactide-co-glycolide) copolymer comprises 73-77 mole% D,L-lactide and 23-27 mole% glycolide.

3. A pharmaceutical composition according to claim 1, wherein the biodegradable polymer matrix comprises the poly(D,L-lactide-co-glycolide) copolymer in an amount of about 10% to about 30% and comprises the poly(D,L-lactide) homopolymer in an amount of about 70% to about 90%.

4. A pharmaceutical composition for treating an ocular condition, comprising
A) a biodegradable polymer matrix; and
B) at least one therapeutic agent homogenously dispersed within the polymer matrix;
wherein the biodegradable polymer matrix contains a mixture of polymers comprising
i) an ester end-capped biodegradable poly(D,L-lactide) homopolymer having an inherent viscosity of 0.25 to 0.35 dL/g measured at 0.1% w/v in CHCl₃ at 25°C with an Ubbelohde size 0c glass capillary viscometer; and
ii) an ester end-capped biodegradable poly(D,L-lactide) homopolymer having an inherent viscosity of 1.8 to 2.2 dL/g measured at 0.1% w/v in CHCl₃ at 25°C with an Ubbelohde size 0c glass capillary viscometer.

5. A pharmaceutical composition according to claim 1 or claim 4, wherein the biodegradable polymer matrix is present in an amount of about 68% to about 71% w/w of the pharmaceutical composition.

6. A pharmaceutical composition according to claim 1 or claim 4, wherein the at least one therapeutic agent is present in an amount of about 29% to about 32% w/w of the pharmaceutical composition.

7. A pharmaceutical composition according to claim 1 or claim 4, wherein the at least one therapeutic agent is selected from a prostaglandin, a prostaglandin prodrug, a prostaglandin analogue, a prostamide, and combinations thereof.

8. A pharmaceutical composition according to claim 1 or claim 4, wherein the at least one therapeutic agent is selected from latanoprost, travoprost, bimatoprost, tafluprost, and unoprostone isopropyl.

9. A pharmaceutical composition according to claim 1 or claim 4, which is in the form of an ocular implant.

10. A pharmaceutical composition as defined in any preceding claim for use in the treatment of an ocular condition.

11. A pharmaceutical composition for use according to claim 10, wherein the ocular condition is glaucoma or ocular hypertension.
